# EUROPEAN PATENT APPLICATION

(11) **EP 1 702 926 A1**
(43) Date of publication of application: **20.09.2006**
(21) Application number: 05703673.3
(22) Date of filing: 07.01.2005
(51) Int. Cl.: C07H 15/04, C07H 15/10, A61P 9/00, A61P 29/00, A61P 31/04, A61P 37/02, A61P 37/06, A61P 43/00

(54) **LEVULOSE GLUCOSELIPID A ANALOGUE**

(30) Priority: 08.01.2004 JP 2004002902
(71) Applicant: Sankyo Company, Limited, Tokyo 103-8426 (JP)
(72) Inventor: SHIOZAKI, Masao c/o Sankyo Company, Limited, Tokyo 140-8710 (JP); SHIMOZATO, Takaichi c/o Sankyo Company, Limited, Tokyo 140-8710 (JP)
(74) Representative: Gibson, Christian John Robert
(86) International application number: PCT/JP2005/000434
(87) International publication number: WO 2005/066193

(57) **Abstract**

A compound having excellent macrophage activity inhibitory action, which is represented by the general following formula: 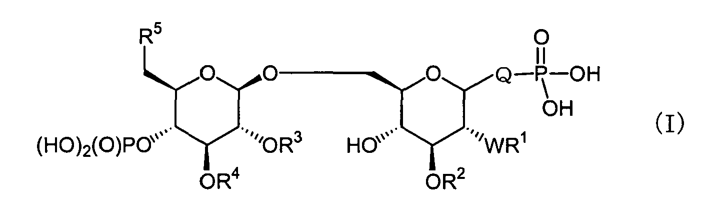 [wherein Q represents an oxygen atom, a C₁-C₃ alkylene group, a -O-Alk- group or a -O-Alk-O- group (in which Alk is a C₁-C₃ alkylene group),
R¹ represents a C₁-C₂₀ alkanoyl group which may be substituted, a C₃-C₂₀ alkenoyl group which may be substituted or a C₃-C₂₀ alkynoyl group which may be substituted,
R², R³ and R⁴, which may be the same or different, represent a hydrogen atom, a C₁-C₂₀ alkyl group which may be substituted, a C₂-C₂₀ alkenyl group which may be substituted, a C₂-C₂₀ alkynyl group which may be substituted, a C₁-C₂₀ alkanoyl group which may be substituted, a C₃-C₂₀ alkenoyl group which may be substituted or a C₃-C₂₀ alkynoyl group which may be substituted,
R⁵ represents a hydrogen atom, a halogen atom, a hydroxyl group, a C₁-C₆ alkoxy group which may be substituted, a C₂-C₆ alkenyloxy group which may be substituted or a C₂-C₆ alkynyloxy group which may be substituted, and
W represents an oxygen atom or a -NH- group], or a pharmacologically acceptable salt thereof.

## Description

### Technical Field

The present invention relates to a novel lipid A analogue or a pharmacologically acceptable salt thereof having excellent macrophage activity inhibitory action and useful as an anti-inflammatory agent, an anti-autoimmune disease agent, an immunosuppressive agent, an antiseptic agent or a prognosis-improving agent after coronary artery bypass surgery, a pharmaceutical composition containing the same as an active ingredient, use of the lipid A analogue or a pharmacologically acceptable salt thereof for producing the pharmaceutical composition, and a method for prophylaxis or treatment of disease, which comprises administering a pharmacologically effective dose of the lipid A analogue or a pharmacologically acceptable salt thereof to a warm-blooded animal (particularly a human).

### Background Art

Gram-negative bacteria from enterobacteria contain, in the outermost membrane of the cell wall, a toxic component (endotoxin) that is not extracellularly secreted. In addition to the endotoxic activity, the endotoxin exhibits various biological activities such as immunoadjuvant activating action associated with biophylaxis, macrophage activating action, mitogen activating action, pyrogenic action, tumor necrosis action, antibody production enhancing action and TNF inducing action.

Such endotoxin is composed of lipopolysaccharides, and a portion called "lipid A" has been confirmed to be the active center for the endotoxin activity (Imoto et al., Tetrahedron Letters, 1985, vol.26, P.1545).

On the other hand, a bacterial lipid A related compound (Rs-DPLA) found in *Rhodobacter sphaeroides* (Koresh et al., Journal of Biological Chemistry, 1988, vol.263, P.5502 and Infection and Immunity, vol.57, P.1336) exhibits LPS antagonist activity against human macrophage, and is possibly useful as an anti-autoimmune disease agent or an antiseptic agent. Thus, development of drugs which exhibit macrophage inhibitory action instead of macrophage activating action has been undertaken.

These research results have prompted attempts to synthesize derivatives of such compounds having useful activity among the above various activities. For example, derivatives described in Christ et al., Journal of American Chemical Society, vol. 116, P.3637, Japanese Patent Application Publication Nos. Hei-10-324694, Hei-5-194470 and 2001-348396 and U.S. Patent No. 5935938 are known.

### Disclosure of the Invention

The present inventors aim at finding a novel lipid A analogue having excellent macrophage activity inhibitory action and useful as an anti-inflammatory agent, an anti-autoimmune disease agent, an immunosuppressive agent, an antiseptic agent or a prognosis-improving agent after coronary artery bypass surgery.

The present inventors have conducted intensive studies in order to solve the above problem, and as a result have found that a certain group of compounds of glycosyl lipid A analogues containing a phosphono group or a phosphonooxyethyl group at position 1 has excellent macrophage activity inhibitory action, and completed the present invention.

The compound according to the present invention is:
1) a compound represented by the general formula: 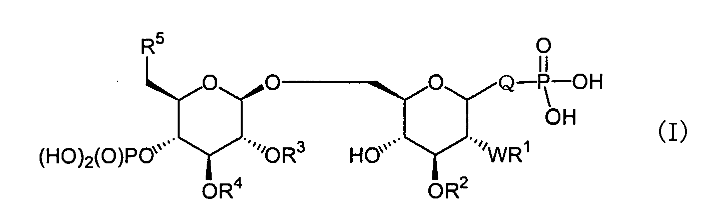 [wherein Q represents an oxygen atom, a C₁-C₃ alkylene group, a -O-Alk- group or a -O-Alk-O- group (in which Alk represents a C₁-C₃ alkylene group),
   W represents an oxygen atom or a -NH- group,
   R¹ (when W is a -NH- group) represents a C₁-C₂₀ alkanoyl group which may be substituted by at least one group selected from the following Substituent group A, a C₃-C₂₀ alkenoyl group which may be substituted by at least one group selected from the following Substituent group A or a C₃-C₂₀ alkynoyl group which may be substituted by at least one group selected from the following Substituent group A,
   R¹ (when W is an oxygen atom), R², R³ and R⁴, which may be the same or different, represent a hydrogen atom, a C₁-C₂₀ alkyl group which may be substituted by at least one group selected from the following Substituent group A, a C₂-C₂₀ alkenyl group which may be substituted by at least one group selected from the following Substituent group A, a C₂-C₂₀ alkynyl group which may be substituted by at least one group selected from the following Substituent group A, a C₁-C₂₀ alkanoyl group which may be substituted by at least one group selected from the following Substituent group A, a C₃-C₂₀ alkenoyl group which may be substituted by at least one group selected from the following Substituent group A or a C₃-C₂₀ alkynoyl group which may be substituted by at least one group selected from the following Substituent group A,
   R⁵ represents a hydrogen atom, a halogen atom, a hydroxyl group, a C₁-C₆ alkoxy group which may have an oxo group, a C₂-C₆ alkenyloxy group which may have an oxo group or a C₂-C₆ alkynyloxy group which may have an oxo group, the Substituent group A consisting of a halogen atom, a hydroxyl group, an oxo group, a C₁-C₂₀ alkoxy group which may have an oxo group, a (C₁-C₂₀ alkoxy) C₁-C₂₀ alkoxy group, a {(C₁-C₂₀ alkoxy) C₁-C₂₀ alkoxy} C₁-C₂₀ alkoxy group, a C₂₋C₂₀ alkenyloxy group which may have an oxo group, a C₂-C₂₀ alkynyloxy group which may have an oxo group, a C₁-C₂₀ alkanoyloxy group which may have an oxo group, a C₃-C₂₀ alkenoyloxy group which may have an oxo group and a C₃-C₂₀ alkynoyloxy group which may have an oxo group], or a pharmacologically acceptable salt thereof.

Of the compounds (I) of the present invention, preferred are
2) a compound wherein W is a -NH- group and R¹ is a C₈-C₁₈ alkanoyl or C₈-C₁₈ alkenoyl group, which may have a substituent selected from the Substituent group A;
3) a compound wherein W is a -NH- group and R¹ is a C₁₀-C₁₈ alkanoyl or C₁₀-C₁₈ alkenoyl group, which may have a substituent selected from the Substituent group A;
4) a compound wherein W is a -NH- group and R¹ is a C₁₂-C₁₆ alkanoyl or C₁₂-C₁₆ alkenoyl group, which may have a substituent selected from the Substituent group A;
5) a compound wherein W is an oxygen atom and R¹, R², R³ and R⁴, which may be the same or different, are a C₄-C₁₈ alkyl, C₄-C₁₈ alkenyl, C₄-C₁₈ alkanoyl or C₄-C₁₈ alkenoyl group, which may have a substituent selected from the Substituent group A;
6) a compound wherein W is an oxygen atom and R¹, R², R³ and R⁴, which may be the same or different, are a C₈-C₁₈ alkyl, C₈-C₁₈ alkenyl, C₈-C₁₈ alkanoyl or C₈-C₁₈ alkenoyl, which may have a substituent selected from the Substituent group A;
7) a compound wherein W is an oxygen atom and R¹, R², R³ and R⁴, which may be the same or different, are a C₁₀-C₁₈ alkyl, C₁₀-C₁₈ alkenyl, G₁₀-C₁₈ alkanoyl or C₁₀₋C₁₈ alkenoyl, which may have a substituent selected from the Substituent group A;
8) a compound wherein W is an oxygen atom and R¹, R², R³ and R⁴, which may be the same or different, are a C₁₂-C₁₆ alkyl, C₁₂-C₁₆ alkenyl, C₁₂-C₁₆ alkanoyl or C₁₂₋C₁₆ alkenoyl group, which may have a substituent selected from the Substituent group A;
9) a compound wherein W is an oxygen atom, R¹ and R³, which may be the same or different, are a C₁₂-C₁₆ alkanoyl or C₁₂-C₁₆ alkenoyl group, which may have a substituent selected from the Substituent group A, and R² and R⁴, which may be the same or different, are a C₁₂-C₁₆ alkyl or C₁₂-C₁₆ alkenyl group, which may have a substituent selected from the Substituent group A;
10) a compound wherein W is an oxygen atom, R¹ and R³, which may be the same or different, are a decanoyl, dodecanoyl, tetradecanoyl, dodecenoyl, tetradecenoyl or octadecenoyl group, which may have a substituent selected from the Substituent group A, and R² and R⁴, which may be the same or different, are a decyl, dodecyl, tetradecyl, dodecenyl, tetradecenyl or octadecenyl group, which may have a substituent selected from the Substituent group A;
11) a compound wherein the substituent selected from the Substituent group A is a fluorine atom, a hydroxyl group, a C₁-C₂₀ alkoxy group, a C₁₂-C₁₄ alkenyloxy group, a C₁₂-C₁₄ alkanoyloxy group or a C₁₂-C₁₄ alkenoyloxy group;
12) a compound wherein the substituent selected from the Substituent group A is a dodecyloxy group, a tetradecyloxy group, a dodecenyloxy group, a tetradecenyloxy group, a dodecanoyloxy group, a tetradecanoyloxy group, a dodecenoyloxy group, a tetradecenoyloxy group or an octadecenoyl group;
13) a compound wherein R⁵ is a halogen atom, a hydroxyl group or an unsubstituted C₁-C₆ alkoxy group;
14) a compound wherein R⁵ is a fluorine atom, a hydroxyl group or a methoxy group;
15) a compound wherein Q is an oxygen atom;
16) a compound wherein Q is a phosphonoethyl group; and
17) a compound wherein position 1 of the right-side glucose or glucosamine takes the α-configuration.
   Of these, more preferred compounds are
18) phosphono 3-O-decyl-2-deoxy-6-O-{3-O-[(R)-3-methoxydecyl]-6-O-methyl-2-O-[(Z)-11-octadecenoyl]-4-O-phosphono-β-D-glucopyranosyl}-2-(3-oxotetradecanoylamino)-α-D-glucopyranoside (Compound No. 1),
   phosphono 3-O-decyl-2-deoxy-6-O-{3-O-[(R)-3-methoxydecyl]-6-O-methyl-2-O-[(Z)-11-octadecenyl]-4-O-phosphono-β-D-glucopyranosyl}-2-(3-oxotetradecanoylamino)-α-D-glucopyranoside (Compound No. 2),
   phosphono 3-O-decyl-2-deoxy-6-O-{3-O-[(R)-3-methoxydecyl]-2-O-[(Z)-11-octadecenoyl]-4-O-phosphono-β-D-glucopyranosyl}-2-(3-oxotetradecanoylamino)-α-D-glucopyranoside (Compound No. 3),
   phosphono 3-O-decyl-2-deoxy-6-O-{3-O-[(R)-3-methoxydecyl]-2-O-[(Z)-11-octadecenyl]-4-O-phosphono-β-D-glucopyranosyl}-2-(3-oxotetradecanoylamino)-α-D-glucopyranoside (Compound No. 4),
   2-(phosphonooxy)ethyl 3-O-decyl-2-deoxy-6-O-{3-O-[(R)-3-methoxydecyl]-6-O-methyl-2-O-[(Z)-11-octadecenoyl]-4-O-phosphono-β-D-glucopyranosyl}-2-(3-oxotetradecanoylamino)-α-D-glucopyranoside (Compound No. 5),
   2-(phosphonooxy)ethyl 3-O-decyl-2-deoxy-6-O- {3-O-[(R)-3-methoxydecyl]-2-O-[(Z)-11-octadecenoyl]-4-O-phosphono-β-D-glucopyranosyl}-2-(3-oxotetradecanoylamino)-α-D-glucopyranoside (Compound No. 6),
   2-(phosphonooxy)ethyl 6-O-{3-O-[(R)-3-methoxydecyl]-6-O-methyl-2-O-[(Z)-11-octadecenoyl]-4-O-phosphono-β-D-glucopyranosyl}-2,3-di-O-dodecyl-α-D-glucopyranoside (Compound No. 7),
   phosphono 3-O-decyl-6-O-{3-O-[(R)-3-methoxydecyl]-6-O-methyl-2-O-[(Z)-11-octadecenoyl]-4-O-phosphono-β-D-glucopyranosyl}-2-O-(3-oxotetradecanoyl)-α-D-glucopyranoside (Compound No. 8),
   phosphono 3-O-decyl-6-O-{3-O-[(R)-3-methoxydecyl]-2-O-[(Z)-11-octadecenoyl]-4-O-phosphono-β-D-glucopyranosyl}-2-O-(3-oxotetradecanoyl)-α-D-glucopyranoside (Compound No. 9),
   2-(phosphonooxy)ethyl 2,3-di-O-dodecyl-6-O-{6-O-methyl-3-O-[(R)-3-methoxydecyl]-2-O-[(Z)-11-octadecenyl]-4-O-phosphono-β-D-glucopyranosyl}-α-D-glucopyranoside (Compound No. 10) or
   phosphono 6-O-{4-O-phosphono-3-O-[(R)-3-methoxydecyl]-6-O-methyl-2-O-[(Z)-11-octadecenyl]-β-D-glucopyranosyl}-3-O-dodecyl-2-O-[(R)-3-hydroxytetradecyl]-α,β-D-glucopyranoside (Compound No. 13)
   Particularly preferred compounds are
19) Compound No. 8 and Compound No. 9.

The present invention also provides a medicament comprising the above compound (I) as an active ingredient, which is specifically an agent for prophylaxis or treatment of inflammation, an agent for prophylaxis or treatment of an autoimmune disease, an immunosuppressive agent, an agent for prophylaxis or treatment of sepsis or a prognosis-improving agent after coronary artery bypass surgery.

In the above formula (I), the alignment in Q is not particularly limited, and the -O-Alk- group may be -O-Alk- or -Alk-O-.

The "C₁-C₃ alkylene group" in the present invention is, for example, a linear or branched C₁-C₃ alkylene group such as a methylene, ethylene, propylene or trimethylene group, preferably a methylene, ethylene or trimethylene group, more preferably an ethylene group.

The "C₁-C₂₀ alkanoyl group" and the "C₁-C₂₀ alkanoyl" moiety in the "C₁-C₂₀ alkanoyloxy group which may have an oxo group" in the present invention are, for example, a linear or branched C₁-C₂₀ alkanoyl group such as formyl, acetyl, propionyl, isopropionyl, butyryl, isobutyryl, sec-butyryl, tert-butyryl, valeryl, isovaleryl, pivaloyl, hexanoyl, isohexanoyl, heptanoyl, isoheptanoyl, octanoyl, isooctanoyl, nonanoyl, isononanoyl, decanoyl, isodecanoyl, undecanoyl, isoundecanoyl, lauroyl, isolauroyl, tridecanoyl, isotridecanoyl, myristoyl, pentadecanoyl, palmitoyl, heptadecanoyl, stearoyl, isostearoyl, nonadecanoyl or icosanoyl group, preferably a C₄-C₁₈ alkanoyl group, more preferably a C₈-C₁₈ alkanoyl group, further preferably a C₁₀-C₁₈ alkanoyl group and particularly preferably a C₁₂-C₁₆ alkanoyl group.

The "C₃-C₂₀ alkenoyl group" and the "C₃-C₂₀ alkenoyl" moiety in the "C₃-C₂₀ alkenoyloxy group" in the present invention are, of the C₁-C₂₀ alkanoyl groups listed above, a group having 3 to 20 carbon atoms and 1 to 3 double bonds, preferably a C₄₋C₁₈ alkenoyl group, more preferably a C₈-C₁₈ alkenoyl group, further preferably a C₁₀-C₁₈ alkenoyl group, particularly preferably a C₁₂-C₁₆ alkenoyl group.

The "C₃-C₂₀ alkynoyl group" and the "C₃-C₂₀ alkynoyl" moiety in the "C₃-C₂₀ alkynoyloxy group" in the present invention are, of the C₁-C₂₀ alkanoyl groups listed above, a group having 3 to 20 carbon atoms and 1 to 3 triple bonds, preferably a C₄₋C₁₈ alkynoyl group, more preferably a C₈-C₁₈ alkynoyl group, further preferably a C₁₀-C₁₈ alkynoyl group, particularly preferably a C₁₂-C₁₆ alkynoyl group.

The "C₁-C₂₀ alkyl group" and the "C₁-C₂₀ alkyl" moiety in the "C₁-C₂₀ alkoxy group" in the present invention are, for example, a linear or branched alkyl group having 1 to 20 carbon atoms such as methyl, ethyl, propyl, isopropyl, butyl, isobutyl, s-butyl, tert-butyl, pentyl, isopentyl, 2-methylbutyl, neopentyl, 1-ethylpropyl, hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, heptyl, 1-methylhexyl, 2-methylhexyl, 3-methylhexyl, 4-methylhexyl, 5-methylhexyl, 1-propylbutyl, 4,4-dimethylpentyl, octyl, 1-methylheptyl, 2-methylheptyl, 3-methylheptyl, 4-methylheptyl, 5-methylheptyl, 6-methylheptyl, 1-propylpentyl, 2-ethylhexyl, 5,5-dimethylhexyl, nonyl, 3-methyloctyl, 4-methyloctyl, 5-methyloctyl, 6-methyloctyl, 1-propylhexyl, 2-ethylheptyl, 6,6-dimethylheptyl, decyl, 1-methylnonyl, 3-methylnonyl, 8-methylnonyl, 3-ethyloctyl, 3,7-dimethyloctyl, 7,7-dimethyloctyl, undecyl, 4,8-dimethylnonyl, dodecyl, tridecyl, tetradecyl, pentadecyl, 3,7,11-trimethyldodecyl, hexadecyl, 4,8,12-trimethyltridecyl, 1-methylpentadecyl, 14-methylpentadecyl, 13,13-dimethyltetradecyl, heptadecyl, 15-methylhexadecyl, octadecyl, 1-methylheptadecyl, nonadecyl, icosyl or 3,7,11,15-tetramethylhexadecyl group, preferably a C₄-C₁₈ alkyl group, more preferably a C₈-C₁₈ alkyl group, further preferably a C₁₀-C₁₈ alkyl group, particularly preferably a C₁₂-C₁₆ alkyl group.

The "C₂-C₂₀ alkenyl group" in the present invention is, of the C₁-C₂₀ alkyl groups listed above, a group having 2 to 20 carbon atoms and 1 to 3 double bonds, preferably a C₄-C₁₈ alkenyl group, more preferably a C₈-C₁₈ alkenyl group, further preferably a C₁₀-C₁₈ alkenyl group, particularly preferably a C₁₂-C₁₆ alkenyl group.

The "C₂-C₂₀ alkynyl group" in the present invention is, of the C₁-C₂₀ alkyl groups listed above, a group having 2 to 20 carbon atoms and 1 to 3 triple bonds, preferably a C₁₀-C₁₈ alkynyl group, more preferably a C₈-C₁₈ alkynyl group, further preferably a C₁₀-C₁₈ alkynyl group, particularly preferably a C₁₂-C₁₆ alkynyl group.

The "halogen atom" in the present invention is a fluorine, chlorine, bromine or iodine atom, preferably a fluorine, chlorine or bromine atom, more preferably a fluorine atom.

The "C₁-C₆ alkoxy group" in R⁵ in the present invention is, for example, a linear or branched alkoxy group having 1 to 6 carbon atoms, such as methoxy, ethoxy, propoxy, isopropoxy, butoxy, isobutoxy, s-butoxy, tert-butoxy, pentyloxy, isopentyloxy, 2-methylbutyloxy, neopentyloxy, 1-ethylpropyloxy, hexyloxy, isohexyloxy, 4-methylpentyloxy, 3-methylpentyloxy, 2-methylpentyloxy, 1-methylpentyloxy, 3,3-dimethylbutyloxy, 2,2-dimethylbutyloxy, 1,1-dimethylbutyloxy, 1,2-dimethylbutyloxy, 1,3-dimethylbutyloxy, 2,3-dimethylbutyloxy or 2-ethylbutyloxy group, preferably a linear or branched alkoxy group having 1 to 4 carbon atoms, more preferably a methoxy group.

The "C₂-C₆ alkenyloxy group" in R⁵ in the present invention is, of the C₁-C₆ alkoxy groups listed above, a group having 2 to 6 carbon atoms and 1 double bond, preferably a C₂-C₄ alkenyloxy group.

The "C₂-C₆ alkynyloxy group" in R⁵ in the present invention is, of the C₁-C₆ alkoxy groups listed above, a group having 2 to 6 carbon atoms and 1 triple bond, preferably a C₂-C₄ alkynyloxy group.

The "(C₁-C₂₀ alkoxy) C₁-C₂₀ alkoxy group" in the present invention is a "C₁₋C₂₀ alkoxy group" listed above substituted by one "C₁-C₂₀ alkoxy group" listed above, preferably a (C₁-C₆ alkoxy) C₁-C₆ alkoxy group.

The "{(C₁-C₂₀ alkoxy) C₁-C₂₀ alkoxy} C₁-C₂₀ alkoxy group" in the present invention is a "C₁-C₂₀ alkoxy group" listed above substituted by one "(C₁-C₂₀ alkoxy) C₁-C₂₀ alkoxy group" listed above, preferably a {(C₁-C₆ alkoxy) C₁-C₆ alkoxy} C₁₋C₆ alkoxy group.

In the present invention, substitution in alkyl, alkenyl, alkynyl, alkoxy, alkenyloxy or alkynyloxy group by an oxo group does not include substitution of the carbon atom at position 1 by the oxo group. In other words, alkyl, alkenyl, alkynyl, alkoxy, alkenyloxy or alkynyloxy substituted by an oxo group does not include alkanoyl, alkenoyl, alkynoyl, alkanoyloxy, alkenoyloxy or alkynoyloxy.

The "Substituent group A" in the present invention is preferably the group consisting of a fluorine atom, a hydroxyl group, a C₁-C₂₀ alkoxy group, a C₁₂-C₁₄ alkenyloxy group, a C₁₂-C₁₄ alkanoyloxy group and a C₁₂-C₁₄ alkenoyloxy group, more preferably the group consisting of a dodecyloxy group, a tetradecyloxy group, a dodecenyloxy group, a tetradecenyloxy group, a dodecanoyloxy group, a tetradecanoyloxy group, a dodecenoyloxy group, a tetradecenoyloxy group and an octadecenoyl group.

In the above formula (I), the position of substitution by the Substituent group A is preferably position 3.

The compound of the above formula (I) may be formed into a salt, and preferred examples of such salts include alkali metal salts or alkaline earth metal salts such as sodium salts, potassium salts, magnesium salts or calcium salts; and salts of organic base such as triethylamine salts or trimethylamine salts.

In addition, when the compound (I) of the present invention is left in the atmosphere, the compound (I) may absorb moisture or water is adsorbed thereto to form a hydrate, and the present invention encompasses such salts.

Further, the compound (I) of the present invention may absorb certain kinds of solvent to form a solvate, and the present invention encompasses such salts.

The compound of the above formula (I) has stereoisomers containing some asymmetric carbons in its molecule with S-configuration or R-configuration, and the present invention encompasses each of them and a mixture thereof.

### Advantages of the Invention

The left-side glucose lipid A analogue of the present invention exhibits excellent macrophage activity inhibitory action and is useful as an anti-inflammatory agent, an anti-autoimmune disease agent, an immunosuppressive agent, an antiseptic agent or a prognosis-improving agent after coronary artery bypass surgery.

### Best Mode for Carrying out the Invention

The compound of the formula (I) of the present invention can be produced by the following method using a known compound (II) as a starting material.

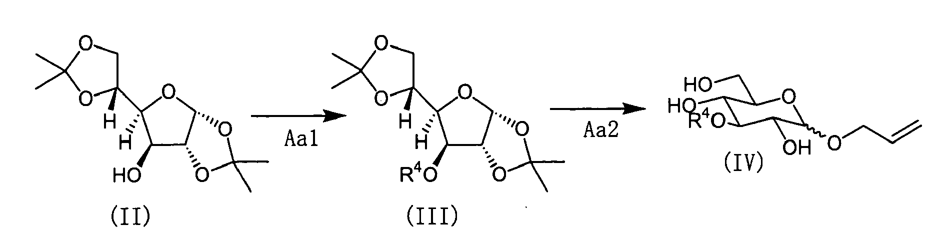

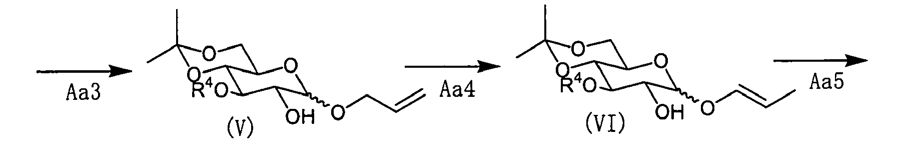

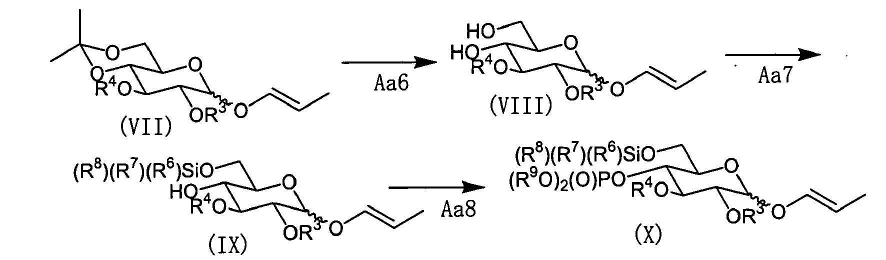

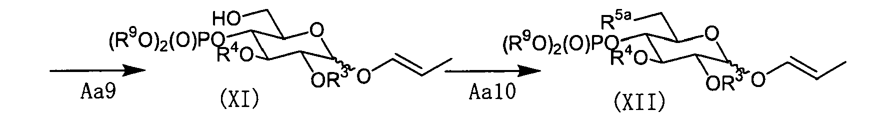

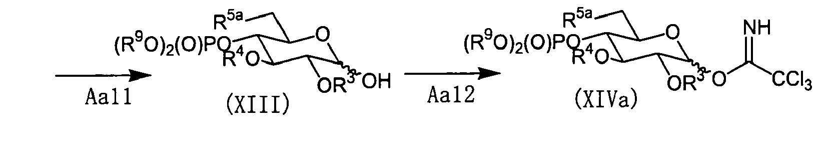

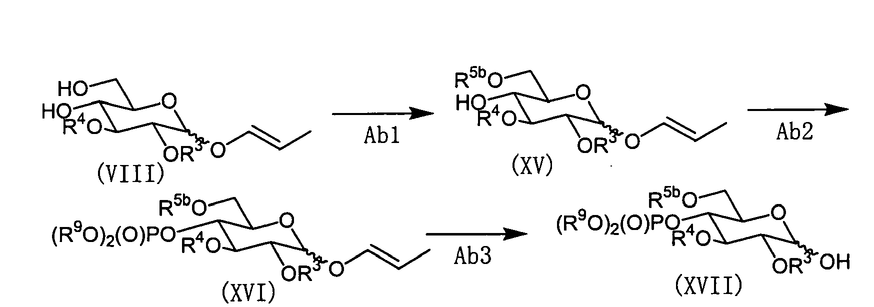

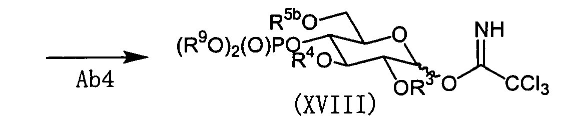

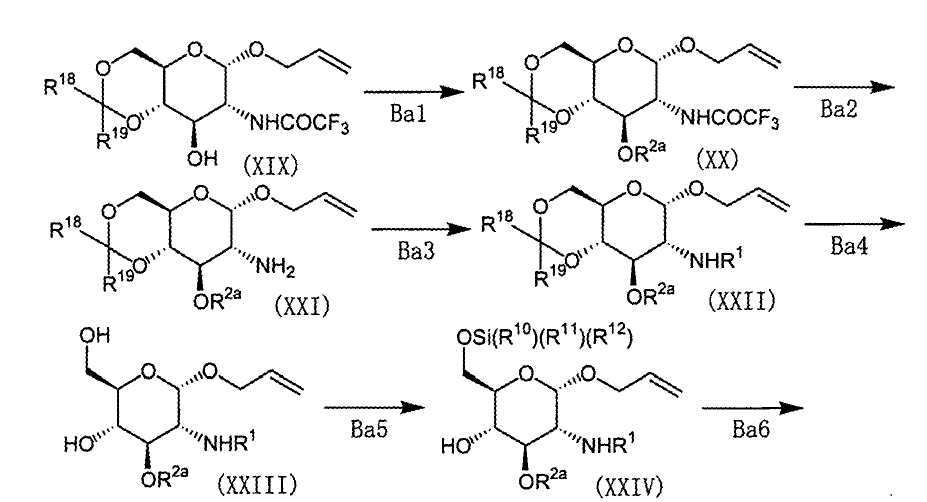

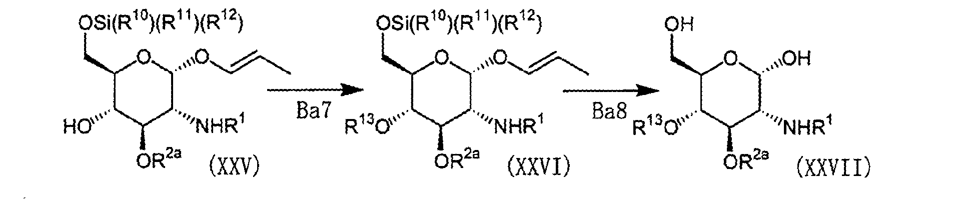

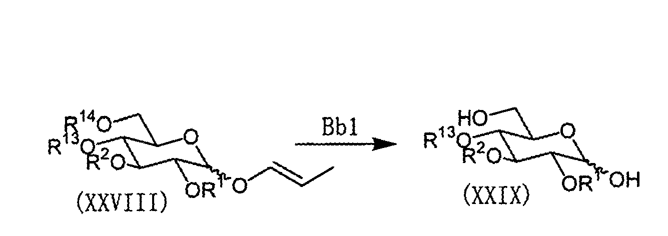

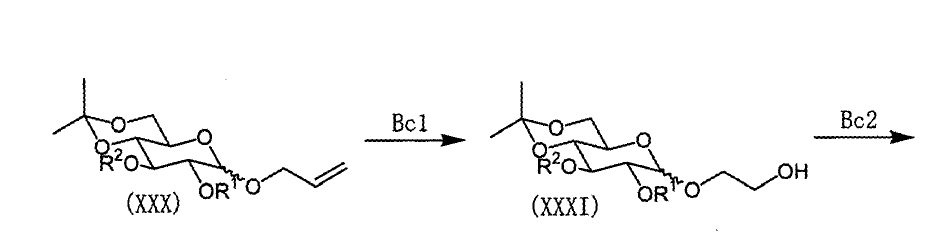

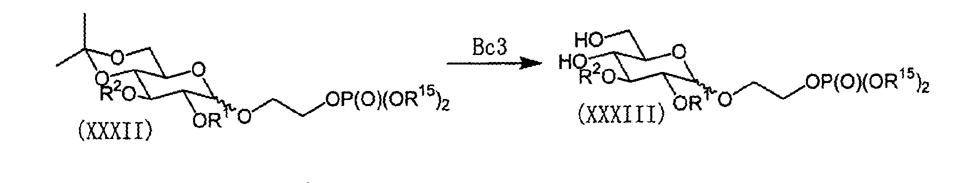

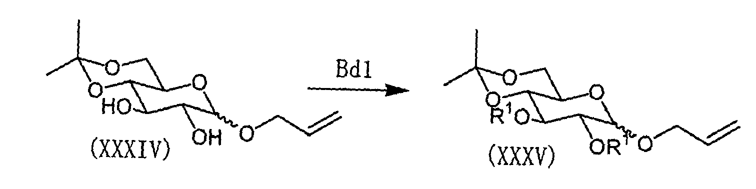

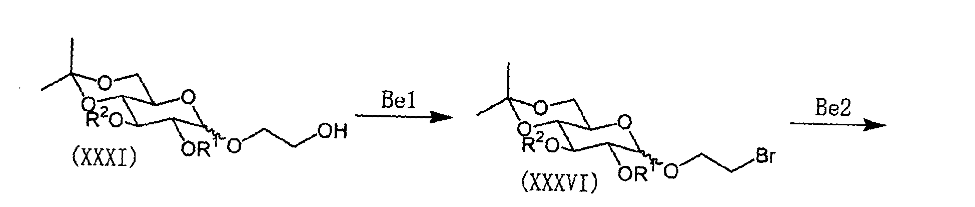

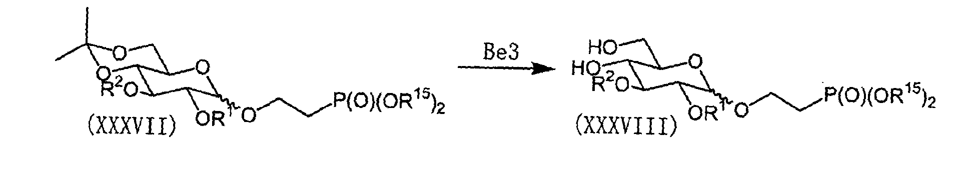

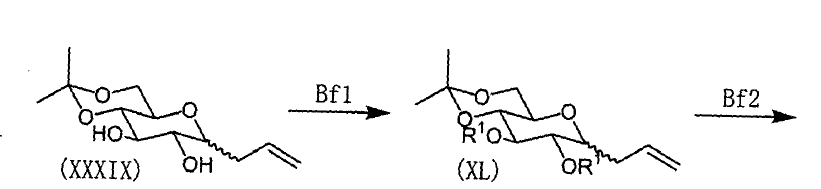

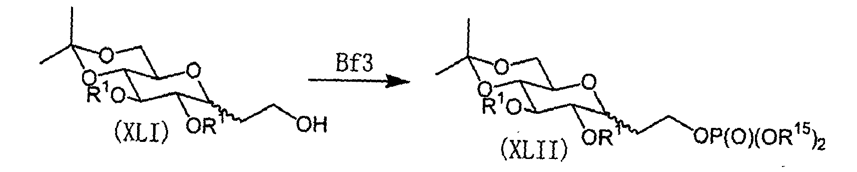

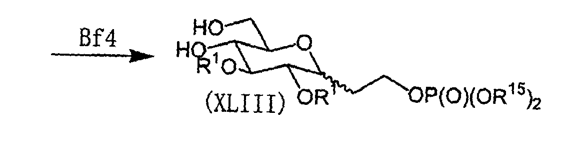

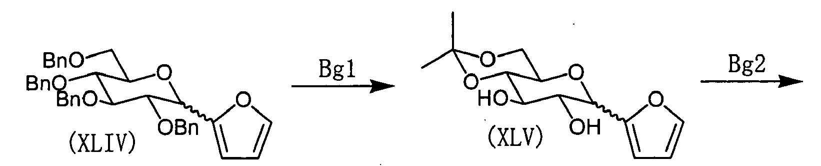

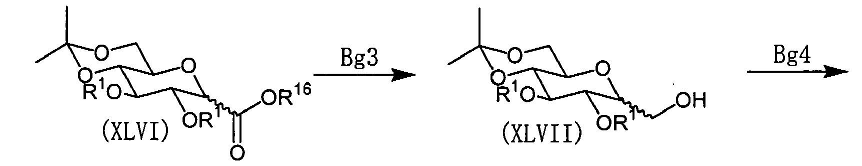

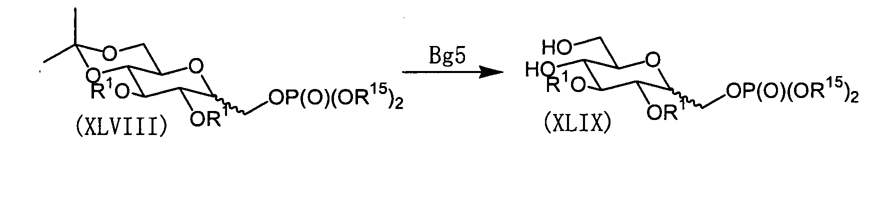

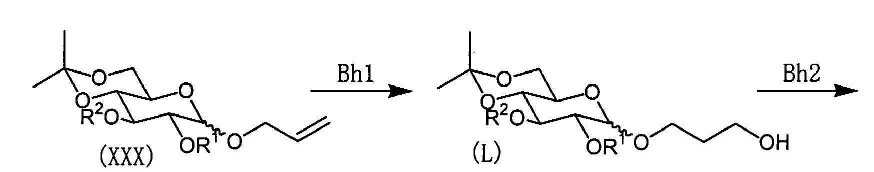

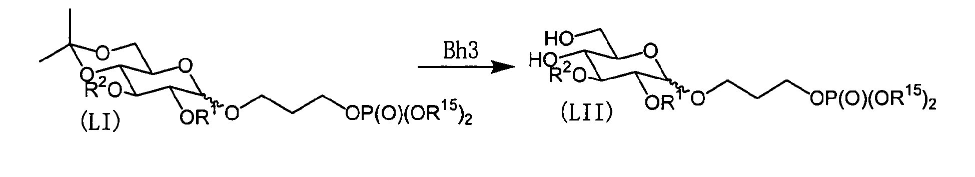

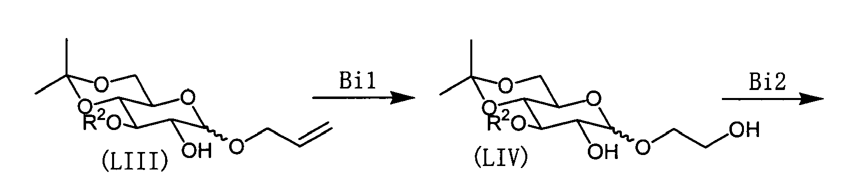

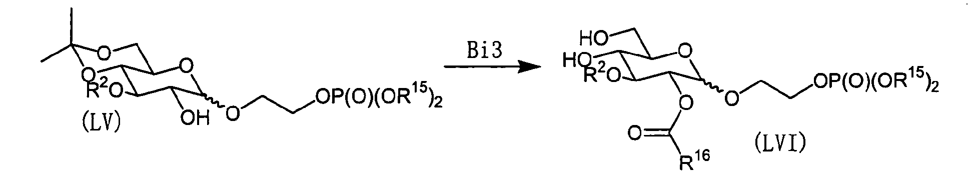

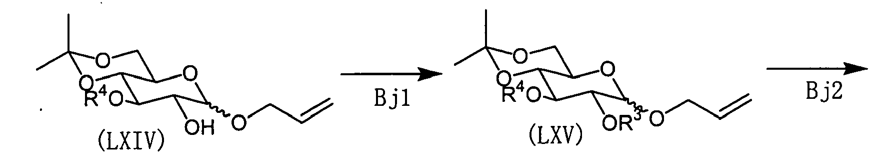

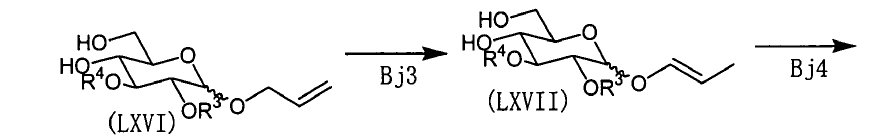

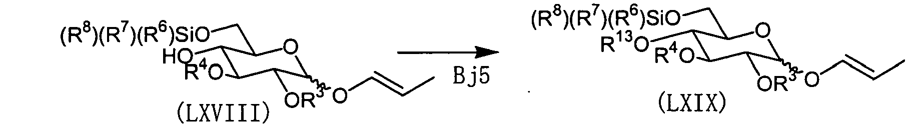

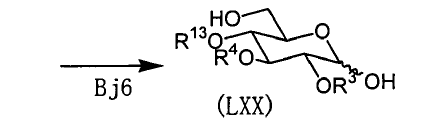

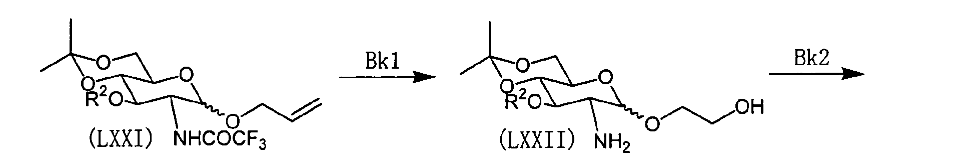

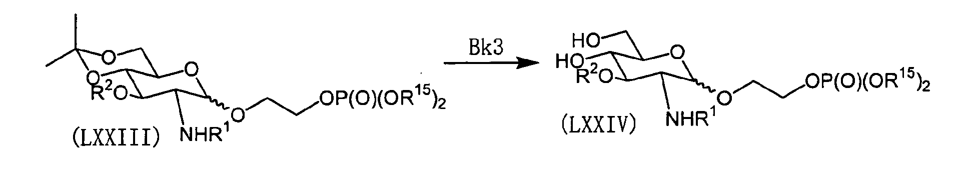

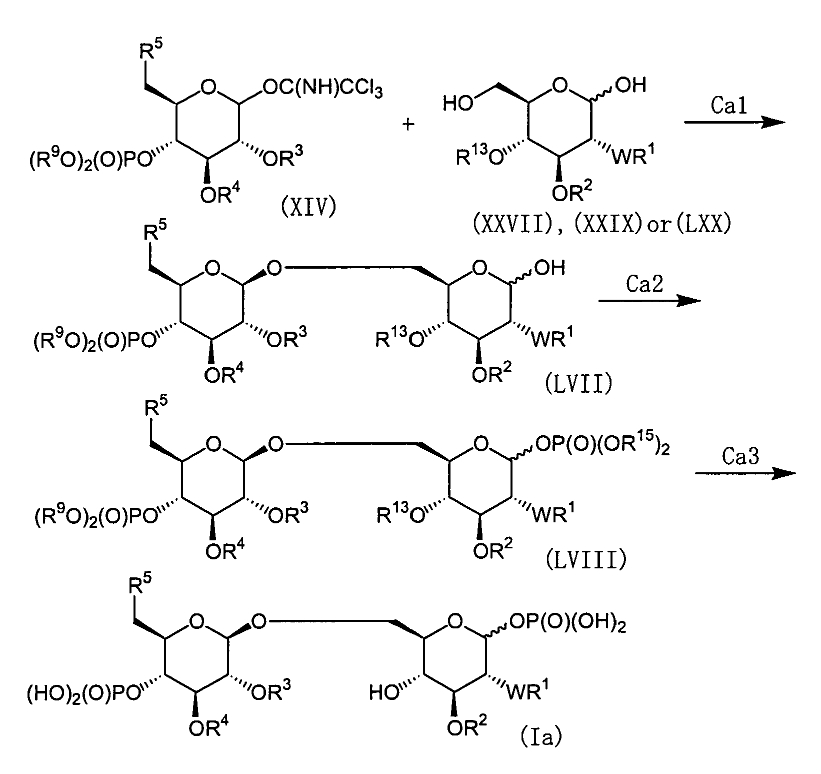

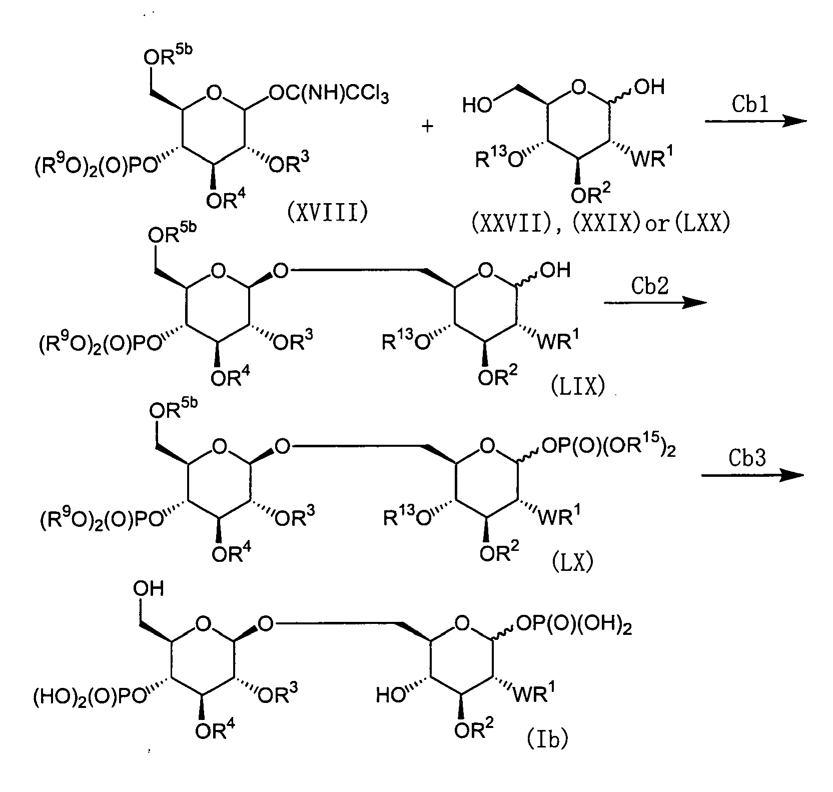

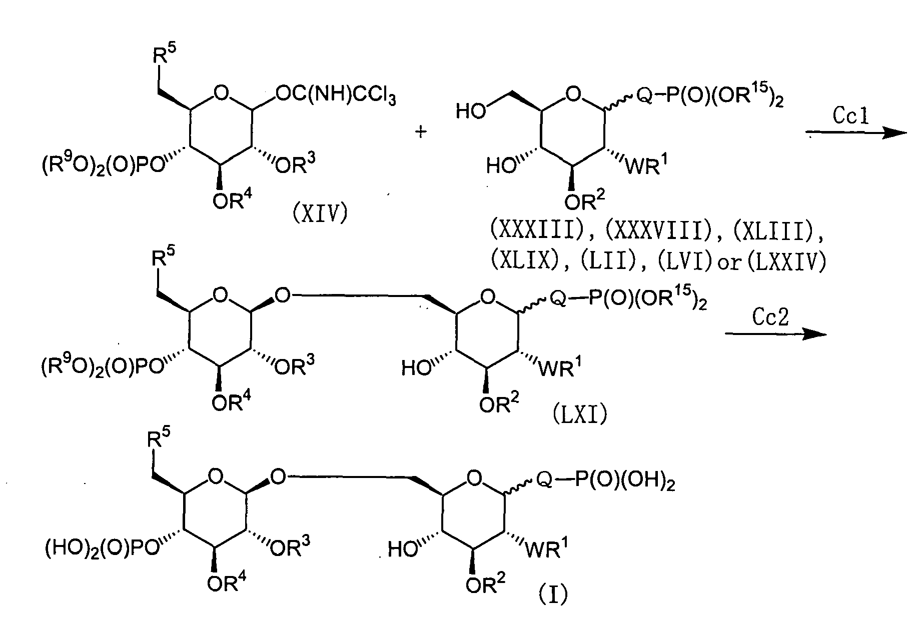

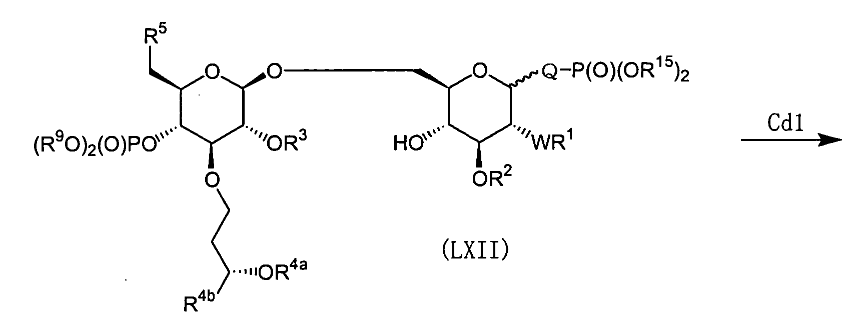

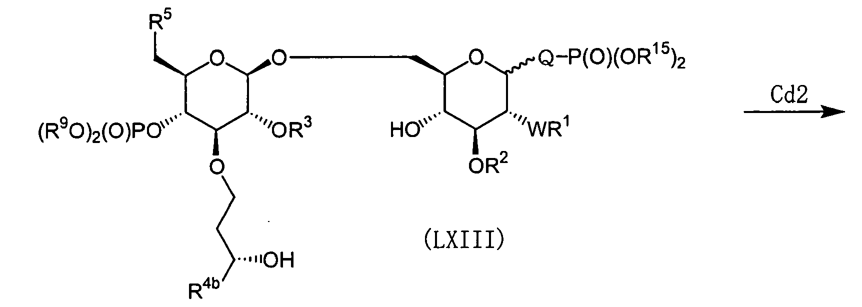

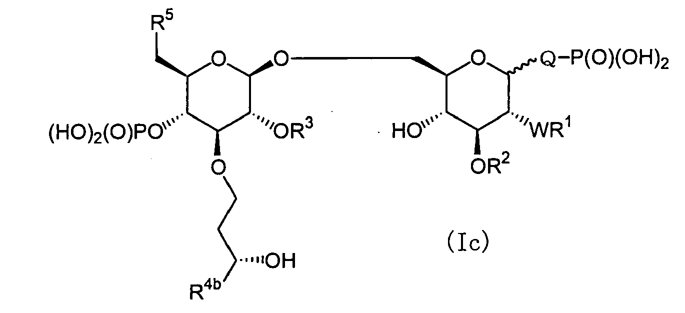

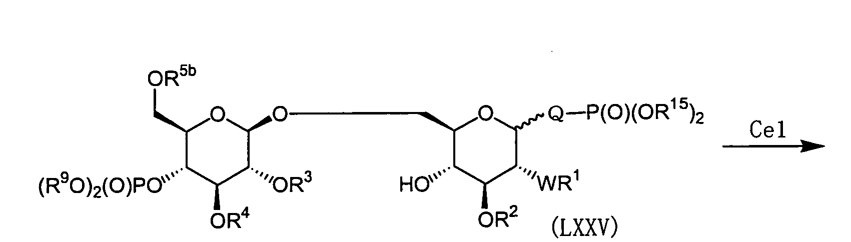

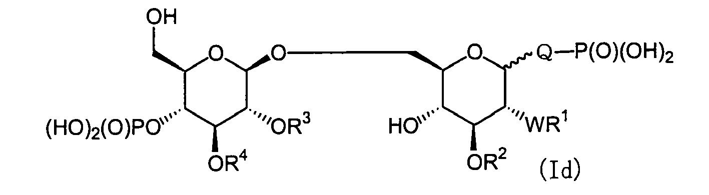

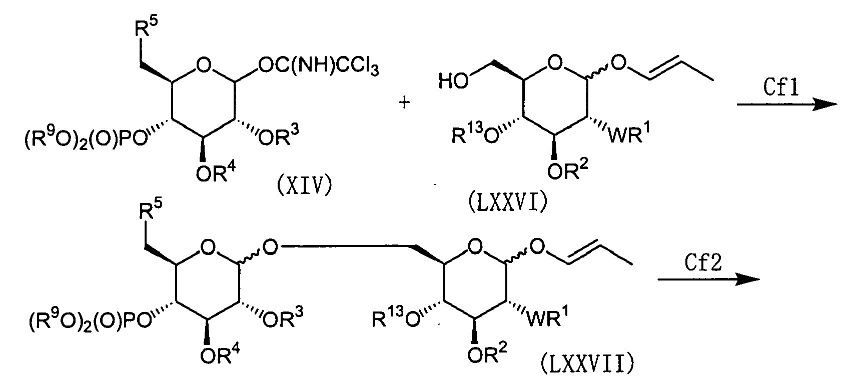

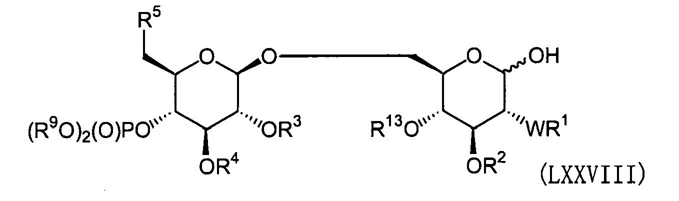

In the above steps, Q, R¹, R², R³, R⁴, R⁵ and W have the same meaning as described above.
R^{2a} has the same meaning as defined in R², except for the hydrogen atom.
R^{4a} is a p-methoxybenzyl group.
R^{4b} is a C₁-C₁₇ alkyl group, a C₂-C₁₇ alkenyl group or a C₂-C₁₇ alkynyl group.
R^{5a} has the same meaning as defined in R⁵, except for the non-protected hydroxyl group.
R^{5b} is a protective group of a hydroxyl group, preferably a benzyloxycarbonyl group or an allyloxycarbonyl group.
R⁶, R⁷, R⁸, R¹⁰, R¹¹ and R¹², which may be the same or different, represent a C₁-C₄ alkyl group or a C₆-C₁₀ aryl group.
R⁹ and R¹⁵, which may be the same or different, represent an allyl group, a C₆-C₁₀ aryl group which may be substituted or a C₇-C₁₁ aralkyl group which may be substituted, preferably an allyl group, a phenyl group or a benzyl group.
R¹³ represents an allyloxycarbonyl group.
R¹⁴ represents a hydrogen atom or a tri(C₁-C₆ alkyl)silyl group.
R¹⁶ represents a C₁-C₆ alkyl group.

The processes of producing the compound (I) of the present invention include processes A to C.
(1) The process A is a process for producing an intermediate (XIVa) or (XVIII).
(2) The process B is a process for producing an intermediate (XXVII), (XXIX), (XXXIII), (XXXV), (XXXVIII), (XLIII), (XLIX), (LII), (LVI), (LXX) or (LXXIV).
(3) The process C is a process for producing the target compound (Ia), (Ib), (I) or (Ic) by condensing the intermediate which can be produced by the process A and the intermediate which can be produced by the process B.

In the following, each step is described.

### (1) Process A

### (1-1) Process a

This process produces an intermediate (XIVa) used for producing a compound (I) in which R⁵ is a group other than a hydrogen atom.

### (Step Aa1)

This step is for producing a compound (III) in which the hydroxyl group at position 3 of a diacetone-D-glucose compound (II) is alkylated (including introduction of an alkenyl group or an alkynyl group; the same definition applies in the following description of the production steps) or acylated. The alkylation is accomplished by the following method (a) and the acylation is accomplished by the following method (b).

### (a) Alkylation

This step is accomplished by reacting the compound (II) with an alkylating agent in an inert solvent in the presence of a base.

Examples of solvents to be used include ethers such as dioxane and tetrahydrofuran; amides such as formamide and dimethylformamide; and halogenated hydrocarbons such as dichloromethane. The solvent is preferably dimethylformamide.

Examples of bases to be used include alkali metal hydroxides such as sodium hydroxide and potassium hydroxide; alkyllithiums such as n-butyllithium and t-butyllithium; and alkali metal hydrides such as potassium hydride and sodium hydride. The base is preferably sodium hydride.

Examples of alkylating agents to be used include halogenated hydrocarbons and sulfonic esters, and the alkylating agent is preferably bromide (R⁴Br), iodide (R⁴I) or methanesulfonic acid ester (R⁴OSO₂Me)

The reaction is performed at a reaction temperature of generally 0°C to 100°C, preferably 20°C to 60°C.

The reaction time varies depending on the reaction temperature, starting materials, reagents or solvents to be used, and is generally 20 minutes to 48 hours, preferably 2 to 24 hours.

After completion of the reaction, the target compound of the reaction is collected from the reaction mixture by a conventional method. For example, the target compound can be obtained by neutralizing the reaction mixture, then concentrating the mixture, adding an organic solvent immiscible with water such as ethyl acetate thereto, and after washing with water, separating the organic layer containing the target compound, drying the same over anhydrous magnesium sulfate or the like, and removing the solvent.

The obtained compound may be further purified by a conventional method such as recrystallization or silica gel chromatography, if necessary.

### (b) Acylation

This step is accomplished by reacting a compound (II) with an acylating agent such as carboxylic acid or acid chloride in an inert solvent in the presence or absence of a condensing agent and a base.

Examples of solvents to be used include ethers such as tetrahydrofuran and dioxane; halogenated hydrocarbons such as methylene chloride, chloroform and carbon tetrachloride; and amides such as N,N-dimethylformamide. The solvent is preferably methylene chloride.

Examples of condensing agents used in the reaction with carboxylic acid include carbodiimides such as 1,3-dicyclohexylcarbodiimide, 1,3-diisopropylcarbodiimide and 1-[3-(dimethylamino)propyl]-3-ethylcarbodiimide hydrochloride (WSCI). The condensing agent is preferably 1,3-dicyclohexylcarbodiimide or WSCI.

Examples of bases used in the reaction with acid chloride include organic bases such as pyridine, dimethylaminopyridine and triethylamine. The base is preferably 4-dimethylaminopyridine or triethylamine.

Examples of acylating agents to be used include carboxylic acid represented by the formula R^{2'}OH or acid chloride represented by the formula R^{4'}Cl (wherein R^{4'} represents an alkanoyl group, an alkenoyl group or an alkynoyl group in the definition of R⁴). When the acylating agent contains a hydroxyl group or an oxo group, those groups may be protected, and for example, the oxo group is protected to form a ketal group.

The reaction temperature is generally 0°C to 100°C, preferably 15°C to 25°C (room temperature).

The reaction time is generally 20 minutes to 24 hours, preferably 1 to 5 hours.

After completion of the reaction, the target compound of the reaction is collected from the reaction mixture by a conventional method. For example, the target compound can be obtained by filtrating the reaction mixture, adding an organic solvent immiscible with water such as ethyl acetate thereto, and after washing with water, separating the organic layer containing the target compound, drying the same over anhydrous magnesium sulfate or the like, and removing the solvent.

The obtained compound may be further purified by a conventional method such as recrystallization or silica gel chromatography, if necessary.

### (Step Aa2)

This step is for obtaining a compound (IV) by heating the compound (III) in an allyl alcohol solvent in the presence of hydrochloric acid gas.

Referring to the reaction temperature, the reaction is generally performed at room temperature to heating reflux temperature of the solvent, preferably room temperature to 80°C.

The reaction time is generally 20 minutes to 24 hours, preferably 30 minutes to 5 hours.

### (Step Aa3)

This step is for obtaining a compound (V) by bridging isopropylidene to the hydroxyl groups at positions 4 and 6 of the compound (IV). The step is accomplished by reacting the compound (IV) with 2,2-dimethoxypropane in an inert solvent in the presence of an acid catalyst.

Examples of solvents to be used include amides such as dimethylformamide.

Examples of acid catalysts to be used include p-toluenesulfonic acid and camphorsulfonic acid.

The reaction temperature is generally room temperature to 50°C.

The reaction time is generally 20 minutes to 24 hours.

After completion of the reaction, the target compound (V) of the reaction is collected from the reaction mixture by a conventional method. For example, the target compound can be obtained by concentrating the reaction mixture, adding an organic solvent immiscible with water such as ethyl acetate thereto, and after washing with water, separating the organic layer containing the target compound, drying the same over anhydrous magnesium sulfate or the like, and removing the solvent.

The obtained compound may be further purified by a conventional method such as recrystallization or silica gel chromatography, if necessary.

### (Step Aa4)

This step is for producing a compound (VI) by converting the double bond in the allyl group of the compound (V) to an enol ether form. The step is accomplished by reacting the compound (V) with a base or an iridium complex in an inert solvent.

Examples of solvents to be used include ethers such as tetrahydrofuran and dioxane; and sulfoxides such as dimethyl sulfoxide. When a base is used, the solvent is preferably dimethyl sulfoxide, and when an iridium complex is used, the solvent is preferably tetrahydrofuran.

Examples of bases to be used include alkali metal alkoxides such as potassium tert-butoxide, sodium methoxide, sodium ethoxide. The base is preferably potassium tert-butoxide.

Examples of iridium complexes to be used include (1,5-cyclooctadiene)bis(methyldiphenylphosphine)iridium (I) hexafluorophosphate.

The reaction temperature is generally room temperature to heating reflux temperature of the solvent in the case of using a base, and is generally 0°C to 80°C, preferably room temperature in the case of using an iridium complex.

The reaction time is generally 1 hour to 24 hours, preferably 16 hours in the case of using a base.

After completion of the reaction, the target compound (VI) of the reaction is collected from the reaction mixture by a conventional method. For example, the target compound can be obtained by concentrating the reaction mixture, adding an organic solvent immiscible with water such as ethyl acetate thereto, and after washing with water, separating the organic layer containing the target compound, drying the same over anhydrous magnesium sulfate or the like, and removing the solvent.

The obtained compound may be further purified by a conventional method such as recrystallization or silica gel chromatography, if necessary.

### (Step Aa5)

This step is for obtaining a compound (VII) by alkylating or acylating the hydroxyl group at position 2 of the compound (VI). The step is accomplished by performing the reaction under the same conditions as in Step Aa1.

### (Step Aa6)

This step is for producing a compound (VIII) by deprotection of isopropylidene bridged to the hydroxyl groups at positions 4 and 6 of the compound (VII). The step is accomplished by reacting the compound (VII) with an acid catalyst or NBS in an 80% aqueous acetic acid solution or an alcohol solvent.

Examples of solvents to be used include alcohols such as methanol and ethanol.

Examples of acid catalysts to be used include organic acids such as p-toluenesulfonic acid and camphorsulfonic acid; and inorganic acids such as hydrochloric acid and sulfuric acid.

The reaction temperature is generally room temperature to 100°C in the aqueous acetic acid solvent, and is generally 0°C to 50°C, preferably room temperature in alcohol.

The reaction time is generally 6 minutes to 24 hours, preferably 6 minutes to 3 hours.

After completion of the reaction, the target compound (VIII) of the reaction is collected from the reaction mixture by a conventional method. For example, the target compound can be obtained by concentrating the reaction mixture, adding an organic solvent immiscible with water such as ethyl acetate thereto, and after washing with water, separating the organic layer containing the target compound, drying the same over anhydrous magnesium sulfate or the like, and removing the solvent.

The obtained compound may be further purified by a conventional method such as recrystallization or silica gel chromatography, if necessary.

### (Step Aa7)

This step is for producing a compound (IX) by protecting the primary hydroxyl group of the diol, which is the compound (VIII), by a silyl group. The step is accomplished by reacting the compound (VIII) with a silylating agent in an inert solvent in the presence of a base.

Examples of solvents to be used include ethers such as tetrahydrofuran and dioxane; and halogenated hydrocarbons such as methylene chloride, chloroform and carbon tetrachloride. The solvent is preferably methylene chloride or tetrahydrofuran.

Examples of bases to be used include organic bases such as pyridine, dimethylaminopyridine and triethylamine.

Examples of silylating agent to be used include trialkylsilyl halide groups such as a tert-butyldimethylsilyl chloride group.

The reaction temperature is generally 0°C to 80°C, preferably room temperature.

The reaction time is generally 6 minutes to 24 hours.

After completion of the reaction, the target compound (IX) of the reaction is collected from the reaction mixture by a conventional method. For example, the target compound can be obtained by concentrating the reaction mixture, adding an organic solvent immiscible with water such as ethyl acetate thereto, and after washing with water, separating the organic layer containing the target compound, drying the same over anhydrous magnesium sulfate or the like, and removing the solvent.

The obtained compound may be further purified by a conventional method such as recrystallization or silica gel chromatography, if necessary.

### (Step Aa8)

This step is for producing a compound (X) by converting the compound (IX) to its phosphite and oxidizing the same. The step is accomplished by reacting the compound (IX) with diisopropylphosphoramidite in an inert solvent in the presence of 1H-tetrazole, and allowing the resultant to react with an oxidant in an inert solvent.

Examples of solvents to be used include ethers such as tetrahydrofuran and dioxane; and halogenated hydrocarbons such as methylene chloride, chloroform, carbon tetrachloride, dichloroethane and chlorobenzene.

Examples of oxidants to be used include peroxides such as hydrogen peroxide, m-chloroperbenzoic acid and OXONE® (potassium peroxymonosulfate).

The reaction temperature is generally 0°C to 100°C, preferably room temperature.

The reaction time is generally 6 minutes to 24 hours.

After completion of the reaction, the target compound (X) of the reaction is collected from the reaction mixture by a conventional method. For example, the target compound can be obtained by concentrating the reaction mixture, adding an organic solvent immiscible with water such as ethyl acetate thereto, and after washing with water, separating the organic layer containing the target compound, drying the same over anhydrous magnesium sulfate or the like, and removing the solvent.

The obtained compound may be further purified by a conventional method such as recrystallization or silica gel chromatography, if necessary.

### (Step Aa9)

This step is for producing a compound (XI), which is primary alcohol, by deprotection of the silyl protective group in the compound (X). The step is accomplished by reacting the compound (X) with an acid catalyst in an inert solvent.

Examples of solvents to be used include ethers such as tetrahydrofuran and dioxane; nitriles such as acetonitrile; and halogenated hydrocarbons such as methylene chloride, chloroform, carbon tetrachloride, dichloroethane and chlorobenzene.

Examples of acid catalysts to be used include inorganic acids such as hydrochloric acid and hydrofluoric acid; and organic acids such as p-toluenesulfonic acid.

The reaction temperature is generally 20°C to 50°C, preferably room temperature.

The reaction time is generally 6 minutes to 2 hours, preferably 15 minutes.

After completion of the reaction, the target compound (XI) of the reaction is collected from the reaction mixture by a conventional method. For example, the target compound can be obtained by concentrating the reaction mixture, adding an organic solvent immiscible with water such as ethyl acetate thereto, and after washing with water, separating the organic layer containing the target compound, drying the same over anhydrous magnesium sulfate or the like, and removing the solvent.

The obtained compound may be further purified by a conventional method such as recrystallization or silica gel chromatography, if necessary.

### (Step Aa10)

This step is for producing a compound (XII) which is obtained by converting the hydroxyl group at position 6 of the compound (XI). The step is accomplished by method 1) when R^{5a} is a C₁-C₆ alkoxy group which may have an oxo group, a C₂₋C₆ alkenyloxy group which may have an oxo group, or a C₂-C₆ alkynyloxy group which may have an oxo group, by method 2) particularly when R^{5a} is a methoxy group, or by method 3) when R^{5a} is a halogen atom.
1) When R^{5a} is a C₁-C₆ alkoxy group which may have an oxo group, a C₂-C₆ alkenyloxy group which may have an oxo group or a C₂-C₆ alkynyloxy group which may have an oxo group:
   This step is accomplished by reacting the compound (XI) with an alkylating agent in an inert solvent in the presence of a base or a silver oxide (II)(AgO).
   The solvent to be used is not particularly limited as long as it does not inhibit the reaction and dissolves more than a certain amount of starting materials.

Examples thereof include aliphatic hydrocarbons such as hexane, heptane and ligroin; aromatic hydrocarbons such as benzene, toluene and xylene; halogenated hydrocarbons such as methylene chloride, chloroform, carbon tetrachloride, dichloroethane and chlorobenzene; esters such as ethyl acetate, propyl acetate and diethyl carbonate; ethers such as diethyl ether, diisopropyl ether, tetrahydrofuran and dioxane; and nitriles such as acetonitrile and isobutyronitrile; and amides such as formamide, N,N- dimethylformamide and N,N-dimethylacetamide. The solvent is preferably an ether.

Examples of bases to be used include alkali metal carbonates such as sodium carbonate and potassium carbonate; alkali metal hydrogen carbonates such as sodium hydrogen carbonate and potassium hydrogen carbonate; alkali metal hydrides such as sodium hydride and potassium hydride; and organic bases such as N-methylmorpholine, triethylamine, tributylamine, diisopropylethylamine, dicyclohexylamine, N-methylpiperizine, pyridine, picoline, 4-(N,N-dimethylamino)pyridine, 2,6-di(t-butyl)-4-methylpyridine, N,N-dimethylaniline, N,N-diethylaniline, 1,5-diazabicyclo[4.3.0]nona-5-ene (DBN), 1,4-diazabicyclo [2.2.2]octane (DABCO) and 1,8-diazabicyclo[5.4.0]undeca-7-ene (DBU).

Preferred are organic bases and more preferred is DBN or DBU.

Examples of alkylating agents to be used include the formula R^{5a'}Z (wherein R^{5a'} has the same meaning as defined in the aforementioned R^{5a} except for the halogen atom, Z is an iodine atom, a bromine atom, a chlorine atom, a para-toluenesulfonyloxy group or a methanesulfonyloxy group).

The reaction is performed at a reaction temperature of generally 0°C to 100°C, preferably 0°C to 30°C.

The reaction time is generally 10 minutes to 24 hours, preferably 1 to 18 hours.
2) When R^{5a} is a methoxy group:
   This step is accomplished by reacting the compound (XI) with trimethyloxonium tetrafluoroborate in an inert solvent in the presence of a base.
   Examples of solvents to be used include ethers such as ether, dioxane and tetrahydrofuran; halogenated hydrocarbons such as methylene chloride, chloroform and carbon tetrachloride; and amides such as formamide, N,N-dimethylformamide and N,N-dimethylacetamide. The solvent is preferably methylene chloride.
   Preferred examples of bases to be used include 2,6-di-tert-butyl-4-methylpyridine.
   The reaction is performed at a reaction temperature of generally -50°C to 100°C, preferably 0°C to 30°C.
   The reaction time is generally 1 to 24 hours, preferably 2 to 5 hours.
   After completion of the reaction, the target compound (XII) of the reaction is collected from the reaction mixture by a conventional method. For example, the target compound can be obtained by neutralizing the reaction mixture, then concentrating the mixture, adding an organic solvent immiscible with water such as ethyl acetate thereto, and after washing with water, separating the organic layer containing the target compound, drying the same over anhydrous magnesium sulfate or the like, and removing the solvent.
   The obtained compound may be further purified by a conventional method such as recrystallization or silica gel chromatography, if necessary.
3) When R^{5a} is a halogen atom:
   3-1) When R^{5a} is a fluorine atom:
      This step is accomplished by reacting the compound (XI) with a fluorinating agent in an inert solvent.
      Examples of solvents to be used include halogenated hydrocarbons such as methylene chloride and fluorotrichloromethane; and ethers such as ether and 1,2-dimethoxyethane, and preferred is methylene chloride.
      Examples of fluorinating agents to be used include diethylaminosulfur trifluoride (DAST).
      The reaction is performed at a reaction temperature of generally -78°C to 25°C, preferably 0°C to 25°C.
      The reaction time is generally 1 to 18 hours, preferably 1 to 5 hours.
   3-2) When R^{5a} is a chlorine atom or a bromine atom:
      This step is accomplished by reacting the compound (XI) with phosphorus trichloride, phosphorus tribromide, phosphoryl trichloride, phosphoryl tribromide, thionyl chloride or thionyl bromide in an inert solvent.
      Preferred examples of solvents to be used include halogenated hydrocarbons such as methylene chloride, chloroform and carbon tetrachloride.
      The reaction is performed at a reaction temperature of generally -50°C to 50°C, preferably -10°C to 30°C.
      The reaction time is generally 1 to 18 hours, preferably 1 to 5 hours.
   3-3) When R^{5a} is an iodine atom:
      This step is accomplished by reacting the compound (XI) with iodine and triphenyl phosphine.
      The solvent to be used is not particularly limited as long as it does not inhibit the reaction and dissolves more than a certain amount of starting materials. Examples thereof include aliphatic hydrocarbons such as hexane, heptane and ligroin; aromatic hydrocarbons such as benzene, toluene and xylene; halogenated hydrocarbons such as methylene chloride, chloroform, carbon tetrachloride, dichloroethane and chlorobenzene; esters such as ethyl acetate, propyl acetate and diethyl carbonate; ethers such as diethyl ether, diisopropyl ether, tetrahydrofuran and dioxane; alcohols such as methanol and ethanol; and amides such as formamide, N,N-dimethylformamide and N,N-dimethylacetamide. The solvent is preferably ether.
      The reaction is performed at a reaction temperature of generally -50°C to 100°C, preferably 0°C to 30°C.
      The reaction time is generally 1 to 18 hours, preferably 1 to 5 hours.
      After completion of the reaction, the target compound (XII) of the reaction is collected from the reaction mixture by a conventional method. For example, the target compound can be obtained by neutralizing the reaction mixture, then concentrating the mixture, adding an organic solvent immiscible with water such as ethyl acetate thereto, and after washing with water, separating the organic layer containing the target compound, drying the same over anhydrous magnesium sulfate or the like, and removing the solvent.
      The obtained compound may be further purified by a conventional method such as recrystallization or silica gel chromatography, if necessary.

### (Step Aa11)

This step is for producing a compound (XIII) by deprotection of the protective group of the hydroxyl group at position 1 of the compound (XII). The step is accomplished by reacting the compound (XII) with an acid catalyst in an inert solvent.

Examples of solvents to be used include halogenated hydrocarbons such as methylene chloride, chloroform, carbon tetrachloride, dichloroethane, chlorobenzene and dichlorobenzene; and nitriles such as acetonitrile and isobutyronitrile.

Examples of acid catalysts to be used include a 48% hydrofluoric acid aqueous solution.

The reaction temperature is generally 0°C to 100°C, preferably room temperature.

The reaction time is generally 6 minutes to 24 hours, preferably 16 hours.

### (Step Aa12)

This step is for producing a trichloroacetimidate compound (XIVa). The step is accomplished by allowing trichloroacetonitrile to react with the hydroxyl group at position 1 of the compound (XIII) in an inert solvent in the presence of a base.

Examples of solvents to be used include halogenated hydrocarbons such as methylene chloride, chloroform and carbon tetrachloride; ethers such as ether, dioxane and tetrahydrofuran; esters such as ethyl acetate; and nitriles such as acetonitrile. The solvent is preferably a halogenated hydrocarbon (methylene chloride).

Examples of bases to be used include organic bases such as 1,8-diazabicyclo[5,4,0]-7-undecene (DBU); and inorganic bases such as sodium hydride, potassium carbonate and cesium carbonate. The base is preferably DBU or cesium carbonate.

The reaction is performed at a reaction temperature of generally -25°C to 50°C, preferably 0°C to 25°C.

The reaction time is generally 10 minutes to 24 hours, preferably 30 minutes to 2 hours.

After completion of the reaction, the target compound (XIVa) of the reaction is collected from the reaction mixture by a conventional method. For example, the target compound can be obtained by neutralizing the reaction mixture, then concentrating the mixture, adding an organic solvent immiscible with water such as ethyl acetate thereto, and after washing with water, separating the organic layer containing the target compound, drying the same over anhydrous magnesium sulfate or the like, and removing the solvent.

### (1-2) Process b

This process produces an intermediate (XVIII) used for producing a compound (I) in which R⁵ is a protected hydroxyl group.

### (Step Ab1)

This step is for producing a compound (XV) which is obtained by selectively protecting the hydroxyl group at position 6 of the compound (VIII) as desired.

When an unsaturated bond is present in R³, R⁴ and R^{5b}, an allyl group or an allyloxycarbonyl group removable without employing a reduction reaction is used. When no unsaturated bond is present in R³, R⁴ or R^{5b}, a protecting reaction may be immediately performed.

This reaction is accomplished by reacting a protecting agent with the compound (VIII) in an inert solvent in the presence of a base.

Examples of solvents to be used include halogenated hydrocarbons such as methylene chloride, chloroform and carbon tetrachloride; ethers such as ether, dioxane and tetrahydrofuran; and esters such as ethyl acetate. The solvent is preferably methylene chloride.

Examples of bases to be used include organic bases such as pyridine, dimethylaminopyridine, triethylamine and N,N-dimethylaniline. The base is preferably pyridine.

The protecting agent to be used may be those such that the hydroxyl group at position 6 can be restored by a deprotection reaction at a good yield. Examples thereof include alkyloxycarbonyl halides such as tert-butoxycarbonyl chloride, aralkyloxycarbonyl halides such as benzyloxycarbonyl chloride and alkenyloxycarbonyl halides such as allyloxycarbonyl chloride. When no unsaturated bond is present in R³, R⁴ or R^{5b}, the protecting agent is preferably benzyloxycarbonyl chloride. When an unsaturated bond is present in R³, R⁴ and R^{5b}, the protecting agent is preferably allyloxycarbonyl chloride.

The reaction is performed at a reaction temperature of generally -50°C to 50°C, preferably -10°C to 30°C.

The reaction time is generally 10 minutes to 24 hours, preferably 30 minutes to 5 hours.

After completion of the reaction, the target compound (XV) of the reaction is collected from the reaction mixture by a conventional method. For example, the target compound can be obtained by neutralizing the reaction mixture, then concentrating the mixture, adding an organic solvent immiscible with water such as ethyl acetate thereto, and after washing with water, separating the organic layer containing the target compound, drying the same over anhydrous magnesium sulfate or the like, and removing the solvent.

The obtained compound may be further purified by a conventional method such as recrystallization or silica gel chromatography, if necessary.

### (Step Ab2)

This step is for producing a compound (XVI) by converting the compound (XV) to its phosphite and oxidizing the same. The step is accomplished by performing the reaction under the same conditions as in Step Aa8.

### (Step Ab3)

This step is for producing a compound (XVII) by deprotection of the protective group of the hydroxyl group at position 1 of the compound (XVI). The step is accomplished by performing the reaction under the same conditions as in Step Aa 11.

### (Step Ab4)

This step is for producing a trichloroacetimidate compound (XVIII), and is accomplished by performing the reaction under the same conditions as in Step Aa12.

### (2) Process B

### (2-1) Process a

This process produces an intermediate (XXVII) used for producing a compound (I) in which W is an NH group.

### (Step Ba1)

This step is for producing a compound (XX) which is obtained by alkylating or acylating the hydroxyl group at position 3 of the compound (XIX). The step is accomplished by performing the reaction under the same conditions as in Step Aa1.

### (Step Ba2)

This step is for producing a compound (XXI) by deprotecting position 2 of the compound (XX).

The step is for deprotection by removing trifluoroacetamide at position 2 of the compound (XIX) in an inert solvent under an alkaline condition.

Examples of solvents to be used include alcohols such as methanol and ethanol; ethers such as diethyl ether and tetrahydrofuran; and nitriles such as acetonitrile. The solvent is preferably an alcohol (ethanol).

Examples of alkali to be used include alkali metal hydrogen carbonates such as sodium hydrogen carbonate and potassium hydrogen carbonate; alkali metal hydroxides such as sodium hydroxide and potassium hydroxide; and alkali metal alkoxides such as sodium methoxide and sodium ethoxide. The alkali is preferably alkali metal hydroxide.

The reaction temperature is generally 0°C to 100°C, preferably 25°C to the reflux temperature.

The reaction time is generally 30 minutes to 24 hours, preferably 1 to 16 hours.

### (Step Ba3)

This step is for producing a compound (XXII) by amidating the amino group of the compound (XXI) with carboxylic acid, or acid halide or acid anhydride thereof.

Examples of condensing agents used for the condensation reaction with carboxylic acid include carbodiimides such as DCC and WSCI.

Examples of bases used for the amide forming reaction with acid halide include alkali metal carbonates such as sodium carbonate, potassium carbonate and lithium carbonate; alkali metal hydrogen carbonates such as sodium hydrogen carbonate, potassium hydrogen carbonate and lithium hydrogen carbonate; and organic bases such as pyridine, dimethylaminopyridine and triethylamine.

Examples of solvents used for the condensation reaction include halogenated hydrocarbons such as methylene chloride, chloroform, carbon tetrachloride, dichloroethane, chlorobenzene and dichlorobenzene; ethers such as diethyl ether, diisopropyl ether, tetrahydrofuran, dioxane, dimethoxyethane and diethylene glycol dimethyl ether; nitriles such as acetonitrile and isobutyronitrile; amides such as formamide, N,N-dimethylformamide, N,N-dimethylacetamide, N-methyl-2-pyrrolidone, N-methylpyrrolidinone and hexamethylphosphorotriamide; water; and mixed solvents thereof.

The reaction time of the condensation reaction is generally 0°C to 100°C.

The reaction temperature of the condensation reaction is generally 6 minutes to 24 hours.

### (Step Ba4)

This step is for producing a compound (XXIII) by deprotection of isopropylidene bridged to the hydroxyl groups at positions 4 and 6 of the compound (XXII). The step is accomplished by performing the reaction under the same conditions as in Step Aa6.

### (Step Ba5)

This step is for producing a compound (XXIV) by protecting the primary hydroxyl group of the diol, which is the compound (XXIII), by a silyl group. The step is accomplished by performing the reaction under the same conditions as in Step Aa7.

### (Step Ba6)

This step is for producing a compound (XXV) by converting the double bond in the allyl group of the compound (XXIV) to an enol ether form. The step is accomplished by performing the reaction under the same conditions as in Step Aa4.

### (Step Ba7)

This step is for producing a compound (XXVI) by protecting the secondary hydroxyl group of the compound (XXV) by an allyloxycarbonyl group. The step is accomplished by reacting the compound (XXV) with a carbonating agent and further with alcohol in an inert solvent in the presence of a base.

Examples of solvents to be used include ethers such as tetrahydrofuran and dioxane; aromatic hydrocarbons such as benzene, toluene and xylene; and halogenated hydrocarbons such as methylene chloride, chloroform, carbon tetrachloride, dichloroethane and chlorobenzene.

Examples of bases to be used include organic bases such as pyridine, dimethylaminopyridine and triethylamine.

Examples of carbonating agents to be used include triphosgene.

Examples of alcohols to be used include allyl alcohol.

The reaction temperature is generally -20°C to 50°C, preferably 0°C.

The reaction time after addition of the carbonating agent and before addition of alcohol is generally 10 minutes to 1 hour, preferably 10 minutes. The reaction time after addition of alcohol is generally 10 minutes to 1 hour, preferably 1 hour.

After completion of the reaction, the target compound (XXVI) of the reaction is collected from the reaction mixture by a conventional method. For example, the target compound can be obtained by concentrating the reaction mixture, adding an organic solvent immiscible with water such as ethyl acetate thereto, and after washing with water, separating the organic layer containing the target compound, drying the same over anhydrous magnesium sulfate or the like, and removing the solvent.

The obtained compound may be further purified by a conventional method such as recrystallization or silica gel chromatography, if necessary.

### (Step Ba8)

This step is for producing a compound (XXVII) by deprotection of the protective group of the hydroxyl group at position 1 of the compound (XXVI). The step is accomplished by performing the reaction under the same conditions as in Step Aa11.

### (2-2) Process b

This process produces an intermediate (XXXIII) used for producing a compound (I) in which W is an oxygen atom.

### (Step Bb1)

This step is for producing a compound (XXIX), which is primary alcohol, by deprotection of the silyl protective group of the compound (XXVIII) and simultaneous deprotection by removing enol ether at the anomeric position. The step is accomplished by employing reaction conditions harsher than those in Step Aa9, for example, by extending the reaction time.

The reaction time is generally 4 hours to 24 hours, preferably 10 hours to 16 hours.

After completion of the reaction, the target compound (XXIX) of the reaction is collected from the reaction mixture by a conventional method. For example, the target compound can be obtained by concentrating the reaction mixture, adding an organic solvent immiscible with water such as ethyl acetate thereto, and after washing with water, separating the organic layer containing the target compound, drying the same over anhydrous magnesium sulfate or the like, and removing the solvent.

The obtained compound may be further purified by a conventional method such as recrystallization or silica gel chromatography, if necessary.

### (2-3) Process c

This process produces an intermediate (XXXIX) used for producing a compound (I) in which W is an oxygen atom and Q is a group represented by -OCH₂CH₂O-.

### (Step Bc1)

This step is for producing a compound (XXXI) by oxidizing the allyl double bond of the compound (XXX) to form aldehyde followed by reduction. The step is accomplished by reacting the compound (XXX) with an oxidant in an inert solvent and further with a reducing agent in an inert solvent.

Examples of solvents to be used in the oxidation include a mixed solvent of ethers such as tetrahydrofuran or dioxane with water, and examples thereof in the reduction include alcohols such as methanol and ethanol.

Examples of oxidants to be used include combination of osmium tetroxide and sodium periodate.

Examples of reducing agents to be used include metal hydrides such as lithium aluminium hydride and sodium borohydride. The reducing agent is preferably sodium borohydride.

The reaction temperature is generally 0°C to 100°C, preferably room temperature.

The reaction time is generally 6 minutes to 24 hours.

After completion of the reaction, the target compound (XXXI) of the reaction is collected from the reaction mixture by a conventional method. For example, the target compound can be obtained by concentrating the reaction mixture, adding an organic solvent immiscible with water such as ethyl acetate thereto, and after washing with water, separating the organic layer containing the target compound, drying the same over anhydrous magnesium sulfate or the like, and removing the solvent.

The obtained compound may be further purified by a conventional method such as recrystallization or silica gel chromatography, if necessary.

### (Step Bc2)

This step is for producing a compound (XXXII) by converting the compound (XXXI) to its phosphite and oxidizing the same. The step is accomplished by performing the reaction under the same conditions as in Step Aa8.

### (Step Bc3)

This step is for producing a compound (XXXIII) by deprotection of isopropylidene bridged to the hydroxyl groups at positions 4 and 6 of the compound (XXXII). The step is accomplished by performing the reaction under the same conditions as in Step Aa6.

### (2-4) Process d

This process produces a compound (XXXV) in which W is an oxygen atom and the hydroxyl groups at positions 2 and 3 are protected by an identical protective group.

### (Step Bd1)

This step is for obtaining a compound (XXXV) by alkylating or acylating two hydroxyl groups of a known compound (XXXIV). The step is accomplished by performing the reaction under conditions similar to those in Step Aa1.

### (2-5) Process e

This process produces an intermediate (XXXVIII) used for producing a compound (I) in which W is an oxygen atom and Q is a group represented by -OCH₂CH₂-.

### (Step Be1)

This step is for producing a compound (XXXVI) by converting the primary hydroxyl group of the compound (XXXI) to a bromine atom. The step can be accomplished by reacting the compound (XXXI) with carbon tetrabromide-triphenylphosphine in an inert solvent.

Examples of solvents to be used include halogenated hydrocarbons such as methylene chloride and chloroform; ethers such as tetrahydrofuran; and aromatic hydrocarbons such as benzene.

The reaction temperature is generally 0°C to the heating reflux temperature of the solvent, preferably room temperature.

The reaction time is generally 6 minutes to 16 hours, preferably 1 hour to 3 hours.

After completion of the reaction, the target compound (XXXVI) of the reaction is collected from the reaction mixture by a conventional method. For example, the target compound can be obtained by neutralizing the reaction mixture, then concentrating the mixture, adding an organic solvent immiscible with water such as ethyl acetate thereto, and after washing with water, separating the organic layer containing the target compound, drying the same over anhydrous magnesium sulfate or the like, and removing the solvent.

The obtained compound (XXXVI) may be further purified by a conventional method such as recrystallization or silica gel chromatography, if necessary.

### (Step Be2)

This step is for producing diallyl phosphate (XXXVII) by allowing a bromine atom in the compound (XXXVI) to react with triallyl phosphite. The step is generally performed neat.

The reaction temperature is generally 150°C to 200°C.

### (Step Be3)

This step is for preparing diol (XXXVIII) by deprotection of the protective group of the hydroxyl groups at positions 4 and 6 of the compound (XXXVII). The step is accomplished by reacting the compound (XXXVII) with acid in an inert solvent.

Examples of solvents to be used include alcohols such as methanol and ethanol; and an aqueous acetic acid solution (e.g., an 80% aqueous acetic acid solution).

Examples of acids to be used include organic acids such as acetic acid and p-toluenesulfonic acid; and inorganic acids such as hydrogen chloride.

The reaction temperature is generally 50°C to 80°C.

The reaction time is generally 1 hour to 6 hours.

### (2-6) Process f

This process produces an intermediate (XLIII) used for producing a compound (I) in which W is an oxygen atom and Q is a group represented by -CH₂CH₂O-.

### (Step Bf1)

This step is for obtaining a compound (XL) by alkylating or acylating the hydroxyl groups at positions 2 and 3 of the compound (XXXIX). The step is accomplished by performing the reaction under conditions similar to those in Step Aa5.

### (Step Bf2)

This step is for producing a compound (XLI) by oxidizing the allyl double bond of the compound (XL) to form aldehyde followed by reduction. The step is accomplished by performing the reaction under the same conditions as in Step Bc1.

### (Step Bf3)

This step is for producing a compound (XLII) by converting the compound (XLI) to phosphite and oxidizing the same. The step is accomplished by performing the reaction under the same conditions as in Step Aa8.

### (Step Bf4)

This step is for producing a compound (XLIII) by deprotection of isopropylidene bridged to the hydroxyl groups at positions 4 and 6 of the compound (XLII). The step is accomplished by performing the reaction under the same conditions as in Step Aa6.

### (2-7) Process g

This process produces an intermediate (XLIX) used for producing a compound (I) in which W is an oxygen atom and Q is a group represented by -CH₂O-.

### (Step Bg1)

The step is for obtaining a compound (XLV) by removing four benzyl groups of a known compound (XLIV) and bridging isopropylidene to the hydroxyl groups at positions 4 and 6. The step for removing benzyl groups is accomplished by a usual hydrogenation reaction, and the step for bridging isopropylidene is accomplished by performing the reaction under the same conditions as in Step Aa3.

### (Step Bg2)

This step is for obtaining a compound (LV) by (a) alkylating or acylating two hydroxyl groups of the compound (XLV), (b) then oxidatively opening the furan ring, and (c) esterifying the generated carboxyl group.
(a) The alkylation or acylation step is accomplished by performing the reaction under the same conditions as in Step Bd1.
(b) The ring opening step of the furan ring is accomplished by allowing the furan compound to react with sodium periodate in an inert solvent.
   Examples of solvents to be used include halogenated hydrocarbons such as methylene chloride, chloroform and carbon tetrachloride; nitriles such as acetonitrile; water; and a mixed solvent thereof. The solvent is preferably a mixed solvent of carbon tetrachloride, acetonitrile and water.
   The reaction temperature is generally 0°C to 100°C, preferably 40°C to 80°C.
   The reaction time is generally 2 hours to 20 hours, preferably 10 hours to 15 hours.
(c) The esterification step can be accomplished by a conventional method. Specifically, in the case of methyl esterification, the step is accomplished by allowing a carboxylic acid compound to react with diazomethane or trimethylsilyldiazomethane in an inert solvent.

Examples of solvents to be used include ethers such as ether and tetrahydrofuran.

The reaction temperature is generally 0°C to room temperature.

### (Step Bg3)

This step is for obtaining a compound (XLVII) by reducing the ester group of the compound (XLVI). The step is accomplished by reacting the compound (XLVI) with a reducing agent in an inert solvent.

Examples of solvents to be used include alcohols such as methanol and ethanol; and ethers such as diethyl ether and tetrahydrofuran.

Examples of reducing agents to be used include metal hydrides such as lithium aluminium hydride and sodium borohydride. The reducing agent is preferably lithium aluminium hydride.

The reaction temperature is generally 0°C to 50°C, preferably room temperature.

The reaction time is generally 6 minutes to 24 hours.

After completion of the reaction, the target compound (XLVII) of the reaction is collected from the reaction mixture by a conventional method. For example, the target compound can be obtained by concentrating the reaction mixture, adding an organic solvent immiscible with water such as ethyl acetate thereto, and after washing with water, separating the organic layer containing the target compound, drying the same over anhydrous magnesium sulfate or the like, and removing the solvent.

The obtained compound may be further purified by a conventional method such as recrystallization or silica gel chromatography, if necessary.

### (Step Bg4)

This step is for producing a compound (XLVIII) by converting the compound (XLVII) to its phosphite and oxidizing the same. The step is accomplished by performing the reaction under the same conditions as in Step Aa8.

### (Step Bg5)

This step is for producing a compound (XLIX) by deprotection of isopropylidene bridged to the hydroxyl groups at positions 4 and 6 of the compound (XLVIII). The step is accomplished by performing the reaction under the same conditions as in Step Aa6.

### (2-8) Process h

This process produces an intermediate (LII) used for producing a compound (I) in which W is an oxygen atom and Q is a group represented by -O(CH₂)₃O-.

### (Step Bh1)

This step is for producing a compound (L) by oxidizing the allyl double bond of the compound (XXX) to form aldehyde followed by reduction. The step is accomplished by performing the reaction under the same conditions as in Step Bc1.

### (Step Bh2)

This step is for producing a compound (LI) by converting the compound (L) to its phosphite and oxidizing the same. The step is accomplished by performing the reaction under the same conditions as in Step Aa8.

### (Step Bh3)

This step is for producing a compound (LII) by deprotection of isopropylidene bridged to the hydroxyl groups at positions 4 and 6 of the compound (LI). The step is accomplished by performing the reaction under the same conditions as in Step Aa6.

### (2-9) Process i

This process produces an intermediate (LVI) used for producing a compound (I) in which W is an oxygen atom and Q is a group represented by -OCH₂CH₂O-.

### (Step Bi1)

This step is for producing a compound (LIV) by oxidizing the allyl double bond of the compound (LIII) to form aldehyde followed by reduction. The step is accomplished by performing the reaction under the same conditions as in Step Bc1.

### (Step Bi2)

This step is for producing a compound (LV) by converting the compound (LIV) to its phosphite and oxidizing the same. The step is accomplished by performing the reaction under the same conditions as in Step Aa8.

### (Step Bi3)

This step is for producing a compound (LVI) by protecting the secondary hydroxyl group of the compound (LV) by an acyl group. The step is accomplished by performing the reaction under the same conditions as in Step Ba1(b) and further by deprotection of the isopropylidene group at positions 4 and 6 under acid conditions under the same conditions as in Step Aa6.

### (2-10) Process j

This process produces an intermediate (LXX) used for producing a compound (I) in which W is an oxygen atom.

### (Step Bj1)

This step is for obtaining a compound (LXV) by alkylating or acylating the hydroxyl group at position 2 of the compound (LXIV). The step is accomplished by performing the reaction under the same conditions as in Step Aa1.

### (Step Bj2)

This step is for producing a compound (LXVI) by deprotection of isopropylidene bridged to the hydroxyl groups at positions 4 and 6 of the compound (LXV). The step is accomplished by performing the reaction under the same conditions as in Step Aa6.

### (Step Bj3)

This step is for producing a compound (LXVII) by converting the double bond in the allyl group of the compound (LXVI) to an enol ether form. The step is accomplished by performing the reaction under the same conditions as in Step Aa4.

### (Step Bj4)

This step is for producing a compound (LXVIII) by protecting the primary hydroxyl group of the diol, which is the compound (LXVII), by a silyl group. The step is accomplished by performing the reaction under the same conditions as in Step Aa7.

### (Step Bj5)

This step is for producing a compound (LXIX) by protecting the secondary hydroxyl group of the compound (LXVIII) by an allyloxycarbonyl group. The step is accomplished by performing the reaction under the same conditions as in Step Ba7.

### (Step Bj6)

This step is for producing a compound (LXX) by deprotection of the protective group of the hydroxyl group at position 1 of the compound (LXIX). The step is accomplished by performing the reaction under the same conditions as in Step Aa11.

### (2-11) Process k

This process produces an intermediate (LXXIV) used for producing a compound (I) in which W is an NH group.

### (Step Bk1)

This step is for producing a compound (LXXII) by oxidizing the allyl double bond of the compound (LXXI) to form aldehyde followed by reduction, and further deprotecting at position 2. The step is accomplished by performing the reaction under the same conditions as in Step Bc1 and Step Ba2.

### (Step Bk2)

This step is for producing a compound (LXXIII) by converting the compound (LXXII) to its phosphite, oxidizing the same and further amidating the amino group. The step is accomplished by performing the reaction under the same conditions as in Steps Aa8 and Ba3.

### (Step Bk3)

This step is for producing a compound (LXXIV) by deprotection of isopropylidene bridged to the hydroxyl groups at positions 4 and 6 of the compound (LXXIII). The step is accomplished by performing the reaction under the same conditions as in Step Aa6.

### (3) Process C

### (3-1) Process a

This process produces a compound (Ia) of the present invention in which Q is an oxygen atom.

### (Step Ca1)

This step is for producing a compound (LVII) by subjecting the compound (XXVII), (XXIX) or (LXX) to a glycosylation reaction with the aforementioned compound (XIV). The step is performed in an inert solvent in the presence of a catalyst.

Examples of solvents to be used include halogenated hydrocarbons such as methylene chloride and chloroform.

Examples of catalysts to be used include Lewis acids such as tin tetrachloride, trifluoroboron/etherate, aluminium chloride, ferric chloride, trimethylsilyl triflate and silver triflate; and molecular sieves 4A.

The reaction temperature is generally -50°C to 40°C.

The reaction time is generally 1 hour to 24 hours, preferably 4 hours.

After completion of the reaction, the target compound (LVII) of the reaction is collected from the reaction mixture by a conventional method. For example, the target compound can be obtained by neutralizing the reaction mixture, then concentrating the mixture, adding an organic solvent immiscible with water such as ethyl acetate thereto, and after washing with water, separating the organic layer containing the target compound, drying the same over anhydrous magnesium sulfate or the like, and removing the solvent.

The obtained compound (LVII) may be further purified by a conventional method such as recrystallization or silica gel chromatography, if necessary.

### (Step Ca2)

This step is for producing a compound (LXIII) by converting the compound (LVII) to its phosphite and oxidizing the same. The step is accomplished by performing the reaction under the same conditions as in Step Aa8.

### (Step Ca3)

This step is for producing a compound (Ia) of the present invention by deprotection of the protective group of the compound (LVIII) (the protective group is preferably an allyl group or an allyloxycarbonyl group when R¹, R², R³ and R⁴ contain a double bond). The step is accomplished by the method described in a literature (e.g., T. W. Greene, Protective Groups in Organic Synthesis) or by the following method. In addition, when plural kinds of protective groups are present in the compound (LXVI), the step can be accordingly performed by combining methods appropriate for the protective groups.
1) When the protective group is an aralkyl group and R¹, R², R³ and R⁴ contain no double bond, deprotection can be performed by hydrogenolysis in an inert solvent in the presence of a catalyst under a hydrogen atmosphere.
   Examples of solvents to be used include ethers such as tetrahydrofuran, dioxane and ether; and esters such as ethyl acetate; alcohols such as methanol and ethanol; and organic acids such as formic acid and acetic acid. The solvent is preferably ethanol.
   Examples of catalysts to be used include palladium/carbon, palladium hydroxide, palladium hydroxide/carbon and palladium black. The catalyst is preferably palladium hydroxide/carbon.
   The reaction temperature is generally 0°C to 50°C, preferably 15°C to 25°C.
   The reaction time is generally 1 to 48 hours, preferably 3 to 24 hours.
   After completion of the reaction, the target compound (Ia) of the reaction is collected from the reaction mixture by a conventional method. For example, the target compound can be obtained by separating the catalyst in the reaction mixture by filtration and concentrating the resulting filtrate.
   The obtained compound may be further purified by a conventional method such as recrystallization or silica gel chromatography, if necessary.
2) When the protective group is a phenyl group and R¹, R², R³ and R⁴ contain no double bond, deprotection can be performed by catalytic reduction in an inert solvent in the presence of a catalyst.
   Examples of solvents to be used include ethers such as tetrahydrofuran, dioxane and ether; and esters such as ethyl acetate; alcohols such as methanol and ethanol; and organic acids such as formic acid and acetic acid. The solvent is preferably tetrahydrofuran.
   The catalyst to be used is preferably platinum oxide.
   The reaction temperature is generally 0°C to 50°C, preferably 15°C to 25°C.
   The reaction time is generally 1 to 48 hours, preferably 1 to 24 hours.
   After completion of the reaction, the target compound (Ia) of the reaction is collected from the reaction mixture by a conventional method. For example, the target compound can be obtained by separating the catalyst in the reaction mixture by filtration and concentrating the resulting filtrate.
   The obtained compound may be further purified by a conventional method such as recrystallization or cellulose chromatography, if necessary.
3) When the protective group is an allyl group or an allyloxycarbonyl group, deprotection of the allyl group or the allyloxycarbonyl group in the compound (LVIII) can be performed by refluxing in an inert solvent in the presence of a palladium catalyst using a formic acid-triethylamine mixture or acetic acid-triethylamine, or in the presence of a rhodium catalyst in a 95% aqueous ethanol solution.

The obtained compound may be further purified by a conventional method such as recrystallization or cellulose chromatography, if necessary.

### (3-2) Process b

This process produces a compound (Ib) of the present invention in which Q is an oxygen atom and R⁵ is a hydroxyl group.

### (Step Cb1)

This step is for producing a compound (LIX) by subjecting the compound (XXVII), (XXIX) or (LXX) to a glycosylation reaction with the aforementioned compound (XVIII). The step is accomplished by performing the reaction under the same conditions as in Step Ca1.

### (Step Cb2)

This step is for producing a compound (LX) by converting the compound (LIX) to its phosphite and oxidizing the same. The step is accomplished by performing the reaction under the same conditions as in Step Aa8.

### (Step Cb3)

This step is for producing a compound (Ib) of the present invention by deprotection of the protective group of the compound (LX). The step is accomplished by performing the reaction under the same conditions as in Step Ca3.

### (3-3) Process c

This process produces a compound (I) of the present invention.

### (Step Cc1)

This step is for producing a compound (LXI) by subjecting the compound (XXXIII), (XXXVIII), (XLIII), (XLIX), (LII), (LVI) or (LXXIV) to a glycosylation reaction with the aforementioned compound (XIV). The step is accomplished by performing the reaction under the same conditions as in Step Ca1.

### (Step Cc2)

This step is for producing a compound (I) of the present invention by deprotection of the protective group of the compound (LXI). The step is accomplished by performing the reaction under the same conditions as in Step Ca3.

### (3-4) Process d

This process produces a compound (Ic) of the present invention by removing a group R^{4a} when R⁴ in the compound (LXII) is a group represented by the formula: 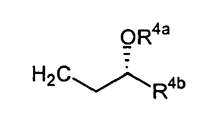 (wherein R^{4a} and R^{4b} have the same meaning as defined above) and by deprotection of other protective groups.

### (Step Cd1)

This step is for producing a compound (LXIII) by deprotection of the protective group R^{4a} of the compound (LXII). The step is accomplished by reacting the compound (LXII) with 2,3-dichloro-5,6-dicyano-1,4-benzoquinone (DDQ) in an inert solvent.

Examples of solvents to be used include halogenated hydrocarbons such as methylene chloride.

The reaction temperature is generally room temperature to 50°C.

The reaction time is generally 5 minutes to 24 hours.

After completion of the reaction, the target compound (LXIII) of the present invention is collected from the reaction mixture by a conventional method. For example, the target compound can be obtained by diluting the reaction mixture with an inert solvent, washing with an aqueous alkaline solution and water, drying the resultant over anhydrous magnesium sulfate, and then removing the solvent.

The obtained compound may be further purified by a conventional method such as recrystallization or silica gel chromatography, if necessary.

### (Step Cd2)

This step is for producing a compound (Ic) of the present invention by deprotection of the protective group of the compound (LXIII). The step is accomplished by performing the reaction under the same conditions as in Step Ca3.

### (3-4) Process e

This process produces a compound (Id) of the present invention in which R⁵ is a hydroxyl group.

### (Step Ce1)

This step is for producing a compound (Id) of the present invention in which R⁵ is a hydroxyl group by deprotection of the protective group of the compound (LXXV). The step is accomplished by performing the reaction under the same conditions as in Step Ca3.

### (3-5) Process f

This process produces a compound (LXXVIII) which is a intermediate product of the present invention in which Q is an oxygen atom.

### (Step Cf1)

This step is for producing a compound (LXXVII) by subjecting the compound (LXXVI) to a glycosylation reaction with the compound (XIV). The step is accomplished by performing the reaction under the same conditions as in Step Ca1.

### (Step Cf2)

This step is for producing a compound (LXXVIII) by deprotection of the protective group of the hydroxyl group at position 1 of the compound (LXXVII). The step is accomplished by performing the reaction under the same conditions as in Step Aa11.

Examples of administration routes of the compound (I) of the present invention include oral administration in the form of tablets, capsules, granules, powder or syrup, and parenteral administration in the form of injections or suppositories. These pharmaceutical formulations are prepared by well-known methods using additives such as excipients, binders, disintegrants, lubricants, stabilizers and corrigents.

The dose varies depending on symptoms, age and other factors. The compound can be administered at a dose of generally 0.01 to 10 mg/kg body weight for an adult in one or divided doses per day.

In the following, the present invention is described in more detail with reference to Examples, Reference Examples and Test Examples, but the present invention is not limited thereto.

### [Reference Example 1]

1,2:5,6-Di-O-isopropylidene-3-O-[(R)-3-methoxydecyl]-α-D-glucofuranose (Step Aa1)

1,2:5,6-Di-O-isopropylidene-α-D-glucofuranose (14.80 g, 56.857 mmol) and (R)-3-methoxydecyl p-toluenesulfonate (16.50 g, 48.175 mmol) were dissolved in dimethylformamide (DMF, 60 mL), and sodium hydride (55% dispersion in oil, 3.72 g, 85.286 mmol) was added to the solution under ice cooling. After stirring at 0°C for 15 minutes, the mixture was stirred at room temperature overnight. After completion of the reaction, methanol was added to the reaction solution under ice cooling to decompose sodium hydride, and the solution was diluted with ethyl acetate. The solution was washed with water and saturated brine, dried over magnesium sulfate, filtrated and concentrated in vacuo. The resulting residue was purified by silica gel chromatography (eluent: cyclohexane /ethyl acetate=15/1, then 2/1) to give the title compound as an oil (15.20 g, yield 98%).
IR νₘₐₓ(film) 2987, 2932, 2960 cm⁻¹.
400 MHz ¹H NMR (CDCl₃) δ 0.88 (3H, t, J=6.6 Hz), 1.28 (10H, bs), 1.37 (3H, s), 1.35 (3H, s), 1.43-1.52 (8H, m, containing two 3H, s, at 1.43 and 1.50 ppm), 1.68-1.74 (2H, m), 3.30 (1H, m), 3.32 (3H, s), 3.57 (1H, m), 3.73 (1H, m), 3.86 (1H, d, J=2.9 Hz), 3.99 (1H, m), 4.08 (1H, m), 4.12 (1H, dd, J=2.9, 7.3 Hz), 4.30 (1H, dd, J=5.9, 13.9 Hz), 4.55 (1H, d, J=3.7 Hz), 5.87 (1H, d, J=3.7 Hz).
FABMS (positive-ion) m/z; 431 [M+H]⁺.
HRFABMS; Calcd. for C₂₃H₄₃O₇: 431.3009. Found: 431.3018.

### [Reference Example 2]

Allyl 4,6-O-isopropylidene-3-O-[(R)-3-methoxydecyl]-α,β-D-glucopyranoside (Steps Aa2 and Aa3)

1,2:5,6-Di-O-isopropylidene-3-O-[(R)-3-methoxydecyl]-α-D-glucofuranose obtained in Reference Example 1 (5.79 g, 13.446 mmol) was dissolved in allyl alcohol (80 mL) containing 2% of hydrochloric acid, and the mixture was refluxed for 30 minutes. The reaction solution was concentrated in vacuo and the resultant was dissolved in DMF (12 mL) and 2,2-dimethoxypropane (15 mL). To the solution was added p-toluenesulfonic acid (200 mg), and the mixture was stirred at room temperature for 16 hours. The reaction solution was diluted with ethyl acetate, washed with sodium bicarbonate solution and saturated brine, dried over magnesium sulfate, filtrated and concentrated in vacuo. The resulting residue was purified by silica gel chromatography (eluent: cyclohexane/ethyl acetate =3/1) to give an anomer mixture of the title compound as an oil (4.12 g, yield 71%).
physical constant of α-anomer:
IR νₘₐₓ(film) 3461 (br), 2994, 2929, 2874, 2859 cm⁻¹.
400 MHz ¹H NMR (CDCl₃) δ 0.88 (3H, t, J=6.6 Hz), 1.28 (10H, bs), 1.41 (3H, s), 1.42-1.48 (2H, m), 1.49 (3H, s), 1.72-1.76 (2H, m), 3.04 (1H, d, J=6.6 Hz, OH), 3.33 (3H, s), 3.38 (1H, m), 3.47-3.93 (8H, m), 4.05 (1H, m), 4.21 (1H, m), 4.93 (1H, d, J=3.7 Hz), 5.22-5.35 (2H, m), 5.95 (1H, m).
FABMS (positive-ion) m/z; 431 [M+H]⁺, 453 [M+Na]⁺.
HRFABMS; Calcd. for C₂₃H₄₃O₇: 431.3009. Found: 431.2985.

### [Reference Example 3]

### (E)-1-Propenyl 4,6-O-isopropylidene-3-O-[(R)-3-methoxydecyl]-α,β-D-glucopyranoside (Step Aa4)

Allyl 4,6-O-isopropylidene-3-O-[(R)-3-methoxydecyl]-α,β-D-glucopyranoside obtained in Reference Example 2 (4.10 g, 9.522 mmol) was dissolved in tetrahydrofuran (THF, 100 mL). To the solution was added Ir[C₈H₁₂(MePh₂P)₂]PF₆ (50 mg) activated by hydrogen, and the mixture was stirred at room temperature for 6 hours and then concentrated in vacuo. The resulting residue was purified by silica gel column chromatography to separate an anomer mixture of the title compound. Specifically, α-anomer (1.24 g, yield 30%, Rf=0.511) and β-anomer (2.49 g, yield 61%, Rf=0.413) were obtained.
physical constant of α-anomer:
IR νₘₐₓ(film) 3441 (br), 2928, 2859, 1679 cm⁻¹.
400 MHz ¹H NMR (CDCl₃) δ 0.88 (3H, t, J=6.6 Hz), 1.28 (10H, bs), 1.40-1.60 (11H, m, containing two 3H, s, at 1.41 and 1.49 ppm, and 3H, dd, J=1.5 and 6.6 Hz, at 1.56 ppm), 1.72-1.77 (2H, m), 1.85 (1H, br, OH), 3.26 (1H, m), 3.33 (3H, s), 3.39 (1H, m), 3.51-3.95 (7H, m), 5.08 (1H, d, J=3.7 Hz), 5.20 (1H, m), 6.15 (1H, J=1.9, 12.1 Hz). FABMS (positive-ion) m/z; 431 [M+H]⁺, 453 [M+Na]⁺.
HRFABMS; Calcd. for C₂₃H₄₃O₇: 431.3009. Found: 431.3012.

### [Reference Example 4]

### (E)-1-Propenyl 4,6-O-isopropylidene-3-O-[(R)-3-methoxydecyl]-2-O-[(Z)-11-octadecenoyl]-α-D-glucopyranoside (Step Aa5)

(E)-1-Propenyl 4,6-O-isopropylidene-3-O-[(R)-3-methoxydecyl]-α-D-glucopyranoside obtained in Reference Example 3 (1.70 g, 3.948 mmol) was dissolved in methylene chloride (25 mL). Thereto were added (Z)-11-octadecenoic acid (1.67g, 5.922 mmol), dimethylaminopyridine (DMAP, 727 mg, 5.946 mmol) and WSCI hydrochloride (1.14 g, 5.946 mmol), and the mixture was stirred at room temperature for 1 hour. The reaction solution was directly purified by silica gel column chromatography (eluent: cyclohexane/ethyl acetate =9/1) to give the title compound (1.81 g, yield 66%).
IR νₘₐₓ(film) 2927, 2856, 1743, 1680 (w) cm⁻¹.
400 MHz ¹H NMR (CDCl₃) δ 0.88 (6H, t, J=6.6 Hz), 1.27 (28H, bs), 1.41 (3H, s), 1.42-1.46 (2H, m), 1.49 (3H, s), 1.53 (3H, dd, J=1.5, 6.6 Hz), 1.59-1.69 (6H, m), 1.99-2.03 (4H, m), 2.33-2.37 (2H, m), 3.30 (1H, m), 3.31 (3H, s), 3.60-3.86 (7H, m), 4.77 (1H, m), 5.16-5.18 (2H, m, containing 1H, d, J=3.7 Hz, at 5.17 ppm), 5.33-5.36 (2H, m), 6.07 (1H, dd, J=1.5, 12.5 Hz).
FABMS (positive-ion) m/z; 637, 695 [M+H]⁺, 717 [M+Na]⁺.
HRFABMS; Calcd. for C₄₁H₇₄O₈Na: 717.5282. Found: 717.5275.

### [Reference Example 5]

### (E)-1-Propenyl 3-O-[(R)-3-methoxydecyl]-2-O-[(Z)-11- octadecenoyl]-α-D-glucopyranoside (Step Aa6)

(E)-1-Propenyl 4,6-O-isopropylidene-3-O-[(R)-3-methoxydecyl]-2-O-[(Z)-11-octadecenoyl]-α-D-glucopyranoside obtained in Reference Example 4 (250 mg, 0.360 mmol) was dissolved in an 80% aqueous acetic acid solution (10 mL). The solution was stirred at 60°C for 1.5 hours and then concentrated in vacuo. The resulting residue was purified by silica gel column chromatography (eluent: cyclohexane/ethyl acetate=2/1, then 1/1) to give the title compound (234 mg, yield 99%).
IR νₘₐₓ(film) 3407 (br), 2927, 2856, 1741, 1680 (w) cm⁻¹.
400 MHz ¹H NMR (CDCl₃) δ 0.88 (6H, t, J=6.6 Hz), 1.27 (30H, bs), 1.41-1.49 (2H, m), 1.55 (3H, dd, J=1.5, 6.6 Hz), 1.57-1.77 (4H, m), 1.98-2.05 (4H, m), 2.32-2.41 (2H, m), 3.31 (1H, s), 3.35 (3H, m), 3.60 (1H, m), 3.65-3.74 (3H, m), 3.76-3.85 (2H, m), 3.87 (1H, d, J=1.5 Hz, OH), 4.97 (1H, m), 4.71 (1H, m, C2-H), 5.15 (1H, dd, J=7.3, 12.5 Hz), 5.21 (1H, d, J=3.7 Hz, anomeric H), 5.33-5.37 (2H, m), 5.33-5.36 (2H, m), 6.10 (1H, dd, J=1.5, 12.5 Hz).
FABMS (positive-ion) m/z; 655 [M+H]⁺, 677 [M+Na]⁺.
HRFABMS; Calcd. for C₃₈H₇₁O₈: 655.5149. Found: 655.5153.

### [Reference Example 6]

### (E)-1-Propenyl 6-O-(t-butyldimethylsilyl)-3-O-[(R)-3-methoxydecyl]-2-O-[(Z)-11-octadecenoyl]-α-D-glucopyranoside (Step Aa7)

(E)-1-Propenyl 3-O-[(R)-3-methoxydecyl]-2-O-[(Z)-11-octadecenoyl]-α-D-glucopyranoside obtained in Reference Example 5 (1.729 g, 2.640 mmol) was dissolved in methylene chloride (20 mL). To the solution were added t-butyldimethylsilyl chloride (437 mg, 2.904 mmol) and DMAP(362 mg, 2.962 mmol), and the mixture was stirred at room temperature for 16 hours. The reaction solution was directly purified by silica gel column chromatography (eluent: cyclohexane/ethyl acetate =4/1) to give the title compound (1.784 g, yield 88%).
IR νₘₐₓ(film) 3510-3430, 2928, 2857, 1744, 1680 (w), 1464 cm⁻¹.
400 MHz ¹H NMR (CDCl₃) δ 0.08 (6H, s), 0.88 (6H, t, J=6.6 Hz), 0.90 (9H, s), 1.27 (30H, bs), 1.43-1.50 (2H, m), 1.54 (3H, dd, J=1.5, 6.6 Hz), 1.59-1.65 (2H, m), 1.72-1.76 (2H, m), 1.99-2.05 (4H, m), 2.33-2.38 (2H, m), 3.30 (3H, s), 3.35 (1H, m), 3.56-3.93 (7H, m), 4.70 (1H, dd, J=3.7, 9.5 Hz), 5.14 (1H, m), 5.18 (1H, d, J=3.7 Hz, anomeric H), 5.33-5.36 (2H, m), 6.12 (1H, dd, J=2.2, 12.5 Hz).
FABMS (positive-ion) m/z; 769 [M+H]⁺, 791 [M+Na]⁺.

### [Reference Example 7]

### (E)-1-Propenyl 6-O-(t-butyldimethylsilyl)-4-O-diallylphosphono-3-O-[(R)-3-methoxydecyl]-2-O-[(Z)-11-octadecenoyl]-α-D-glucopyranoside (step Aa8)

(E)-1-Propenyl 6-O-(t-butyldimethylsilyl)-3-O-[(R)-3-methoxydecyl]-2-O-[(Z)-11-octadecenoyl]-α-D-glucopyranoside obtained in Reference Example 6 (1.784 g, 2.319 mmol) and 1H-tetrazole (325 mg, 4.639 mmol) were dissolved in methylene chloride (30 mL). To the solution was added diallyl diisopropylphosphorimidite (853 mg, 3.479 mmol), and the mixture was stirred at room temperature for 15 minutes. To the reaction solution were added THF (30 mL) and a 30% aqueous hydrogen peroxide solution (1.20 mL), and the solution was stirred at room temperature for 10 minutes. The reaction solution was washed with a 10% sodium thiosulfate solution, dried over magnesium sulfate, filtrated and concentrated in vacuo. The resulting residue was purified by silica gel column chromatography (eluent: cyclohexane/ethyl acetate=4/1) to give the title compound (2.100 g, yield 97%).
IR νₘₐₓ(film) 2928, 2857, 1745, 1680 (w), 1463 cm⁻¹.
400 MHz ¹H NMR (CDCl₃) δ 0.05 (6H, s), 0.88 (6H, t, J=6.6 Hz), 0.89 (9H, s), 1.27 (28H, bs), 1.40-1.43 (2H, m), 1.53 (3H, dd, J=1.5, 7.3 Hz), 1.59-1.75 (6H, m), 1.99-2.02 (4H, m), 2.34-2.39 (2H, m), 3.24 (1H, m), 3.26 (3H, s), 3.73-3.93 (7H, m), 4.26 (1H, m), 4.55-4.58 (4H, m), 4.71 (1H, m), 5.13-5.38 (7H, m), 5.90-5.97 (2H, m), 6.12 (1H, dd, J=1.5, 12.5 Hz).
FABMS (positive-ion) m/z; 929 [M+H]⁺, 951 [M+Na]⁺.

### [Reference Example 8]

### (E)-1-Propenyl 4-O-diallylphosphono-3-O-[(R)-3-methoxydecyl]-2-O-[(Z)-11-octadecenoyl]-a-D-glucopyranoside (Step Aa9)

(E)-1-Propenyl 6-O-(t-butyldimethylsilyl)-4-O-diallylphosphono-3-O-[(R)-3-methoxydecyl]-2-O-[(Z)-11-octadecenoyl]-α-D-glucopyranoside obtained in Reference Example 7 (2.100 g, 2.260 mmol) was dissolved in acetone (20 mL) and a 5% aqueous sulfuric acid solution (2 mL). The solution was stirred at room temperature for 5 hours and concentrated to half of its volume. The concentrate was diluted with ethyl acetate and the solution was washed with water, saturated sodium bicarbonate solution and saturated brine, dried over magnesium sulfate, filtrated and concentrated in vacuo. The resulting residue was purified by silica gel column chromatography (eluent: cyclohexane/ethyl acetate=3/2) to give the title compound (1.670 g, yield 91%).
IR νₘₐₓ(film) 3439, 2927, 2856, 1744, 1679 (w), 1461 cm⁻¹.
400 MHz ¹H NMR (CDCl₃) δ 0.88 (6H, t, J=6.6 Hz), 1.27 (28H, bs), 1.39-1.45 (4H, m), 1.54 (3H, dd, J=1.5, 7.3 Hz), 1.61-1.75 (4H, m), 1.99-2.03 (4H, m), 2.31-2.41 (2H, m), 3.23 (1H, m), 3.27 (3H, s), 3.65-3.96 (7H, m), 4.40 (1H, m), 4.57-4.66 (4H, m), 4.77 (1H, dd, J=3.7, 9.5 Hz), 5.16 (1H, m), 5.41 (1H, dd, J=1.5, 3.7 Hz), 5.26-5.38 (5H, m), 5.89-5.99 (2H, m), 6.11 (1H, dd, J=1.5, 12.5 Hz).
FABMS (positive-ion) m/z; 815 [M+H]⁺, 837 [M+Na]⁺.
HRFABMS; Calcd. for C₄₄H₈₀O₁₁P: 815.5438. Found: 815.5444.

### [Reference Example 9]

### (E)-1-Propenyl 4-O-diallylphosphono-3-O-[(R)-3-methoxydecyl]-6-O-methyl-2-O-[(Z)-11-octadecenoyl]-a-D-glucopyranoside (Step Aa10)

(E)-1-Propenyl 4-O-diallylphosphono-3-O-[(R)-3-methoxydecyl]-2-O-[(Z)-11-octadecenoyl]-α-D-glucopyranoside obtained in Reference Example 8 (167 mg, 0.205 mmol) was dissolved in methylene chloride (5 mL). To the solution were added 2,6-di-t-butyl-4-methylpyridine (210 mg, 1.024 mmol) and trimethyloxonium tetrafluoroborate (152 mg, 1.027 mmol), and the mixture was stirred at room temperature for 5 hours. The reaction solution was diluted with methylene chloride, washed with a saturated sodium bicarbonate solution, dried over magnesium sulfate, filtrated and concentrated in vacuo. The resulting residue was purified by silica gel column chromatography (eluent: cyclohexane/ethyl acetate=3/1) to give the title compound (161 mg, yield 95%).
IR νₘₐₓ(film) 2927, 2856, 1745, 1680 (w), 1460 cm⁻¹.
400 MHz ¹H NMR (CDCl₃) δ 0.88 (6H, t, J=6.6 Hz), 1.26 (30H, bs), 1.39-1.43 (2H, m), 1.54 (3H, dd, J=1.5, 7.3 Hz), 1.58-1.78 (4H, m), 1.99-2.04 (4H, m), 2.31-2.40 (2H, m), 3.24 (1H, m), 3.26 (3H, s), 3.39 (3H, s), 3.60-3.90 (6H, m), 4.39 (1H, m), 4.44-4.61 (4H, m), 4.78 (1H, m), 5.14-5.40 (8H, m), 5.90-5.98 (2H, m), 6.12 (1H, m). FABMS (positive-ion) m/z; 829 [M+H]⁺, 851 [M+Na]⁺.

HRFABMS; Calcd. for C₄₅H₈₂O₁₁P: 829.5595. Found: 829.5551.

### [Reference Example 10]

### (E)-1-Propenyl 4-O-diallylphosphono-3-O-[(R)-3-methoxydecyl]-6-O-methyl-2-O-[(Z)-11-octadecenoyl]-α-D-glucopyranoside (Steps Aa5 to Aa10)

The title compound was obtained in 6 steps at a yield of 52% by performing a reaction in the same manner as in Reference Examples 4 to 9, using (E)-1-propenyl 4,6-O-isopropylidene-3-O-[(R)-3-methoxydecyl]-β-D-glucopyranoside obtained in Reference Example 3 as a starting material.
400 MHz ¹H NMR (CDCl₃) δ 0.88 (6H, t, J=6.6 Hz), 1.27 (30H, bs), 1.40-1.45 (2H, m), 1.51-1.72 (7H, m), 1.99-2.02 (4H, m), 2.31-2.35 (2H, m), 3.22 (1H, m), 3.26 (3H, s), 3.39 (3H, s), 3.49-3.77 (6H, m), 4.34 (1H, m), 4.55-4.60 (4H, m), 4.96-5.10 (2H, m), 5.24-5.40 (6H, m), 5.89-5.98 (2H, m), 6.17 (1H, dd, J=1.5, 12.5 Hz).

### [Example 1]

Phosphono 3-O-decyl-2-deoxy-6-O-{3-O-[(R)-3-methoxydecyl]-6-O-methyl-2-O-[(Z)-11-octadecenoyl]-4-O-phosphono-β-D-glucopyranosyl}-2-(3-oxotetradecanoylamino)-α-D-glucopyranoside (Compound No. 1)

### (1) (E)-1-Propenyl 4-O-diallylphosphono-3-O-[(R)-3-methoxydecyl]-6-O-methyl-2-O-[(Z)-11-octadecenoyl]-α,β-D-glucopyranoside (Steps Aa5 to Aa10)

The title compound was obtained in 6 steps at a yield of 50% by performing a reaction in the same manner as in Reference Examples 4 to 9, using a mixture (2:1) of (E)-1-propenyl 4,6-O-isopropylidene-3-O-[(R)-3-methoxydecyl]-α-D-glucopyranoside and a β-anomer thereof obtained in Reference Example 3 as starting materials.

### (2) 4-O-Diallylphosphono-3-O-[(R)-3-methoxydecyl]-6-O-methyl-2-O-[(Z)-11-octadecenoyl]-α,β-D-glucopyranoside (Step Aa11)

(E)-1-Propenyl 4-O-diallylphosphono-3-O-[(R)-3-methoxydecyl]-6-O-methyl-2-O-[(Z)-11-octadecenoyl]-α,β-D-glucopyranoside obtained in (1) (mixing ratio of α-anomer to β-anomer=2:1, 1.290 g, 1.556 mmol) was dissolved in acetone-water (15 mL). N-bromosuccinimide (420 mg, 2.360 mmol) was added to the solution, and the mixture was stirred for 2 hours under ice cooling. The reaction solution was diluted with ethyl acetate, washed with a 10% sodium thiosulfate solution and saturated sodium bicarbonate solution, dried over magnesium sulfate, filtrated and concentrated in vacuo. The resulting residue was purified by silica gel column chromatography (eluent: cyclohexane/ethyl acetate=1/1) to give the title compound (980 mg, yield 80%).
IR νₘₐₓ(film) 3327 (br), 2927, 2856, 1742, 1461 cm⁻¹.
400 MHz ¹H NMR (CDCl₃) δ 0.88 (6H, t, J=6.6 Hz), 1.27 (30H, bs), 1.41-1.46 (2H, m), 1.60-1.76 (4H, m), 1.99-2.03 (4H, m), 2.35-2.40 (2H, m), 3.23 (1H, m), 3.26 (3H, s), 3.39 (3H, s), 3.58-3.78 (5H, m), 3.86 (1H, t, J=9.5 Hz), 4.12 (1H, m), 4.27 (1H, m), 4.57-4.61 (4H, m), 4.76 (1H, m), 5.23-5.40 (6H, m), 5.90-6.00 (2H, m).
FABMS (positive-ion) m/z; 789 [M+H]⁺, 811 [M+Na]⁺.
HRFABMS; Calcd. for C₄₂H₇₈O₁₁P: 789.5282. Found: 789.5286.

### (3) Trichloroacetimidoyl 4-O-diallylphosphono-3-O-[(R)-3-methoxydecyl]-6-O-methyl-2-O-[(Z)-11-octadecenoyl]-α-D-glucopyranoside (Step Aa12)

4-O-Diallylphosphono-3-O-[(R)-3-methoxydecyl]-6-O-methyl-2-O-[(Z)-11-octadecenoyl]-α,β-D-glucopyranoside obtained in (2) (368 mg, 0.466 mmol) and CCl₃CN (673 mg, 4.664 mmol) were dissolved in methylene chloride (7 mL). Cesium carbonate (76 mg, 0.233 mmol) was added to the solution, and the mixture was stirred at room temperature for 1.5 hours. The reaction solution was directly purified by silica gel column chromatography (eluent: cyclohexane/ethyl acetate= 2/1) to give the title compound (415 mg, yield 95%).
IR νₘₐₓ(film) 3349 (w), 2927, 2856, 1750, 1675, 1465 cm⁻¹.
400 MHz ¹H NMR (CDCl₃) δ 0.88 (6H, t, J=6.6 Hz), 1.27 (30H, bs), 1.40-1.44 (2H, m), 1.65-1.80 (4H, m), 1.99-2.02 (4H, m), 2.23 (2H, t, J=7.3 Hz), 3.24-3.28 (4H, m, containing 3H, s, at 3.27 ppm), 3.38 (3H, s), 3.64-3.80 (4H, m), 3.91 (1H, t, J=9.5 Hz), 4.03 (1H, m), 4.50 (1H, q, J=9.5 Hz), 4.57-4.61 (4H, m), 5.00 (1H, dd, J=3.7, 10.3 Hz), 5.25-5.40 (6H, m), 5.90-6.00 (2H, m), 6.50 (1H, d, J=3.7 Hz), 8.61 (1H, s). FABMS (positive-ion) m/z; 771, 954 [³⁵Cl, M+Na]⁺, 956 [M+Na]⁺.

### (4) Allyl 3-O-decyl-2-deoxy-4,6-O-isopropylidene-2- trifluoroacetamide-β-D-glucopyranoside (Step Ba1)

Allyl 2-deoxy-4,6-O-isopropylidene-2-trifluoroacetamide-β-D-glucopyranoside (10.660 g, 30.000 mmol) was dissolved in DMF (40 mL). Decyl methanesulfonate (17.735 g, 75.000 mmol) and sodium hydride (55% dispersion in oil, 3.49 g, 80.000 mmol) were added to the solution under ice cooling. After stirring for 30 minutes, the mixture was stirred at room temperature overnight. The reaction solution was diluted with ethyl acetate, washed with ice water and saturated brine, dried over magnesium sulfate, filtrated and concentrated in vacuo. The resulting residue was purified by silica gel column chromatography (eluent: cyclohexane/ethyl acetate =3/1) to give the title compound (9.516 g, yield 64%).
IR νₘₐₓ(KBr) 3318 (w), 2925, 2854, 1705, 1561 cm⁻¹.
400 MHz ¹H NMR (CDCl₃) δ 0.88 (3H, t, J=6.6 Hz), 1.25 (14H, bs), 1.41 (3H, s), 1.42-1.53 (5H, m, containing 3H, s, at 1.49 ppm), 3.29-3.41 (2H, m), 3.47 (1H, m), 3.61 (1H, m), 3.74-3.80 (2H, m), 3.81-3.95 (2H, m), 4.05 (1H, m), 4.31 (1H, m), 4.92 (1H, d, J=8.1 Hz), 5.19-5.28 (2H, m), 5.78-5.86 (1H, m), 6.43 (1H, d, J=8.1 Hz, NH). FABMS (positive-ion) m/z; 496 [M+H]⁺.

### (5) Allyl 2-amino-3-O-decyl-2-deoxy-4,6-O- isopropylidene-β-D-glucopyranoside (Step Ba2)

Allyl 3-O-decyl-2-deoxy-4,6-O-isopropylidene-2-trifluoroacetamide-β-D-glucopyranoside obtained in (4) (1.76 g, 2.018 mmol) was dissolved in ethanol (20 mL). To the solution was added 1 mol/L potassium hydroxide (20 mL), and the mixture was refluxed overnight and further stirred at room temperature overnight. The reaction solution was concentrated in vacuo and extracted with ether. The ether layer was washed with water and saturated brine, dried over sodium sulfate, filtrated and concentrated in vacuo. The resulting residue was purified by silica gel column chromatography (eluent: cyclohexane/ethyl acetate=1:4) to give the title compound (870 mg, yield 61%).
IR νₘₐₓ(film) 3395 (w), 2926, 2857 cm⁻¹.
400 MHz ¹H NMR (CDCl₃) δ 0.88 (3H, t, J=6.6 Hz), 1.26 (14H, bs), 1.41 (3H, s), 1.49 (3H, s), 1.50-1.59 (4H, m, containing NH₂), 2.83 (1H, dd, J=7.3, 9.5 Hz), 3.19-3.28 (2H, m), 3.56 (1H, m), 3.67 (1H, dd, J=8.8, 9.5 Hz), 3.77-3.92 (3H, m), 4.11 (1H, m), 4.32 (1H, d, J=8.1 Hz), 4.35 (1H, m), 5.20-5.33 (2H, m), 5.92 (1H, m). FABMS (positive-ion) m/z; 400 [M+H]⁺.
HRFABMS; Calcd. for C₂₂H₄₂NO₅: 400.3063. Found: 400.3056.

### (6) Allyl 3-O-decyl-2-deoxy-4,6-O-isopropylidene-2-(3-oxotetradecanoylamino)-β-D-glucopyranoside (Step Ba3)

Allyl 2-amino-3-O-decyl-2-deoxy-4,6-O-isopropylidene-β-D-glucopyranoside obtained in (5) (974 mg, 2.437 mmol) and 3-oxotetradecanoic acid (886 mg, 3.655 mmol) were dissolved in methylene chloride (5 mL). To the solution was added 1-[3-(dimethylamino)propyl]-3-ethylcarbodiimide (800 mg, 4.173 mmol), and the mixture was stirred for 30 minutes. The reaction solution was diluted with ethyl acetate, washed with water and saturated brine, dried over sodium sulfate, filtrated and concentrated in vacuo. The resulting residue was purified by silica gel column chromatography (eluent: cyclohexane/ethyl acetate =3/1) to give the title compound (1.320 g, yield 87%).
IR νₘₐₓ(KBr) 3265, 3097, 2921, 2851, 1724, 1716, 1648, 1565, 1447 cm⁻¹.
400 MHz ¹H NMR (CDCl₃) δ 0.88 (6H, t, J=6.6 Hz), 1.26 (30H, bs), 1.40 (3H, s), 1.44-1.48 (5H, m, containing 3H, s, at 1.48 ppm), 2.52 (2H, t, J=7.3 Hz), 3.29 (1H, m), 3.39 (2H, s), 3.43-3.59 (2H, m), 3.61 (1H, m), 3.68-3.80 (2H, m), 3.91 (1H, dd, J=5.1, 11.0 Hz), 4.05 (1H, m), 4.30 (1H, m), 4.79 (1H, d, J=8.8 Hz), 5.14-5.26 (2H, m), 5.82 (1H, m), 7.11 (1H, d, J=8.1 Hz, NH).
FABMS (positive-ion) m/z; 624 [M+H]⁺, 646 [M+Na]⁺.
HRFABMS; Calcd. for C₃₆H₆₆NO₇: 624.4839. Found: 624.4844.

### (7) Allyl 3-O-decyl-2-deoxy-2-(3-oxotetradecanoylamino)-β-D-glucopyranoside (Step Ba4)

Allyl 3-O-decyl-2-deoxy-4,6-O-isopropylidene-2-(3-oxotetradecanoylamino)-β-D-glucopyranoside obtained in (6) (1.310 g, 2.100 mmol) was dissolved in an 80% aqueous acetic acid solution (100 mL). The solution was stirred at 60°C for 1 hour and concentrated in vacuo. The resulting residue was purified by silica gel column chromatography (eluent: ethyl acetate, then ethyl acetate containing 5% of methanol) to give the title compound (1.100 g, yield 90%).
IR νₘₐₓ(KBr) 3270, 3094 (w), 2955, 2921, 2851, 1726, 1716, 1648, 1559 cm⁻¹.
400 MHz ¹H NMR (CDCl₃) δ 0.88 (6H, t, J=6.6 Hz), 1.26 (30H, bs), 1.50-1.60 (4H, m), 2.16 (1H, bs, OH), 2.50-2.54 (3H, m, containing OH), 3.40-3.41 (3H, m, containing 2H, s, at 3.40 ppm), 3.58-3.64 (5H, m), 3.81 (1H, dd, J=4.4, 11.7 Hz), 3.91 (1H, dd, J=3.3, 11.7 Hz), 4.06 (1H, dd, J=5.9, 13.2 Hz), 4.06 (1H, d, J=5.5 Hz), 4.73 (1H, d, J=7.3 Hz), 5.15-5.27 (2H, m), 5.84 (1H, m), 7.22 (1H, d, J=7.3 Hz, NH).
FABMS (positive-ion) m/z; 584 [M+H]⁺, 606 [M+Na]⁺.
HRFABMS; Calcd. for C₃₃H₆₂NO₇: 584.4526. Found: 584.4537.

### (8) Allyl 6-O-(t-butyldimethylsilyl)-3-O-decyl-2-deoxy-2-(3-oxotetradecanoylamino)-β-D-glucopyranoside (Step Ba5)

Allyl 3-O-decyl-2-deoxy-2-(3-oxotetradecanoylamino)-β-D-glucopyranoside obtained in (7) (1.030 g, 1.764 mmol) was dissolved in methylene chloride (25 mL). To the solution were added t-butyldimethylsilyl chloride (293 mg, 1.940 mmol) and dimethylaminopyridine (237 mg, 1.940 mmol), and the mixture was stirred at room temperature for 16 hours. The reaction solution was directly purified by silica gel column chromatography (eluent: cyclohexane/ethyl acetate=4:1) to give the title compound (1.072 g, yield 87%).
IR νₘₐₓ(KBr) 3516 (w), 3270, 3087 (w), 2956, 2922, 2852, 1715, 1647, 1556, 1467 cm⁻¹.
400 MHz ¹H NMR (CDCl₃) δ 0.09 (6H, s), 0.88 (6H, m), 0.90 (9H, s), 1.26 (30H, bs), 1.50-1.58 (4H, m), 2.52 (2H, t, J=7.3 Hz), 3.11 (1H, bs, OH), 3.36 (1H, m), 3.39 (2H, s), 2.52-2.62 (4H, m), 3.72 (1H, m), 3.84 (1H, m), 3.91 (1H, m), 4.04 (1H, m), 4.28 (1H, dd, J=5.1, 13.2 Hz), 4.71 (1H, d, J=8.1 Hz), 5.13-5.25 (2H, m), 5.83 (1H, m), 7.09 (1H, d, J=8.1 Hz, NH).
FABMS (positive-ion) m/z; 698 [M+H]⁺, 720 [M+Na]⁺.
HRFABMS; Calcd. for C₃₉H₇₆NO₇Si: 698.5391. Found: 698.5383.

### (9) (E)-1-Propenyl 6-O-(t-butyldimethylsilyl)-3-O-decyl-2-deoxy-2-(3-oxotetradecanoylamino)-β-D-glucopyranoside (Step Ba6)

Allyl 6-O-(t-butyldimethylsilyl)-3-O-decyl-2-deoxy-2-(3-oxotetradecanoylamino)-β-D-glucopyranoside obtained in (8) (315 mg, 0.451 mmol) was dissolved in THF (10 mL). To the solution was added Ir[C₈H₁₂(MePh₂P)₂]PF₆ (5 mg) activated by hydrogen, and the mixture was stirred at room temperature for 3 hours in a nitrogen atmosphere. The reaction solution was concentrated in vacuo and the title compound was quantitatively obtained.
IR νₘₐₓ(KBr) 3509 (w), 3276 (w), 2956, 2922, 2852, 1714, 1642, 1556, 1466 cm⁻¹. 400 MHz ¹H NMR (CDCl₃) δ 0.08 (3H, s), 0.10 (3H, s), 0.86-0.92 (15H, m, containing 9H, s, at 0.90 ppm), 1.26 (30H, bs), 1.50-1.58 (7H, m), 2.51 (2H, t, J=7.7 Hz), 3.19 (1H, bs, OH), 3.39 (2H, s), 3.43 (1H, m), 3.51-3.75 (5H, m), 3.84 (1H, m), 3.91 (1H, m), 4.94 (1H, d, J=8.1 Hz, anomeric H), 5.06 (1H, qd, J=6.6, 12.5 Hz), 6.15 (1H, dd, J=1.5, 12.5 Hz), 7.19 (1H, d, J=8.1 Hz, NH).
FABMS (positive-ion) m/z; 698 [M+H]⁺, 720 [M+Na]⁺.
HRFABMS; Calcd. for C₃₉H₇₆NO₇Si: 698.5391. Found: 698.5407.

### (10) (E)-1-Propenyl 4-O-(allyloxycarbonyl)-6-O-(t-butyldimethylsilyl)-3-O-decyl-2-deoxy-2-(3-oxotetradecanoylamino)-β-D-glucopyranoside (Step Ba7)

(E)-1-Propenyl 6-O-(t-butyldimethylsilyl)-3-O-decyl-2-deoxy-2-(3-oxotetradecanoylamino)-β-D-glucopyranoside obtained in (9) (840 mg, 1.203 mmol) was dissolved in toluene (10 mL). Pyridine (381 mg, 0.602 mmol) was added to the solution, triphosgene (179 mg, 0.602 mmol) was further added thereto under ice cooling, and the mixture was vigorously stirred for 10 minutes. Allyl alcohol (700 mg, 12.05 mmol) was further added to the reaction solution and the mixture was stirred for 1 hour under ice cooling. The reaction solution was diluted with ethyl acetate, washed with water, sodium bicarbonate solution and saturated brine, dried over magnesium sulfate, filtrated and concentrated in vacuo. The resulting residue was purified by silica gel column chromatography (eluent: cyclohexane/ethyl acetate=7/1) to give the title compound in the form of wax (704 mg, yield 75%).
IR νₘₐₓ(KBr) 3269, 3088 (w), 2926, 2855, 1752, 1717, 1681 (w), 1647, 1558, 1464 cm⁻¹.
400 MHz ¹H NMR (CDCl₃) δ 0.03 (3H, s), 0.04 (3H, s), 0.87 (15H, m), 1.22-1.26 (30H, bs), 1.40-1.44 (2H, m), 1.51 (3H, dd, J=1.5, 6.6 Hz), 1.52-1.58 (2H, m), 2.50 (2H, t, J=7.3 Hz), 3.38 (2H, s), 3.49-3.57 (4H, m), 3.68-3.78 (2H, m), 3.93 (1H, t, J=9.2 Hz), 4.62-4.73 (3H, m), 4.99 (1H, d, J=8.1 Hz, anomeric H), 5.08 (1H, m), 5.26-5.39 (2H, m), 5.92 (1H, m), 6.16 (1H, dd, J=1.5, 12.5 Hz), 7.22 (1H, d, J=7.3 Hz, NH).
FABMS (positive-ion) m/z; 804 [M+Na]⁺.
HRFABMS; Calcd. for C₄₃H₇₉NO₉SiNa: 804.5422. Found: 804.5413.

### (11) 4-O-(Allyloxycarbonyl)-3-O-decyl-2-deoxy-2-(3-oxotetradecanoylamino)-α-D-glucopyranoside (Step Ba8)

(E)-1-Propenyl 4-O-(allyloxycarbonyl)-6-O-(t-butyldimethylsilyl)-3-O-decyl-2-deoxy-2-(3-oxotetradecanoylamino)-β-D-glucopyranoside obtained in (10) (630 mg, 0.805 mmol) was dissolved in a methylene chloride-acetonitrile mixed solvent (1:2, 63 mL). A 48% aqueous hydrofluoric acid solution (14 mL) was added to the solution, and the mixture was vigorously stirred at room temperature for 2 hours. The reaction solution was diluted with methylene chloride, washed with water and sodium bicarbonate solution, dried over magnesium sulfate, filtrated and concentrated in vacuo. The resulting residue was purified by silica gel column chromatography (eluent: cyclohexane/ethyl acetate= 1/2, then ethyl acetate/methanol=19/1) to give the title compound in the form of wax (336 mg, yield 66%).
IR νₘₐₓ(KBr) 3546, 3406, 3292, 3085, 2925, 2854, 1758, 1721, 1641, 1555 cm⁻¹. 400 MHz ¹H NMR (CDCl₃) δ 0.88 (6H, t, J=6.6 Hz), 1.26 (30H, bs), 1.40-1.49 (2H, m), 1.51-1.60 (2H, m), 2.45 (1H, m, OH), 2.52 (2H, t, J=7.3 Hz), 3.42 (2H, s), 3.46 (1H, m, OH), 3.52-3.80 (5H, m), 4.00 (1H, m), 4.16 (1H, m), 4.66 (2H, d, J=5.9 Hz), 4.80 (1H, t, J=9.5 Hz, anomeric H), 5.28-5.40 (3H, m), 5.94 (1H, m), 7.33 (1H, d, J=8.8 Hz, NH).
FABMS (positive-ion) m/z; 610, 628 [M+H]⁺, 650 [M+Na]⁺.
HRFABMS; Calcd. for C₃₄H₆₁NO₉Na: 650.4244. Found: 650.4254.

### (12) 4-O-(Allyloxycarbonyl)-3-O-decyl-2-deoxy-6-O-{4-O-diallylphosphono-3-O-[(R)-3-methoxydecyl]-6-O-methyl-2-O-[(Z)-11-octadecenoyl]-β-D-glucopyranosyl}-2-(3-oxotetradecanoylamino)-α-D-glucopyranoside (Step Ca1)

Trichloroacetimidoyl 4-O-diallylphosphono-3-O-[(R)-3-methoxydecyl]-6-O-methyl-2-O-[(Z)-11-octadecenoyl]-α-D-glucopyranoside obtained in (3) (164 mg, 0.175 mmol) and 4-O-(allyloxycarbonyl)-3-O-decyl-2-deoxy-2-(3-oxotetradecanoylamino)-α-D-glucopyranoside obtained in (11) (100 mg, 0.159 mmol) were dissolved in methylene chloride (5 mL). Molecular sieves 4A (250 mg) were added to the solution, and the mixture was stirred for 15 minutes in a nitrogen atmosphere. Thereto were further added silver trifluoromethanesulfonate (AgOTf, 100 mg, 0.389 mmol) and trimethylsilyl trifluoromethanesulfonate (TMSOTf, 10 mg, 0.045 mmol), and the mixture was stirred at room temperature for 16 hours in a nitrogen atmosphere. The reaction solution was diluted with methylene chloride, washed with sodium bicarbonate solution and saturated brine, dried over magnesium sulfate, filtrated and concentrated in vacuo. The resulting residue was purified by silica gel chromatography (eluent: cyclohexane/ethyl acetate =1/2) to give the title compound in the form of gum (135 mg; yield 61%).
IR νₘₐₓ(film) 3650-3200, 2927, 2855, 1752, 1650, 1546, 1465 cm⁻¹.
400 MHz ¹H NMR (CDCl₃) δ 0.86-0.89 (12H, m), 1.26 (60H, bs), 1.41-1.44 (4H, m), 1.55-1.74 (6H, m), 1.99-2.04 (4H, m), 2.31-2.35 (2H, m), 2.52 (2H, t, J=7.3 Hz), 3.22 (1H, m), 3.26 (3H, s), 3.40 (5H, m), 3.49-3.79 (11H, m), 4.18 (1H, m), 4.25 (1H, m), 4.41 (1H, m), 4.52-4.67 (9H, m, containing OH), 4.93 (1H, m), 5.24-5.40 (8H, m), 5.89-5.98 (3H, m), 7.13 (1H, d, J=9.5 Hz, NH).
FABMS (positive-ion) m/z; 1420 [M+Na]⁺.
HRFABMS; Calcd. for C₇₆H₁₃₆NO₁₉PNa: 1420.9342. Found: 1420.9341.

### (13) Diallylphosphono 4-O-(allyloxycarbonyl)-3-O-decyl-2-deoxy-6-O-{4-O-diallylphosphono-3-O-[(R)-3-methoxydecyl]-6-O-methyl-2-O-[(Z)-11-octadecenoyl]-β-D-glucopyranosyl}-2-(3-oxotetradecanoylamino)-α-D-glucopyranoside (Step Ca2)

4-O-(Allyloxycarbonyl)-3-O-decyl-2-deoxy-6-O-{4-O-diallylphosphono-3-O-[(R)-3-methoxydecyl]-6-O-methyl-2-O-[(Z)-11-octadecenoyl]-β-D-glucopyranosyl}-2-(3-oxotetradecanoylamino)-α-D-glucopyranoside obtained in (12) (167 mg, 0.119 mmol) was dissolved in methylene chloride (6 mL). To the solution were added sodium sulfate (350 mg), 1H-tetrazole (121 mg, 1.727 mmol) and diallyl diisopropylphosphoramidite (179 mg, 0.730 mmol). The mixture was stirred at room temperature for 1 hour in a nitrogen atmosphere. The reaction solution was directly purified by silica gel chromatography (eluent: cyclohexane/ethyl acetate =2/3) to give the phosphite as an oil (199 mg). The phosphite was dissolved in THF (7 mL), and a 30% hydrogen peroxide solution (0.4 mL) was added to the solution. The mixture was stirred for 1 hour under ice cooling. The reaction solution was diluted with ethyl acetate, washed with a 10% sodium thiosulfate solution, saturated sodium bicarbonate solution and brine, dried over magnesium sulfate, filtrated and concentrated in vacuo. The resulting residue was purified by silica gel chromatography (eluent: cyclohexane/ethyl acetate =2/3) to give the title compound as an oil (108 mg, yield 58%).
IR νₘₐₓ(film) 3290, 3085 (w), 2926, 2855, 1755, 1723 (w), 1678, 1650, 1552 cm⁻¹.
400 MHz ¹H NMR (CDCl₃) δ 0.86-0.89 (12H, m), 1.25 (60H, bs), 1.42-1.44 (4H, m), 1.55-1.74 (6H, m), 2.00-2.10 (4H, m), 2.33 (1H, m), 2.39 (1H, m), 2.49 (2H, t, J=7.3 Hz), 3.20 (1H, m), 3.25 (3H, s), 3.38 (3H, s), 3.39 (2H, s), 3.51-3.75 (10H, m), 3.95 (1H, m), 4.08 (1H, m), 4.26-4.34 (2H, m), 4.40 (1H, d, J=7.3 Hz, anomeric H), 4.58-4.64 (10H, m), 4.93 (1H, m), 4.76 (1H, dd, J=9.5, 10.3 Hz), 4.90 (1H, dd, J=8.1, 9.5 Hz), 5.24-5.42 (12H, m), 5.69 (1H, m, anomeric H), 5.89-6.03 (5H, m), 7.40 (1H, d, J=8.8 Hz, NH).
FABMS (positive-ion) m/z; ; 1379, 1580 [M+Na]⁺.
HRFABMS; Calcd. for C₈₂H₁₄₅NO₂₂PNa: 1580.9631. Found: 1580.9618.

### (14) Phosphono 3-O-decyl-2-deoxy-6-O-{3-O-[(R)-3-methoxydecyl]-6-O-methyl-2-O-[(Z)-11-octadecenoyl]-4-O-phosphono-β-D-glucopyranosyl}-2-(3-oxo-tetradecanoylamino)-α-D-glucopyranoside (Step Ca3)

Diallylphosphono 4-O-(allyloxycarbonyl)-3-O-decyl-2-deoxy-6-O-{4-O-diallylphosphono-3-O-[(R)-3-methoxydecyl]-6-O-methyl-2-O-[(Z)-11-octadecenoyl]-β-D-glucopyranosyl}-2-(3-oxotetradecanoylamino)-α-D-glucopyranoside obtained in (13) (95 mg, 0.061 mmol) was dissolved in dry THF (5 mL). To the solution were added triphenylphosphine (11 mg, 0.042 mmol), triethylamine (43 mg, 0.425 mmol), formic acid (36 mg, 0.782 mmol) and tetrakis(triphenylphosphine)palladium (0) (Pd(PPh₃)₄, 11 mg, 0.010 mmol). The mixture was stirred at 55°C for 20 hours in a nitrogen atmosphere. The reaction solution was filtrated and concentrated in vacuo. The resulting residue was purified on a column packed with 5 g of DEAE-cellulose (eluent: a 0.05 mol/L ammonium acetate solution of chloroform-methanol-water (2:3:1)) to give a fraction of the target substance. The obtained fraction was collected, and chloroform and a 0.15 mol/L hydrochloric acid solution were added thereto so that the entire mixture contained chloroform-methanol-water (1:1:1) (pH being adjusted to 2-3). The mixture was stirred in a separatory funnel, and the chloroform layer at the bottom was collected and concentrated in vacuo to give the title compound in the form of wax (67 mg, yield 84%).
IR νₘₐₓ(KBr) 3537, 3301, 2955, 2923, 2852, 1722, 1655, 1544, 1467 cm⁻¹.
400 MHz ¹H NMR (CDCl₃+CD₃OD, 4:1) δ 0.87-0.90 (12H, m), 1.26-1.28 (60H, m), 1.42-1.65 (8H, m), 1.71-1.76 (2H, m), 2.00-2.04 (4H, m), 2.30-2.43 (2H, m), 2.56 (2H, t, J=7.3 Hz), 3.26-3.46 (9H, m, containing two 3H, s, at 3.30 (C₆-OMe) and 3.41 (side chain OMe) ppm), 3.48-4.22 (15H, m), 4.52 (1H, d, J=8.1 Hz, anomeric H), 4.88 (1H, t, J=8.8 Hz, O-C₂-H), 5.34-5.36 (2H, m), 5.50 (1H, m, anomeric H). FABMS (negative-ion) m/z; 1312 [M-H]⁻.
HRFABMS; Calcd. for C₆₆H₁₂₄NO₂₀P₂: 1312.8192. Found: 1312.8104.
Anal. Calcd. for C₆₆H₁₂₅NO₂₀P₂3H₂O (1368.7): C, 57.92; H, 9.65; N, 1.02; P, 4.53. Found: C, 57.86; H, 9.55; N, 1.22; P, 4.30.

### [Example 2]

### Phosphono 3-O-decyl-2-deoxy-6-O-{3-O-[(R)-3-methoxydecyl]-6-O-methyl-2-O-[(Z)-11-octadecenyl]-4-O-phosphono-β-D-glucopyranosyl}-2-(3-oxotetradecanoylamino)-α-D-glucopyranoside (Compound No. 2)

### (1) (E)-1-Propenyl 4,6-O-isopropylidene-3-O-[(R)-3-methoxydecyl]-2-O-[(Z)-11-octadecenyl]-α,β-D-glucopyranoside (Step Aa5)

(E)-1-Propenyl 4,6-O-isopropylidene-3-O-[(R)-3-methoxydecyl]-α,β-D-glucopyranoside obtained in Reference Example 3 (3.140 g, 7.293 mmol) and (Z)-11-octadecenyl methanesulfonate (3.040 g, 48.175 mmol) were dissolved in DMF (20 mL). Sodium hydride (55% dispersion in oil, 400 mg, 9.167 mmol) was added to the solution under ice cooling. After stirring the reaction solution at room temperature for 1 hour and at 60°C for 1.5 hours, methanol was added thereto under ice cooling to decompose sodium hydride. The reaction solution was diluted with ethyl acetate, washed with water and saturated brine, dried over magnesium sulfate, filtrated and concentrated in vacuo. The resulting residue was purified by silica gel chromatography (eluent: cyclohexane/ethyl acetate =19/1, then 9/1) to give the title compound as an oil (4.000 g, yield 81 %).
IR νₘₐₓ(film) 2926, 2856, 1680 (w), 1661 (w), 1465 cm⁻¹.
400 MHz ¹H NMR (CDCl₃) δ 0.86-0.89 (6H, m), 1.29 (30H, bs), 1.40-1.80 (17H, m, containing two 3H, s, at 1.42 and 1.49 ppm), 2.01-2.04 (4H, m), 3.19-3.93 (14H, mcontaining 3H, s, at 3.34 ppm), 4.53 (0.3H, d, J=7.4 Hz, anomeric H), 5.07 (0.7H, d, J=3.5 Hz, anomeric H), 5.11-5.22 (1H, m), 5.34-5.38 (2H, m), 6.14-6.23 (1H, m).
FABMS (positive-ion) m/z; 681 [M+H]⁺, 703 [M+Na]⁺.
HRFABMS; Calcd. for C₄₁H₇₆O₇Na: 703.5490. Found: 703.5527.

### (2) (E)-1-Propenyl 3-O-[(R)-3-methoxydecyl]-2-O-[(Z)-11-octadecenyl]-α,β-D-glucopyranoside (Step Aa6)

(E)-1-Propenyl 4,6-O-isopropylidene-3-O-[(R)-3-methoxydecyl]-2-O-[(Z)-11-octadecenyl]-α,β-D-glucopyranoside obtained in (1) (4.000 g, 5.873 mmol) was dissolved in an 80% aqueous acetic acid solution (10 mL). The solution was stirred at 60°C for 2.5 hours and then concentrated in vacuo. The resulting residue was purified by silica gel column chromatography (eluent: cyclohexane/ethyl acetate=2/1, then 2/3) to give the title compound as an oil (2.310 g, yield 61 %).
IR νₘₐₓ(film) 3423 (br), 2926, 2856, 2525 (br), 1680 (w), 1464 cm⁻¹.
400 MHz ¹H NMR (CDCl₃) δ 0.88 (6H, t, J=6.6 Hz), 1.27 (30H, bs), 1.45-1.61 (9H, m, containing two 3H, dd, J=1.6, 6.6 Hz at 1.56 ppm), 1.71-1.80 (2H, m), 1.99-2.08 (4H, m), 3.18 (1H, m), 3.23-3.92 (12H, mcontaining 3H, s, at 3.32 ppm), 4.10 (1H, m), 4.52 (0.3H, d, J=7.0 Hz, anomeric H), 5.11 (0.7H, d, J=3.1 Hz, anomeric H),
5.20 (1H, m), 5.34-5.36 (2H, m), 6.16-6.24 (1H, m).
FABMS (positive-ion) m/z; 641 [M+H]⁺, 663 [M+Na]⁺.
HRFABMS; Calcd. for C₃₈H₇₂O₇Na: 663.5176. Found: 663.5182.

### (3) (E)-1-Propenyl 6-O-(t-butyldimethylsilyl)-3-O-[(R)-3-methoxydecyl]-2-O-[(Z)-11-octadecenyl]-α,β-D-glucopyranoside (Step Aa7)

A reaction was performed in the same manner as in Reference Example 6 using (E)-1-propenyl 3-O-[(R)-3-methoxydecyl]-2-O-[(Z)-11-octadecenyl]-α,β-D-glucopyranoside obtained in (2) to give the title compound as an oil (yield 93%).
IR νₘₐₓ(film) 3432 (br), 2927, 2856, 1681 (w), 1661 (w), 1463 cm⁻¹.
400 MHz ¹H NMR (CDCl₃) δ 0.07 (6H, s), 0.86-0.92 (15H, m), 1.24-1.40 (30H, m), 1.41-1.60 (9H, m), 1.75-1.79 (2H, m), 1.99-2.05 (4H, m), 3.13-4.03 (14H, m, containing 3H, s, at 3.31 ppm), 4.46 (0.4H, d, J=7.4 Hz, anomeric H), 5.08-5.21 (1.6H, m, containing 0.6H, anomeric H), 5.33-5.38 (2H, m), 6.18-6.23 (1H, m). FABMS (positive-ion) m/z; 755 [M+H]⁺.
HRFABMS (on addition of NaI), Calcd. for C₄₄H₈₆O₇SiNa: 777.6040. Found: 777.6022.

### (4) (E)-1-Propenyl 6-O-(t-butyldimethylsilyl)-4-O-diallylphosphono-3-O-[(R)-3-methoxydecyl]-2-O-[(Z)-11-octadecenyl]-α,β-D-glucopyranoside (Step Aa8)

A reaction was performed in the same manner as in Reference Example 7 using (E)-1-propenyl 6-O-(t-butyldimethylsilyl)-3-O-[(R)-3-methoxydecyl]-2-O-[(Z)-11-octadecenyl]-α,β-D-glucopyranoside obtained in (3) to give the title compound as an oil (yield 92%).
IR νₘₐₓ(film) 2928, 2856, 1681 (w), 1463 cm⁻¹.
400 MHz ¹H NMR (CDCl₃) δ 0.05 (6H, s), 0.86-0.92 (15H, m), 1.24-1.28 (30H, bs), 1.43-1.50 (2H, m), 1.53-1.62 (7H, m), 1.78-1.81 (2H, m), 1.99-2.04 (4H, m), 3.18-4.20 (14H, m), 4.46 (0.4H, d, J=7.8 Hz, anomeric H), 4.54-4.58 (4H, m), 5.09-5.39 (8H, m), 5.90-5.98 (2H, m), 6.18-6.23 (1H, m).
FABMS (positive-ion) m/z; 915 [M+H]⁺, 937 [M+Na]⁺.
HRFABMS; Calcd. for C₅₀H₉₆O₁₀Si: 915.6510. Found: 915.6527.

### (5) (E)-1-Propenyl 4-O-diallylphosphono-3-O-[(R)-3-methoxydecyl]-2-O-[(Z)-11-octadecenyl]-α,β-D-glucopyranoside (Step Aa9)

A reaction was performed in the same manner as in Reference Example 8 using (E)-1-propenyl 6-O-(t-butyldimethylsilyl)-4-O-diallylphosphono-3-O-[(R)-3-methoxydecyl]-2-O-[(Z)-11-octadecenyl]-α,β-D-glucopyranoside obtained in (4) to give the title compound as an oil (yield 100%).
IR νₘₐₓ(film) 3431 (br), 2927, 2856, 1680 (w), 1661 (w), 1462 cm⁻¹.
400 MHz ¹H NMR (CDCl₃) δ 0.88 (6H, t, J=6.8 Hz), 1.26 (30H, bs), 1.40-1.44 (2H, m), 1.54-1.61 (7H, m), 1.73-1.78 (2H, m), 1.99-2.03 (4H, m), 3.20-3.39 (5H, m, containing 3H, s, at 3.29 ppm), 3.56-3.93 (8H, m), 4.31 (1H, m), 4.47 (0.4H, d, J=7.8 Hz, anomeric H), 4.55-4.58 (2H, m), 4.61-4.65 (2H, m), 5.08-5.21 (1.6H, m, containing 0.6H, d, J=3.9 Hz, at 5.13 ppm, anomeric H), 5.25-5.41 (6H, m), 5.90-5.98 (2H, m), 6.17-6.22 (1H, m).
FABMS (positive-ion) m/z; 801 [M+H]⁺, 823 [M+Na]⁺.
HRFABMS; Calcd. for C₄₄H₈₁O₁₀PNa: 823.5465. Found: 823.5477.

### (6) (E)-1-Propenyl 4-O-diallylphosphono-3-O-[(R)-3-methoxydecyl]-6-O-methyl-2-O-[(Z)-11-octadecenyl]-α,β-D-glucopyranoside (Step Aa10)

A reaction was performed in the same manner as in Example 1(1) using (E)-1-propenyl 4-O-diallylphosphono-3-O-[(R)-3-methoxydecyl]-2-O-[(Z)-11-octadecenyl]-α,β-D-glucopyranoside obtained in (5) to give the title compound as an oil (yield 87%).
IR νₘₐₓ(film) 2926, 2856, 1681 (w), 1661 (w), 1459 cm⁻¹. 400 MHz ¹H NMR (CDCl₃) δ 0.88 (6H, t, J=6.6 Hz), 1.26 (30H, bs), 1.40-1.46 (2H, m), 1.51-1.60 (7H, m), 1.74-1.80 (2H, m), 1.98-2.01 (4H, m), 3.19-3.91 (16H, m, containing two 3H, s, at 3.28 and 3.37 ppm), 4.42 (0.6H, q, J=9.4 Hz), 4.34 (0.4H, q, J=9.4 Hz), 4.45 (0.4H, d, J=7.8 Hz), 4.53-4.58 (4H, m), 5.06-5.38 (7.6H, m), 5.88-5.97 (2H, m), 6.16-6.22 (1H, m).
FABMS (positive-ion) m/z; 815 [M+H]⁺, 837 [M+Na]⁺.
HRFABMS; Calcd. for C₄₅H₈₃O₁₀PNa: 837.5621. Found: 837.5635.

### (7) 4-O-Diallylphosphono-3-O-[(R)-3-methoxydecyl]-6-O-methyl-2-O-[(Z)-11-octadecenyl]-α,β-D-glucopyranose (Step Aa11)

A reaction was performed in the same manner as in Example 1(2) using (E)-1-propenyl 4-O-diallylphosphono-3-O-[(R)-3-methoxydecyl]-6-O-methyl-2-O-[(Z)-11-octadecenyl]-α,β-D-glucopyranoside obtained in (6) to give the title compound as an oil (yield 75%).
IR νₘₐₓ(film) 3354 (br), 2927, 2856, 1464 cm⁻¹.
400 MHz ¹H NMR (CDCl₃) δ 0.88 (6H, t, J=6.6 Hz), 1.26 (32H, bs), 1.40-1.45 (2H, m), 1.57-1.61 (2H, m), 1.75-1.81 (2H, m), 1.99-2.04 (4H, m), 3.09-3.40 (16H, m, containing two 3H, s, at 3.29 and 3.39 ppm), 3.54-3.88 (7H, m), 4.04-4.29 (3H, m), 4.54-4.65 (4H, m), 5.24-5.40 (6H, m), 5.91-5.99 (2H, m).
FABMS (positive-ion) m/z; 775 [M+H]⁺, 797 [M+Na]⁺.
HRFABMS; Calcd. for C₄₂H₇₉O₁₀PNa: 797.5309. Found: 797.5279.

### (8) Trichloroacetimidoyl 4-O-diallylphosphono-3-O-[(R)-3-methoxydecyl]-6-O-methyl-2-O-[(Z)-11-octadecenyl]-α,β-D-glucopyranose (Step Aa12)

A reaction was performed in the same manner as in Example (3) using 4-O-diallylphosphono-3-O-[(R)-3-methoxydecyl]-6-O-methyl-2-O-[(Z)-11-octadecenyl]-α,β-D-glucopyranose obtained in (7) to give the title compound as an oil (yield 100%).
IR νₘₐₓ(film) 3350-3000, 2927, 2856, 1733, 1674, 1465 cm⁻¹.
400 MHz ¹H NMR (CDCl₃) δ 0.88 (6H, t, J=6.6 Hz), 1.24-1.55 (34H, m), 1.77-1.80 (2H, m), 1.99-2.05 (4H, m), 3.25-4.00 (16H, m, containing two 3H, s, at 3.30 and 3.38 ppm), 4.37-4.48 (1H, m), 4.56-4.62 (4H, m), 5.24-5.41 (6H, m), 5.67 (0.5H, d, J=7.4 Hz, anomeric H), 5.90-6.00 (2H, m), 6.50 (0.5H, d, J=3.2 Hz, anomeric H), 8.59 (0.5H, s), 8.66 (0.5H, s).
FABMS (positive-ion) m/z; 940 [M+Na]⁺.

### (9) 4-O-(Allyloxycarbonyl)-3-O-decyl-2-deoxy-6-O-(4-O-diallylphosphono-3-O-[(R)-3-methoxydecyl]-6-O-methyl-2-O-[(Z)-11-octadecenyl]-α-D-glucopyranosyl}-2-(3-oxotetradecanoylamino)-α-D-glucopyranose (α-anomer) and 4-O-(allyloxycarbonyl)-3-O-decyl-2-deoxy-6-O-{4-O-diallylphosphono-3-O-[(R)-3-methoxydecyl]-6-O-methyl-2-O-[(Z)-11-octadecenyl]-β-D-glucopyranosyl}-2-(3-oxotetradecanoylamino)-α-D-glucopyranose (β-anomer) (Step Ca1)

A reaction was performed in the same manner as in Example 1(12) using trichloroacetimidoyl 4-O-diallylphosphono-3-O-[(R)-3-methoxydecyl]-6-O-methyl-2-O-[(Z)-11-octadecenyl]-α,β-D-glucopyranoside obtained in (8) to give an α-anomer (66 mg, yield 15%) and a β-anomer (143 mg, yield 32%) of the title compound as an oily substance.
physical constant of α-anomer:
IR νₘₐₓ(film) 3450-3300, 2926, 2854, 1751, 1723 (w), 1646, 1554, 1465 cm⁻¹.
400 MHz ¹H NMR (CDCl₃) δ 0.86-0.89 (12H, m), 1.26 (62H, bs), 1.40-1.45 (4H, m), 1.55-1.59 (4H, m), 1.70-1.80 (2H, m), 1.99-2.03 (4H, m), 2.52 (2H, t, J=7.2 Hz), 3.20-3.91 (25H, m, containing two 3H, s, and 2H, s, at 3.28, 3.38 and 3.39, respectively), 4.13-4.24 (2H, m), 4.37 (1H, m, OH), 4.52-4.63 (6H, m), 4.87 (1H, d, J=3.5 Hz, anomeric H), 5.13 (1H, t, J=3.1 Hz, changed to a doublet on addition of D₂O, anomeric H), 5.23-5.40 (8H, m), 5.88-5.98 (3H, m), 7.08 (1H, d, J=9.4 Hz, NH).
FABMS (positive-ion) m/z; 1406 [M+Na]⁺.
HRFABMS; Calcd. for C₇₆H₁₃₈NO₁₈PNa: 1406.9550. Found: 1406.9556.
physical constant of β-anomer:
IR νₘₐₓ(film) 3307 (w), 2926, 2855, 1754, 1722, 1645, 1555 cm⁻¹.
400 MHz ¹H NMR (CDCl₃) δ 0.88 (12H, t, J=6.6 Hz), 1.26 (62H, bs), 1.40-1.50 (4H, m), 1.50-1.60 (4H, m), 1.75-1.79 (2H, m), 1.99-2.02 (4H, m), 2.52 (2H, t, J=7.4 Hz), 3.16-3.88 (25H, m, containing two 3H, s, and 2H, s, at 3.28, 3.38 and 3.40, respectively), 4.20 (1H, m), 4.31 (1H, m), 4.37 (1H, d, J=7.0 Hz, anomeric H), 4.54-4.64 (6H, m), 5.20 (1H, m, changed to a doublet, J=3.1 Hz, on addition of D₂O, anomeric H), 5.25-5.39 (8H, m), 5.89-5.98 (3H, m), 7.12 (1H, d, J=9.3 Hz, NH).
FABMS (positive-ion) m/z; 1406 [M+Na]⁺.
HRFABMS; Calcd. for C₇₆H₁₃₈NO₁₈PNa: 1406.9550. Found: 1406.9575.

### (10) Diallylphosphono 4-O-(allyloxycarbonyl)-3-O-decyl-2-deoxy-6-O-{4-O-diallylphosphono-3-O-[(R)-3-methoxydecyl]-6-O-methyl-2-O-[(Z)-11-octadecenyl]-β-D-glucopyranosyl}-2-(3-oxotetradecanoylamino)-α-D-glucopyranoside (Step Ca2)

A reaction was performed in the same manner as in Example 1(13) using 4-O-(allyloxycarbonyl)-3-O-decyl-2-deoxy-6-O-{4-O-diallylphosphono-3-O-[(R)-3-methoxydecyl]-6-O-methyl-2-O-[(Z)-11-octadecenyl]-β-D-glucopyranosyl}-2-(3-oxotetradecanoylamino)-α-D-glucopyranose obtained in (9) to give the title compound as an oil (yield 58%).
IR νₘₐₓ(film) 3293, 3086 (w), 2926, 2856, 1757, 1721, 1678, 1650, 1552, 1464 cm⁻¹. 400 MHz ¹H NMR (CDCl₃) δ 0.86-0.89 (12H, m), 1.25 (62H, bs), 1.40-1.49 (4H, m), 1.52-1.60 (4H, m), 1.74-1.79 (2H, m), 2.00-2.13 (4H, m), 2.48-2.54 (2H, m), 3.11 (1H, dd, J=7.8, 8.6 Hz), 3.23 (1H, m), 3.28 (3H, s), 3.31-3.38 [22H, m, containing 3H, s, 2H, s, and 1H, d, J=7.4 Hz (anomeric H), at 3.37, 3.40, and 4.27 ppm, respectively], 4.54-4.65 (10H, m), 4.84 (1H, m), 5.23-5.42 (12H, m), 5.72 (1H, dd, J=3.1, 6.3 Hz, anomeric H), 5.87-6.01 (5H, m), 7.41 (1H, d, J=8.6 Hz, NH).
FABMS (positive-ion) m/z; 1366, 1406, 1566 [M+Na]⁺.
HRFABMS; Calcd. for C₈₂H₁₄₇NO₂₁P₂Na: 1566.9838. Found: 1566.9841.

### (11) Phosphono 3-O-decyl-2-deoxy-6-O-{3-O-[(R)-3-methoxydecyl]-6-O-methyl-2-O-[(Z)-11-octadecenyl]-4-O-phosphono-β-D-glucopyranosyl}-2-(3-oxotetradecanoylamino)-α-D-glucopyranoside (Step Ca3)

A reaction was performed in the same manner as in Example 1(14) using diallylphosphono 4-O-(allyloxycarbonyl)-3-O-decyl-2-deoxy-6-O-{4-O-diallylphosphono-3-O-[(R)-3-methoxydecyl]-6-O-methyl-2-O-[(Z)-11-octadecenyl]-β-D-glucopyranosyl}-2-(3-oxotetradecanoylamino)-α-D-glucopyranoside obtained in (10) to give the title compound in the form of wax (yield 60%).
IR νₘₐₓ(KBr) 3296 (w, br), 2924, 2853, 1717, 1649, 1546, 1466 cm⁻¹.
400 MHz ¹H NMR (CDCl₃+CD₃OD, 4:1) δ 0.86-0.90 (12H, m), 1.26-1.28 (62H, bs), 1.51-1.60 (8H, m), 1.81-1.85 (2H, m), 1.99-2.04 (4H, m), 2.54-2.58 (2H, m), 3.14 (1H, m), 3.28-4.11 (26H, m, containing two 3H, s, at 3.34 (C₆-OMe) and 3.40 (side chain OMe) ppm), 4.39 (1H, d, J=7.4 Hz, anomeric H), 5.34-5.39 (2H, m, olefinic H), 5.53 (1H, dd, J=3.1, 6.9 Hz, anomeric H).
FABMS (negative-ion) m/z; 1298 [M-H]⁻.
Anal. Calcd. for C₆₆H₁₂₇NO₁₉P₂ (1300.7): C, 60.95; H, 9.84; N, 1.08; P, 4.76. Found: C, 60.78; H, 9.61; N, 1.32; P, 4.53.

### [Example 3]

Phosphono 3-O-decyl-2-deoxy-6-O-{3-O-[(R)-3-methoxydecyl]-2-O-[(Z)-11-octadecenoyl]-4-O-phosphono-β-D-glucopyranosyl}-2-(3-oxotetradecanoylamino)-α-D-glucopyranoside (Compound No. 3)

### (1) (E)-1-Propenyl 6-O-(allyloxycarbonyl)-3-O-[(R)-3-methoxydecyl]-2-O-[(Z)-11-octadecenoyl]-α,β-D-glucopyranoside (Step Ab1)

(E)-1-Propenyl 3-O-[(R)-3-methoxydecyl]-2-O-[(Z)-11-octadecenoyl]-α,β-D-glucopyranoside (1.49 g, 2.275 mmol) and pyridine (5 g) were dissolved in methylene chloride (90 mL). Allyl chloroformate (5.00 g, 41.48 mmol) was added dropwise to the solution at 0°C, and the mixture was stirred at the same temperature for 30 minutes. The reaction solution was diluted with methylene chloride, washed with sodium bicarbonate solution and brine, dried over magnesium sulfate, filtrated and concentrated in vacuo. The resulting residue was purified by silica gel chromatography (eluent: cyclohexane/ethyl acetate =4/1) to give an anomer mixture of the title compound as an oil (1.52 g, yield 90%). Part of the mixture could be separated into an α-anomer and a β-anomer using silica gel thin layer chromatography.
α-anomer (Rf=0.353, cyclohexane: EtOAc = 4:1):
IR νₘₐₓ(film) 3500-3400, 2927, 2856, 1749, 1680 (w), 1660 (w) cm⁻¹.
400 MHz ¹H NMR (CDCl₃) δ 0.88 (6H, t, J=6.6 Hz), 1.28 (30H, bs), 1.40-1.48 (4H, m), 1.54 (3H, dd, J=1.6, 7.0 Hz), 1.57-1.65 (2H, m), 1.67-1.76 (2H, m), 1.98-2.03 (4H, m), 2.31-2.40 (2H, m), 3.30 (3H, s), 3.34 (1H, m), 3.55 (1H, m), 3.65-3.74 (3H, m), 3.83 (1H, m), 3.95 (1H, m), 4.40 (1H, d, J=3.5 Hz, anomeric H), 4.63 (1H, td, J=1.2, 5.0 Hz), 4.72 (1H, dd, J=3.5, 10.2 Hz), 5.15 (1H, m), 5.22 (1H, d, J=3.9 Hz), 5.25-5.39 (4H, m), 5.93 (1H, m), 6.11 (1H, dd, J=1.6, 12.1 Hz).
FABMS (positive-ion) m/z; 681, 739 [M+H]⁺, 761 [M+Na]⁺.
β-anomer (Rf=0.265, cyclohexane:EtOAc=4:1):
IR νₘₐₓ(film) 3500-3400, 2927, 2856, 1752, 1682 (w), 1662 (w) cm⁻¹. 400 MHz ¹H NMR (CDCl₃) δ 0.88 (6H, t, J=6.6 Hz), 1.27 (30H, bs), 1.40-1.48 (4H, m), 1.53 (3H, d, J=7.0 Hz), 1.60-1.64 (2H, m), 1.68-1.72 (2H, m), 1.99-2.10 (4H, m), 2.31-2.34 (2H, m), 3.29 (3H, s), 3.31-3.39 (2H, m), 3.56-3.64 (3H, m), 3.80-3.85 (2H, m), 4.36 (1H, m), 4.50 (1H, d, J=11.7 Hz), 4.56 (1H, d, J=7.8 Hz), 4.64 (1H, dd, J=1.0, 5.7 Hz), 4.98 (1H, t, J=9.0 Hz), 5.08 (1H, m), 5.27-5.39 (4H, m), 5.93 (1H, m), 6.18 (1H, d, J=12.0 Hz).
FABMS (positive-ion) m/z; 681, 761 [M+Na]⁺.

### (2) (E)-1-Propenyl 6-O-(allyloxycarbonyl)-4-O-diallylphosphono-3-O-[(R)-3-methoxydecyl]-2-O-[(Z)-11-octadecenoyl]-α,β-D-glucopyranoside (Step Ab2)

A reaction was performed in the same manner as in Reference Example 7 using (E)-1-propenyl 6-O-(allyloxycarbonyl)-3-O-[(R)-3-methoxydecyl]-2-O-[(Z)-11-octadecenoyl]-α,β-D-glucopyranoside obtained in (1) to give an anomer mixture of the title compound as an oil (yield 95%). Part of the mixture could be separated into an α-anomer and a β-anomer using silica gel thin layer chromatography.
α-anomer (Rf=0.395, cyclohexane:EtOAc=3:1):
IR νₘₐₓ(film) 2927, 2856, 1750, 1680 (w) cm⁻¹.
400 MHz ¹H NMR (CDCl₃) δ 0.88 (6H, t, J=6.6 Hz), 1.27 (30H, bs), 1.40-1.48 (2H, m), 1.54 (3H, d, J=5.8 Hz), 1.61-1.78 (4H, m), 1.99-2.04 (4H, m), 2.32-2.40 (2H, m), 3.25 (1H, m), 3.26 (3H, s), 3.77-3.94 (4H, m), 4.31-4.38 (2H, m), 4.46 (1H, dd, J=2.0, 11.7 Hz), 4.56-4.63 (6H, m), 4.76 (1H, dd, J=3.9, 9.8 Hz), 5.17 (1H, m), 5.20 (1H, d, J=3.9 Hz), 5.24-5.39 (8H, m), 5.89-5.98 (3H, m), 6.10 (1H, dd, J=2.0, 12.7 Hz). FABMS (positive-ion) m/z; 899 [M+H]⁺, 921 [M+Na]⁺.
HRFABMS; Calcd. for C₄₈H₈₃O₈PNa: 921.5469. Found: 921.5450.
β-anomer (Rf=0.303, cyclohexane:EtOAc=3:1):
400 MHz ¹H NMR (CDCl₃) δ 0.86-0.88 (6H, m), 1.26 (30H, bs), 1.40-1.48 (2H, m), 1.53 (3H, d, J=8.8 Hz), 1.60-1.76 (4H, m), 1.99-2.08 (4H, m), 2.32-2.39 (2H, m), 3.23 (1H, m), 3.26 (3H, m), 3.58 (1H, t, J=8.8 Hz), 3.66-3.80 (3H, m), 4.32-4.38 (2H, m), 4.53-4.59 (7H, m), 4.62 (1H, d, J=5.9 Hz), 5.01 (1H, t, J=8.3 Hz), 5.08 (1H, m), 5.25-5.39 (8H, m), 5.90-5.97 (3H, m), 6.16 (1H, dd, J=2.0, 12.7 Hz).

### (3) 6-O-(Allyloxycarbonyl)-4-O-diallylphosphono-3-O-[(R)-3-methoxydecyl]-2-O-[(Z)-11-octadecenoyl]-α,β-D-glucopyranoside (Step Ab3)

A reaction was performed in the same manner as in Example 1(2) using (E)-1-propenyl 6-O-(allyloxycarbonyl)-4-O-diallylphosphono-3-O-[(R)-3-methoxydecyl]-2-O-[(Z)-11-octadecenoyl]-α,β-D-glucopyranoside obtained in (2) to give the title compound as an oil (yield 81%).
IR νₘₐₓ(film) 3324, 2927, 2856, 1750, 1460 cm⁻¹.
400 MHz ¹H NMR (CDCl₃) δ 0.88 (6H, t, J=6.6 Hz), 1.27 (30H, bs), 1.42-1.50 (2H, m), 1.58-1.77 (4H, m), 1.99-2.02 (4H, m), 2.36-2.40 (2H, m), 2.86 (1H, bs, OH), 3.25 (1H, m), 3.26 (3H, s), 3.76-3.79 (2H, m), 3.87 (1H, t, J=9.2 Hz), 4.20 (1H, m), 4.28-4.37 (3H, m), 4.51-4.64 (7H, m), 4.76 (1H, dd, J=3.5, 9.8 Hz), 5.24-5.39 (8H, m), 5.88-5.99 (3H, m).
FABMS (positive-ion) m/z; 859 [M+H]⁺, 881 [M+Na]⁺.
HRFABMS; Calcd. for C₄₅H₇₉O₁₃PNa: 881.5156. Found: 881.5153.

### (4) Trichloroacetimidoyl 6-O-(allyloxycarbonyl)-4-O-diallylphosphono-3-O-[(R)-3-methoxydecyl]-2-O-[(Z)-11-octadecenoyl]-α,β-D-glucopyranoside (Step Ab4)

A reaction was performed in the same manner as in Example 1(3) using 6-O-(allyloxycarbonyl)-4-O-diallylphosphono-3-O-[(R)-3-methoxydecyl]-2-O-[(Z)-11-octadecenoyl]-α,β-D-glucopyranoside obtained in (3) to give the title compound as an oil (yield 98%, a 7:3 anomer mixture).
IR νₘₐₓ(film) 3348 (w), 2928, 2856, 1752, 1677, 1651 (w), 1460 cm⁻¹. 400 MHz ¹H NMR (CDCl₃) δ 0.86-0.90 (6H, m), 1.24-1.28 (30H, m), 1.41-1.45 (2H, m), 1.58-1.77 (4H, m), 1.99-2.04 (4H, m), 2.28-2.32 (2H, m), 3.24 (1H, m), 3.26 (3H, s), 3.67-4.15 (5H, m), 4.36-4.62 (8H, m), 4.99 (1H, dd, J=3.5, 8.8 Hz), 5.24-5.40 (8H, m), 5.83 (0.3H, d, J=7.4 Hz, anomeric H), 5.88-5.99 (3H, m), 6.49 (0.7H, d, J=3.9 Hz, anomeric H), 8.63 (0.7H, s), 8.65 (0.3H, s).
FABMS (positive-ion) m/z; 1026, 1024 [M+Na]⁺.

### (5) 4-O-(Allyloxycarbonyl)-3-O-decyl-2-deoxy-6-O-{6-O-(allyloxycarbonyl)-4-O-diallylphosphono-3-O-[(R)-3-methoxydecyl]-2-O-[(Z)-11-octadecenoyl]-β-D-glucopyranosyl}-2-(3-oxotetradecanoylamino)-α-D-glucopyranose (Step Ca1)

A reaction was performed in the same manner as in Example 1(12) using trichloroacetimidoyl 6-O-(allyloxycarbonyl)-4-O-diallylphosphono-3-O-[(R)-3-methoxydecyl]-2-O-[(Z)-11-octadecenoyl]-α,β-D-glucopyranoside obtained in (4) to give the title compound as an oil (yield 54%).
IR νₘₐₓ(film) 3293, 3085 (w), 2924, 2854, 1752, 1729 (shoulder), 1707 (shoulder), 1635, 1557, 1466 cm⁻¹.
400 MHz ¹H NMR (CDCl₃) δ 0.86-0.89 (12H, m), 1.26 (60H, bs), 1.40-1.50 (4H, m), 1.52-1.75 (6H, m), 2.01-2.04 (4H, m), 2.33-2.35 (2H, m), 2.50-2.54 (2H, m), 3.25 (1H, m), 3.26 (3H, s), 3.39 (2H, s), 3.44-3.76 (11H, m), 4.11-4.65 (14H, m), 4.92
(1H, t, J=7.0 Hz), 5.18 (1H, s), 5.24-5.39 (10H, m), 5.90-6.00 (4H, m), 7.13 (1H, d, J=9.4 Hz, NH).
FABMS (positive-ion) m/z; 1490 [M+Na]⁺.
HRFABMS; Calcd. for C₇₉H₁₃₈NO₂₁PNa: 1490.9397. Found: 1490.9419.

### (6) Diallylphosphono 4-O-(allyloxycarbonyl)-3-O-decyl-2-deoxy-6-O-{6-O-(allyloxycarbonyl)-4-O-diallylphosphono-3-O-[(R)-3-methoxydecyl]-2-O-[(Z)-11-octadecenoyl]-β-D-glucopyranosyl}-2-(3-oxotetradecanoylamino)-α-D-glucopyranoside (Step Ca2)

A reaction was performed in the same manner as in Example 1(13) using 4-O-(allyloxycarbonyl)-3-O-decyl-2-deoxy-6-O-{6-O-(allyloxycarbonyl)-4-O-diallylphosphono-3-O-[(R)-3-methoxydecyl]-2-O-[(Z)-11-octadecenoyl]-β-D-glucopyranosyl}-2-(3-oxotetradecanoylamino)-α-D-glucopyranose obtained in (5) to give the title compound as an oil (yield 70%).
IR νₘₐₓ(film) 3289 (w), 3086 (w), 2926, 2856, 1754, 1679, 1650, 1552, 1462 cm⁻¹. 400 MHz ¹H NMR (CDCl₃) δ 0.86-0.89 (12H, m), 1.25 (60H, bs), 1.40-1.46 (4H, m), 1.50-1.70 (6H, m), 1.99-2.03 (4H, m), 2.34 (1H, m), 2.40 (1H, m), 2.42-2.51 (2H, m), 3.22 (1H, m), 3.25 (3H, s), 3.39 (2H, s), 3.51-3.72 (10H, m), 3.92 (1H, m), 4.08 (1H, m), 4.25-4.35 (2H, m), 4.42 (1H, d, J=8.2 Hz, anomeric H), 4.57-4.65 (12H, m), 4.76 (1H, m), 4.90 (1H, m), 5.24-5.42 (14H, m), 5.70 (1H, m, anomeric H), 5.90-5.99 (6H, m), 7.38 (1H, d, J=8.2 Hz, NH).
FABMS (positive-ion) m/z; 1450, 1650 [M+Na]⁺.
HRFABMS; Calcd. for C₈₅H₁₄₇NO₂₄P₂Na: 1650.9686. Found: 1650.9692.

### (7) Phosphono 3-O-decyl-2-deoxy-6-O-{3-O-[(R)-3-methoxydecyl]-2-O-[(Z)-11-octadecenoyl]-4-O-phosphono-β-D-glucopyranosyl}-2-(3-oxotetradecanoylamino)-α-D-glucopyranoside (Step Ca3)

A reaction was performed in the same manner as in Example 1(14) using diallylphosphono 4-O-(allyloxycarbonyl)-3-O-decyl-2-deoxy-6-O-{6-O-(allyloxycarbonyl)-4-O-diallylphosphono-3-O-[(R)-3-methoxydecyl]-2-O-[(Z)-11-octadecenoyl]-β-D-glucopyranosyl}-2-(3-oxotetradecanoylamino)-α-D-glucopyranoside obtained in (6) to give the title compound in the form of wax (yield 57%).
IR νₘₐₓ(KBr) 3299 (br), 2955, 2924, 2853, 1722, 1650, 1545, 1467 cm⁻¹.
400 MHz ¹H NMR (CDCl₃+CD₃OD, 5:1) δ 0.87-0.90 (12H, m), 1.26 (60H, bs), 1.42-1.70 (8H, m), 1.70-1.76 (2H, m), 1.98-2.02 (4H, m), 2.35-2.40 (2H, m), 2.54-2.57 (2H, m), 3.26-4.11 (21H, m, containing 3H, s, and 2H, s, at 3.31 and 3.38 ppm, respectively), 4.24 (1H, m), 4.50 (1H, d, J=7.4 Hz, anomeric H), 4.86 (1H, m), 5.35-5.39 (2H, m), 5.49 (1H, m, anomeric H).
FABMS (negative-ion) m/z; 1298 [M-H]⁻.
Anal. Calcd. for C₆₅H₁₂₃NO₂₀P₂·3H₂O: C, 57.63; H, 9.60; N, 1.03; P, 4.57. Found: C, 57.59; H, 9.38; N, 1.29; P, 4.49.

### [Example 4]

Phosphono 3-O-decyl-2-deoxy-6-O-{3-O-[(R)-3-methoxydecyl]-2-O-[(Z)-11-octadecenyl]-4-O-phosphono-β-D-glucopyranosyl}-2-(3-oxotetradecanoylamino)-α-D-glucopyranoside (Compound No. 4)

### (1) (E)-1-Propenyl 6-O-(allyloxycarbonyl)-3-O-[(R)-3-methoxydecyl]-2-O-[(Z)-11-octadecenyl]-α,β-D-glucopyranoside (Step Ab1)

A reaction was performed in the same manner as in Example 3(1) using (E)-1-propenyl 6-O-(t-butyldimethylsilyl)-3-O-[(R)-3-methoxydecyl]-2-O-[(Z)-11-octadecenyl]-α,β-D-glucopyranoside (a 1:1 anomer mixture) obtained in Example 2(3) to give the title compound as an oil (yield 64%).
IR νₘₐₓ(film) 3500-3400, 2927, 2856, 1752, 1681 (w), 1661 (w), 1458 cm⁻¹.
400 MHz ¹H NMR (CDCl₃) δ 0.88 (6H, t, J=6.6 Hz), 1.24-1.38 (30H, m), 1.42-1.49 (4H, m), 1.54-1.62 (7H, m), 1.71-1.79 (2H, m), 1.99-2.05 (4H, m), 3.18 (1H, m), 3.27-3.82 (11H, m, containing 3H, s, at 3.31 ppm), 4.08 (1H, m), 4.30-4.50 (1.5H, m, containing 0.5H anomeric H), 4.62-4.64 (2H, m), 5.08-5.23 (1.5H, m, containing 0.5H, d, J=3.5 Hz, at 5.12 ppm, anomeric H), 5.25-5.39 (4H, m), 5.88-5.98 (1H, m), 6.13-6.24 (1H, m).
FABMS (positive-ion) m/z; 725 [M+H]⁺, 747 [M+Na]⁺.

### (2) (E)-1-Propenyl 6-O-(allyloxycarbonyl)-4-O-diallylphosphono-3-O-[(R)-3-methoxydecyl]-2-O-[(Z)-11-octadecenyl]-α,β-D-glucopyranoside (Step Ab2)

A reaction was performed in the same manner as in Reference Example 7 using (E)-1-propenyl 6-O-(allyloxycarbonyl)-3-O-[(R)-3-methoxydecyl]-2-O-[(Z)-11-octadecenyl]-α,β-D-glucopyranoside (a 1:1 anomer mixture) obtained in (1) to give the title compound as an oil (yield 85%, a 3:2 anomer mixture).
IR νₘₐₓ(film) 2927, 2856, 1753, 1680 (w), 1660-1651 (w), 1460 cm⁻¹.
400 MHz ¹H NMR (CDCl₃) δ 0.88 (6H, t, J=6.6 Hz), 1.26 (32H, bs), 1.40-1.45 (2H, m), 1.54-1.60 (5H, m, containing 3H, dd, J=1.6, 6.6 Hz, at 1.55 ppm), 1.75-1.80 (2H, m), 1.99-2.05 (4H, m), 3.24 (1H, m), 3.29 (3H, s), 3.31-3.38 (1H, m), 3.56-3.92 (7H, m), 4.21-4.52 (2.4H, m, containing 0.4H, d, J=7.8 Hz, at 4.47 ppm, anomeric H), 4.56-4.63 (6H, m), 5.09-5.20 (1.6H, m, containing 0.6H, d, J=3.9 Hz, at 5.10 ppm, anomeric H), 5.23-5.39 (8H, m), 5.87-5.97 (3H, m), 6.16-6.20 (1H, m).
FABMS (positive-ion) m/z; 461, 885 [M+H]⁺, 907 [M+Na]⁺.
HRFABMS; Calcd. for C₄₈H₈₅O₁₂PNa: 907.5677. Found: 907.5654.

### (3) 6-O-(Allyloxycarbonyl)-4-O-diallylphosphono-3-O-[(R)-3-methoxydecyl]-2-O-[(Z)-11-octadecenyl]-α,β-D-glucopyranoside (Step Ab3)

A reaction was performed in the same manner as in Example 1(2) using (E)-1-propenyl 6-O-(allyloxycarbonyl)-4-O-diallylphosphono-3-O-[(R)-3-methoxydecyl]-2-O-[(Z)-11-octadecenyl]-α,β-D-glucopyranoside (a 3:2 anomer mixture) obtained in (2) to give the title compound as an oil (yield 78%, an anomer mixture).
IR νₘₐₓ(film) 3338 (br), 2927, 2856, 1753, 1650 (w), 1460 cm⁻¹.
400 MHz ¹H NMR (CDCl₃) δ 0.88 (6H, t, J=6.7 Hz), 1.27 (32H, bs), 1.42-1.46 (2H, m), 1.59-1.63 (2H, m), 1.75-1.79 (2H, m), 1.99-2.02 (4H, m), 3.12-3.42 (6H, m, containing 3H, s, at 3.29 ppm), 3.60-3.69 (2H, m), 3.78-3.87 (2H, m), 4.16-4.53 (4H, m), 4.56-4.63 (6H, m), 5.23-5.39 (9H, m), 5.87-5.97 (3H, m).
FABMS (positive-ion) m/z; 845 [M+H]⁺, 867 [M+Na]⁺.
HRFABMS; Calcd. for C₄₅H₈₁O₁₂PNa: 867.5363. Found: 867.5362.

### (4) Trichloroacetimidoyl 6-O-(allyloxycarbonyl)-4-O-diallylphosphono-3-O-[(R)-3-methoxydecyl]-2-O-[(Z)-11-octadecenyl]-α,β-D-glucopyranoside (Step Ab4)

A reaction was performed in the same manner as in Example 1(3), using 6-O-(allyloxycarbonyl)-4-O-diallylphosphono-3-O-[(R)-3-methoxydecyl]-2-O-[(Z)-11-octadecenyl]-α,β-D-glucopyranoside obtained in (3) to give the title compound as an oil (yield 100%, a 1:1 anomer mixture).
IR νₘₐₓ(film) 3350-3080, 2928, 2856, 1752, 1675, 1651 (w), 1611 (w) cm⁻¹.
400 MHz ¹H NMR (CDCl₃) δ 0.88 (6H, t, J=6.7 Hz), 1.22-1.29 (32H, m), 1.43-1.53 (4H, m), 1.74-1.82 (2H, m), 1.99-2.04 (4H, m), 3.25 (1H, m), 3.30 (3H, s), 3.44-4.11 (7H, m), 4.33-4.51 (3H, m), 4.56-4.62 (6H, m), 5.23-5.40 (8H, m), 5.71 (0.5H, d, J=7.4 Hz, anomeric H), 5.86-5.99 (3H, m), 6.48 (0.5H, d, J=3.5 Hz, anomeric H), 8.61 (0.5H, s), 8.66 (0.5H, s).
FABMS (positive-ion) m/z; 827, 1010 [³⁵Cl, M+H]⁺, 1012 [M+H]⁺.

### (5) 4-O-(Allyloxycarbonyl)-3-O-decyl-2-deoxy-6-O-{6-O-(allyloxycarbonyl)-4-O-diallylphosphono-3-O-[(R)-3-methoxydecyl]-2-O-[(Z)-11-octadecenyl]-α-D-glucopyranosyl}-2-(3-oxotetradecanoylamino)-α-D-glucopyranoside (α-anomer) and 4-O-(allyloxycarbonyl)-3-O-decyl-2-deoxy-6-O-{6-O-(allyloxycarbonyl)-4-O-diallylphosphono-3-O-[(R)-3-methoxydecyl]-2-O-[(Z)-11-octadecenyl]-β-D-glucopyranosyl}-2-(3-oxotetradecanoylamino)-α-D-glucopyranoside (β-anomer) (Step Ca1)

A reaction was performed in the same manner as in Example 1(12) using trichloroacetimidoyl 6-O-(allyloxycarbonyl)-4-O-diallylphosphono-3-O-[(R)-3-methoxydecyl]-2-O-[(Z)-11-octadecenyl]-α,β-D-glucopyranoside obtained in (4) to give an α-anomer (543 mg, yield 53%) and a β-anomer (280 mg, yield 28%) of the title compound as an oil.
physical constant of α-anomer:
IR νₘₐₓ(film) 3380-3080, 2927, 2856, 1752, 1725, 1690-1672 cm⁻¹.
400 MHz ¹H NMR (CDCl₃) δ 0.86-0.90 (12H, m), 1.26 (62H, bs), 1.40-1.46 (4H, m), 1.53-1.80 (4H, m), 1.70-1.80 (2H, m), 1.98-2.05 (4H, m), 2.53 (2H, t, J=7.4 Hz), 3.21-3.93 (19H, m, containing 3H, s, at 3.28 ppm), 4.15-4.41 (4H, m, containing OH), 4.49-4.64 (10H, m), 4.87 (1H, d, J=3.1 Hz, anomeric H), 5.16 (1H, d, J=3.9 Hz, anomeric H), 5.21-5.40 (10H, m), 5.87-5.99 (4H, m), 7.15 (1H, d, J=9.4 Hz, NH). FABMS (positive-ion) m/z; 867, 1476 [M+Na]⁺.
physical constant of β-anomer:
IR νₘₐₓ(film) 3312 (w, br), 2926, 2855, 1754, 1650, 1547 cm⁻¹.
400 MHz ¹H NMR (CDCl₃) δ 0.88 (12H, t, J=6.6 Hz), 1.26 (62H, bs), 1.40-1.50 (4H, m), 1.52-1.60 (4H, m), 2.52 (2H, t, J=7.4 Hz), 3.19 (1H, t, J=8.0 Hz), 3.26 (1H, m), 3.29 (3H, s), 3.35 (1H, m), 3.37, 3.42 (2H, AB-q, J=14.3 Hz), 3.50-3.89 (11H, m), 4.20 (1H, dt, J=3.4, 10.0 Hz), 4.27-4.39 (2H, m, containing anomeric H), 4.36 (1H, d, J=7.4 Hz, anomeric H), 4.43 (1H, bs, OH), 4.56-4.66 (10H, m), 5.22-5.40 (11H, m), 5.88-5.98 (4H, m), 7.18 (1H, d, J=9.4 Hz, NH).
FABMS (positive-ion) m/z; 1476 [M+Na]⁺.
HRFABMS; Calcd. for C₇₉H₁₄₀NO₂₀PNa: 1476.9604. Found: 1476.9606.

### (6) Diallylphosphono 4-O-(allyloxycarbonyl)-3-O-decyl-2-deoxy-6-O-(6-O-(allyloxycarbonyl)-4-O-diallylphosphono-3-O-[(R)-3-methoxydecyl]-2-O-[(Z)-11-octadecenyl]-β-D-glucopyranosyl}-2-(3-oxotetradecanoylamino)-α-D-glucopyranoside (Step Ca2)

A reaction was performed in the same manner as in Example 1(13) using 4-O-(allyloxycarbonyl)-3-O-decyl-2-deoxy-6-O-{6-O-(allyloxycarbonyl)-4-O-diallylphosphono-3-O-[(R)-3-methoxydecyl]-2-O-[(Z)-11-octadecenyl]-β-D-glucopyranosyl}-2-(3-oxotetradecanoylamino)-α-D-glucopyranoside obtained in (5) to give the title compound as an oil (yield 61 %).
IR νₘₐₓ(film) 3294 (w), 3089 (w), 2926, 2856, 1754, 1678 (w), 1650 (w), 1550 (w) cm⁻¹.
400 MHz ¹H NMR (CDCl₃) δ 0.86-0.90 (12H, m), 1.26 (62H, bs), 1.42-1.46 (4H, m), 1.51-1.57 (4H, m), 1.74-1.80 (2H, m), 2.00-2.02 (4H, m), 2.48-2.51 (2H, m), 3.13 (1H, m), 3.23 (1H, m), 3.29 (3H, s), 3.35 (1H, t, J=9.0 Hz), 3.40 (2H, s), 3.50-4.29 (14H, m), 4.50-4.65 (14H, m), 4.84 (1H, t, J=9.5 Hz), 5.22-5.42 (14H, m), 5.72 (1H, dd, J=3.1, 6.3 Hz), 5.89-5.98 (6H, m), 7.42 (1H, d, J=9.0 Hz, NH).
FABMS (positive-ion) m/z; 1436, 1636 [M+Na]⁺.
HRFABMS; Calcd. for C₈₅H₁₄₉NO₂₃P₂Na: 1636.9894. Found: 1636.9932.

### (7) Phosphono 3-O-decyl-2-deoxy-6-O- {3-O-[(R)-3-methoxydecyl]-2-O-[(Z)-11-octadecenyl]-4-O-phosphono-β-D-glucopyranosyl}-2-(3-oxotetradecanoylamino)-α-D-glucopyranoside (Step Ca3)

A reaction was performed in the same manner as in Example 1(14) except that the reaction temperature was 35°C and diallylphosphono 4-O-(allyloxycarbonyl)-3-O-decyl-2-deoxy-6-O-{6-O-(allyloxycarbonyl)-4-O-diallylphosphono-3-O-[(R)-3-methoxydecyl]-2-O-[(Z)-11-octadecenyl]-β-D-glucopyranosyl}-2-(3-oxotetradecanoylamino)-α-D-glucopyranoside obtained in (6) was used to give the title compound in the form of wax (yield 66%).
IR νₘₐₓ(KBr) 3700-3000, 2925, 2854, 1717, 1652, 1543, 1466 cm⁻¹. 400 MHz ¹H NMR (CDCl₃+CD₃OD, 5:1) δ 0.88 (6H, t, J=6.6 Hz), 0.89 (6H, t, J=6.6 Hz), 1.22-1.34 (62H, m), 1.50-1.58 (8H, m), 1.78-1.84 (2H, m), 1.99-2.04 (4H, m), 2.55 (2H, t, J=7.2 Hz), 3.14 (1H, t, J=8.4 Hz), 3.31-3.38 (6H, m, containing 3H, s, at 3.34 ppm), 3.50 (1H, d, J=9.8 Hz), 3.60-4.15 (16H, m), 4.39 (1H, d, J=7.4 Hz, anomeric H), 5.31-5.39 (2H, m), 5.52 (1H, q, J=3.1 Hz, anomeric H).
FABMS (negative-ion) m/z; 1284 [M-H]⁻.
Anal. Calcd. for C₆₅H₁₂₅NO₁₉P_{2˙}2H₂O: C, 59.03; H, 9.83; N, 1.06; P, 4.68. Found: C, 59.10; H, 9.77; N, 1.07; P, 4.29.

### [Example 5]

2-(Phosphonooxy)ethyl 3-O-decyl-2-deoxy-6-O-{3-O-[(R)-3-methoxydecyl]-6-O-methyl-2-O-[(Z)-11-octadecenoyl]-4-O-phosphono-β-D-glucopyranosyl}-2-(3-oxotetradecanoylamino)-α-D-glucopyranoside (Compound No. 5)

### (1) Allyl 3-O-decyl-2-O-deoxy-4,6-O-isopropylidene-2-trifluoroacetylamino-α-D-glucopyranoside (Step Ba1)

Allyl 2-O-deoxy-4,6-O-isopropylidene-2-trifluoroacetylamino-α-D-glucopyranoside (10.60 g, 29.834 mmol) and decyl methanesulfonate (9.04 g, 38.245 mmol) were dissolved in DMF (40 mL). Sodium hydride (55% dispersion in oil, 3.00 g, 38.750 mmol) was added to the solution under ice cooling, and the mixture was stirred at 0°C for 30 minutes, and then at room temperature overnight. After completion of the reaction, methanol was added to the reaction solution under ice cooling to decompose excess sodium hydride. The reaction solution was diluted with ethyl acetate, washed with water and saturated brine, dried over magnesium sulfate, filtrated and concentrated in vacuo. The resulting residue was purified by silica gel chromatography (eluent: cyclohexane/ethyl acetate =6/1) to give the title compound as an oil (13.20 g, yield 89%).
IR νₘₐₓ(film) 3320, 2927, 2857, 1715, 1556-1543, 1466 cm⁻¹. 400 MHz ¹H NMR (CDCl₃) δ 0.88 (3H, t, J=6.6 Hz), 1.24 (14H, bs), 1.41 (3H, s), 1.42-1.50 (2H, m), 1.51 (3H, s), 3.40-3.51 (2H, m), 3.64-3.88 (5H, m), 3.99 (1H, m), 4.15-4.21 (2H, m), 4.88 (1H, d, J=3.5 Hz, anomeric H), 5.24-5.32 (2H, m), 5.87 (1H, m), 6.40 (1H, d, J=9.4 Hz, NH).
FABMS (positive-ion) m/z; 496 [M+H]⁺.

### (2) 2-Hydroxyethyl 2-amino-3-O-decyl-2-deoxy-4,6-O-isopropylidene-α-D-glucopyranoside (Step Bk1)

Allyl 3-O-decyl-2-O-deoxy-4,6-O-isopropylidene-2-trifluoroacetylamino-α-D-glucopyranoside obtained in (1) (13.20 g, 26.634 mmol) was dissolved in a mixed solvent of THF-water (3:1, 260 mL). Sodium periodate (NaIO₄, 43 g) and osmic acid (OsO₄, 4.5 mL, a 2.5% t-butanol solution) were added to the solution, and the mixture was stirred at room temperature for 1.5 hours. After completion of the reaction, the reaction solution was diluted with ethyl acetate, washed with a saturated sodium bicarbonate solution and saturated brine, dried over magnesium sulfate, filtrated and concentrated in vacuo to give crude aldehyde (16.47 g).

The obtained crude aldehyde was dissolved in 99.5% ethanol (300 mL). Thereto was added sodium borohydride (NaBH₄, 2.00 g), and the mixture was stirred at room temperature for 20 hours. After completion of the reaction, acetic acid was added to the reaction solution under ice cooling to decompose NaBH₄, and the solution was concentrated in vacuo. The concentrate was dissolved in ethyl acetate. The solution was washed with a saturated sodium bicarbonate solution and saturated brine, dried over magnesium sulfate, filtrated and concentrated in vacuo to give an alcohol that is a mixture of amide and amine (about 9.0 g).

The mixture was dissolved in ethanol (100 mL) and 1 mol/L aqueous sodium hydroxide solution (50 mL), and the reaction solution was refluxed for 1.5 hours. After completion of the reaction, the reaction solution was concentrated in vacuo and extracted with ethyl acetate. The organic layer was dried over magnesium sulfate, filtrated and concentrated in vacuo. The resulting residue was purified by NH-silica gel column chromatography (eluent: ethyl acetate/methanol =19/1) to give the title compound (8.10 g, yield 76%).
IR νₘₐₓ(film) 3700-3000, 2926, 2857, 1682, 1593 cm⁻¹.
400 MHz ¹H NMR (CDCl₃+D₂O) δ 0.88 (3H, t, J=6.6 Hz), 1.27 (14H, bs), 1.40 (3H, s), 1.49 (3H, s), 1.52-1.56 (2H, m), 2.86 (1H, dd, J=3.4, 9.8 Hz, C2-H), 3.40 (1H, t, J=9.3 Hz), 3.53-3.87 (10H, m), 4.94 (1H, d, J=3.4 Hz, anomeric H).
FABMS (positive-ion) m/z; 404 [M+H]⁺, 426 [M+Na]⁺.
HRFABMS; Calcd. for C₂₁H₄₂NO₆: 404.3012. Found: 404.3011.

### (3) 2-(Diallylphosphonooxy)ethyl 3-O-decyl-2-deoxy-4,6-O-isopropylidene-2-(3-oxotetradecanoylamino)-α-D-glucopyranoside (Step Bk2)

2-Hydroxyethyl 2-amino-3-O-decyl-2-deoxy-4,6-O-isopropylidene-α-D-glucopyranoside obtained in (2) (2.02 g, 5.000 mmol) was dissolved in methylene chloride (20 mL). To the solution were added 1H-tetrazole (1.56 g, 22.27 mmol) and diallyl diisopropylphosphoramidite (1.69 g, 6.889 mmol), and the mixture was stirred at room temperature for 0.5 hour. Thereafter, a mixed solution of OXONE® (potassium peroxymonosulfate) (4.55 g, 7.401 mmol) in THF-water (2:1, 37.5 mL) was added thereto, and the mixture was stirred at room temperature for 15 minutes. After completion of the reaction, the reaction solution was diluted with methylene chloride, washed with saturated aqueous sodium bicarbonate solution and brine, dried over magnesium sulfate, filtrated and concentrated in vacuo to give the crude phosphate.

The obtained crude phosphate was dissolved in methylene chloride (150 mL). To the solution were added 3-oxotetradecanoic acid (1.46 g, 6.000 mmol), 1-[3-(dimethylamino)propyl]-3-ethylcarbodiimide hydrochloride (1.44 g, 7.511 mmol) and DMAP (0.92 g, 7.530 mmol), and the mixture was stirred at room temperature for 30 minutes. After completion of the reaction, the reaction solution was diluted with methylene chloride, washed with water and brine, dried over magnesium sulfate, filtrated and concentrated in vacuo. The resulting residue was purified by silica gel chromatography (eluent: ethyl acetate/cyclohexane=3/1) to give the title compound (1.96 g, yield in 2 steps: 50%).
IR νₘₐₓ(film) 3290, 3085 (w), 2926, 2856, 1820, 1673, 1658 (shoulder), 1550, 1464 cm⁻¹.
400 MHz ¹H NMR (CDCl₃) δ 0.88 (3H, t, J=6.6 Hz), 1.25 (30H, bs), 1.40 (3H, s), 1.42-1.48 (2H, m), 1.49 (3H, s), 1.53-1.58 (2H, m), 2.52-2.56 (2H, m), 3.43-3.50 (4H, m, containing 2H, s, at 3.43), 3.60-3.88 (7H, m), 4.19-4.26 (3H, m), 4.52-4.61 (4H, m), 4.78 (1H, d, J=3.5 Hz, anomeric H), 5.26-5.44 (4H, m), 5.90-6.02 (2H, m). 7.62 (1H, d, J=9.4 Hz, NH).
FABMS (positive-ion) m/z; 788 [M+H]⁺, 810 [M+Na]⁺.
HRFABMS; Calcd. for C₄₁H₇₄NO₁₁PNa: 810.4897. Found: 810.4916.

### (4) 2-(Diallylphosphonooxy)ethyl 3-O-decyl-2-deoxy-2-(3-oxotetradecanoylamino)-α-D-glucopyranoside (Step Bk3)

2-(Diallylphosphonooxy)ethyl 3-O-decyl-2-deoxy-4,6-O-isopropylidene-2-(3-oxotetradecanoylamino)-α-D-glucopyranoside obtained in (3) (1.06 g, 1.345 mmol) was dissolved in an 80% aqueous acetic acid solution (100 mL). The solution was stirred at 85°C for 1.5 hours. After completion of the reaction, the solution was concentrated in vacuo. The resulting residue was purified by silica gel column chromatography (eluent: ethyl acetate, then ethyl acetate/ methanol=19/1) to give the title compound (550 mg, yield 55%).
IR νₘₐₓ(KBr) 3427, 3298, 3081 (w), 2921, 2851, 1708, 1639, 1549 cm⁻¹. 400 MHz ¹H NMR (CDCl₃) δ 0.88 (6H, t, J=6.8 Hz), 1.25 (28H, bs), 1.50-1.70 (8H, m, containing OHx2), 2.54 (2H, t, J=7.4 Hz), 3.43-3.92 (11H, m, containing 2H, s at 3.45 ppm), 4.19-4.27 (3H, m), 4.52-4.59 (4H, m), 4.78 (1H, d, J=3.5 Hz, anomeric H), 5.62-5.42 (4H, m), 5.89-5.99 (2H, m), 7.71 (1H, d, J=9.4 Hz, NH).
FABMS (positive-ion) m/z; 748 [M+H]⁺,770 [M+Na]⁺.
HRFABMS; Calcd. for C₃₈H₇₁NO₁₁P: 748.4765. Found: 748.4776.

### (5) 2-(Diallylphosphonooxy)ethyl 3-O-decyl-2-deoxy-6-O-{4-O-diallylphosphono-3-O-[(R)-3-methoxydecyl]-6-O-methyl-2-O-[(Z)-11-octadecenoyl]-β-D-glucopyranosyl}-2-(3-oxotetradecanoyl)amino-α-D-glucopyranoside (Step Cc1)

Trichloroacetimidoyl 4-O-diallylphosphono-3-O-[(R)-3-methoxydecyl]-6-O-methyl-2-O-[(Z)-11-octadecenoyl]-α-D-glucopyranoside obtained in Example 1(3) (250 mg, 0.268 mmol) and 2-(diallylphosphonooxy)ethyl 3-O-decyl-2-deoxy-2-(3-oxotetradecanoylamino)-α-D-glucopyranoside obtained in (4) (180 mg, 0.241 mmol) were dissolved in methylene chloride (10 mL). Molecular sieves 4A (0.5 g) were added to the solution, and the mixture was stirred for 30 minutes. Thereto were then added AgOTf (180 mg, 0.701 mmol) and TMSOTf(16 mg, 0.072 mmol) in a nitrogen atmosphere, and the mixture was stirred at room temperature overnight. After completion of the reaction, the reaction solution was diluted with methylene chloride, washed with sodium bicarbonate solution, dried over magnesium sulfate, filtrated and concentrated in vacuo. The resulting residue was purified by silica gel column chromatography (eluent: cyclohexane/ethyl acetate=1/2) to give the title compound (165 mg, yield 45%).
IR νₘₐₓ(film) 3302, 2925, 2854, 1747, 1720, 1636, 1549, 1466 cm⁻¹.
400 MHz ¹H NMR (CDCl₃ + D₂O) δ 0.86-0.89 (12H, m), 1.25 (60H, bs), 1.40-1.72 (10H, m), 1.99-2.02 (4H, m), 2.31-2.35 (2H, m), 2.54 (2H, t, J=7.0-8.2 Hz), 3.21 (1H, m), 3.25 (3H, s), 3.39 (3H, s), 3.43 (2H, s), 3.45-3.78 (12H, m), 3.85 (1H, m), 3.98 (1H, dd, J=2.3, 11.0 Hz), 4.15-4.23 (3H, m), 4.35 (1H, q, J=9.4 Hz), 4.49-4.60 (10H, m), 4.72 (1H, d, J=3.9 Hz), 4.97 (1H, dd, J=8.2, 9.4 Hz), 5.24-5.43 (10H, m), 5.89-6.00 (4H, m), 7.67 (1H, d, J=9.4 Hz).
FABMS (positive-ion) m/z; 1518 [M+H]⁺, 1540 [M+Na]⁺.

### (6) 2-(Phosphonooxy)ethyl 3-O-decyl-2-deoxy-6-O-{3-O-[(R)-3-methoxydecyl]-6-O-methyl-2-O-[(Z)-11-octadecenoyl]-4-O-phosphono-β-D-glucopyranosyl}-2-(3-oxotetradecanoylamino)-α-D-glucopyranoside (Step Cc2)

2-(Diallylphosphonooxy) ethyl 3-O-decyl-2-deoxy-6-O-{4-O-diallylphosphono-3-O-[(R)-3-methoxydecyl]-6-O-methyl-2-O-[(Z)-11-octadecenoyl]-β-D-glucopyranosyl}-2-(3-oxotetradecanoyl)amino-α-D-glucopyranoside obtained in (5) (160 mg, 0.105 mmol) was dissolved in dry THF (8 mL). To the solution were added triphenylphosphine (18 mg, 0.069 mmol), triethylamine (70 mg, 0.692 mmol), formic acid (59 mg, 1.282 mmol) and Pd(PPh₃)₄ (18 mg, 0.016 mmol), and the mixture was stirred at 55°C for 16 hours in a nitrogen atmosphere. After completion of the reaction, the reaction solution was filtrated and concentrated in vacuo. The resulting residue was purified on a column packed with 7 g of DEAE-cellulose (eluent: a 0.05 mol/L ammonium acetate solution of chloroform-methanol-water (2:3:1)) to give a fraction of the target substance. The obtained fraction was collected, and chloroform and a 0.15 mol/L hydrochloric acid solution were added thereto so that the entire mixture contained chloroform-methanol-water (1:1:1) (pH being adjusted to 2-3). The mixture was stirred in a separatory funnel, and the chloroform layer at the bottom was collected and concentrated in vacuo to give the title compound in the form of wax (118 mg, yield 82%). 400 MHz ¹H NMR (CDCl₃+CD₃OD, 5:1) δ 0.86-0.89 (12H, m), 1.20-1.35 (60H, m), 1.38-1.62 (8H, m), 1.71-1.73 (2H, m), 2.00-2.04 (4H, m), 2.31-2.34 (2H, m), 2.55-2.58 (2H, m), 3.29 (3H, s), 3.32-4.18 (25H, m, containing 3H, s, at 3.40 ppm), 4.49 (1H, d, J=7.7 Hz, anomeric H), 4.72 (1H, d, J=3.5 Hz, anomeric H), 4.88 (1H, dd, J=8.2, 9.0 Hz), 5.32-5.35 (2H, m).
FABMS (negative-ion) m/z; 1356 [M-H]⁻.
Anal. Calcd. for C₆₈H₁₂₈NO₂₁P_{2˙}1.4 H₂O: C, 59.02; H, 9.60; N, 1.01; P, 4.48. Found: C, 59.10; H, 9.51; N, 1.09; P, 4.37.

### [Example 6]

2-(Phosphonooxy)ethyl 3-O-decyl-2-deoxy-6-O-{3-O-[(R)-3-methoxydecyl]-2-O-[(Z)-11-octadecenoyl]-4-O-phosphono-β-D-glucopyranosyl}-2-(3-oxotetradecanoylamino)-α-D-glucopyranoside (Compound No. 6)

### (1) 2-(Diallylphosphonooxy)ethyl 3-O-decyl-2-deoxy-6-O-{6-O-allyloxycarbonyl-4-O-diallylphosphono-3-O-[(R)-3-methoxydecyl]-2-O-[(Z)-11-octadecenoyl]-β-D-glucopyranosyl}-2-(3-oxotetradecanoylamino)-α-D-glucopyranoside (Step Cc1)

2,2,2-Trichloroacetimidoyl 6-O-allyloxycarbonyl-4-O-diallylphosphono-3-O-[(R)-3-methoxydecyl]-2-O-[(Z)-11-octadecenoyl]-α,β-D-glucopyranoside (223 mg, 0.222 mmol) and 2-(diallylphosphonooxy)ethyl 3-O-decyl-2-deoxy-2-(3-oxotetradecanoylamino)-α-D-glucopyranoside obtained in Example 5(4) (220 mg, 0.294 mmol) were dissolved in methylene chloride (8 mL). Molecular sieves 4A (0.5 g) were added to the solution, and the mixture was stirred for 30 minutes. AgOTf (180 mg, 0.701 mmol) and TMSOTf(16 mg, 0.072 mmol) were then added thereto in a nitrogen atmosphere and the mixture was stirred at room temperature overnight. After completion of the reaction, the reaction solution was diluted with methylene chloride, washed with sodium bicarbonate solution, dried over magnesium sulfate, filtrated and concentrated in vacuo. The resulting residue was purified by silica gel column chromatography (eluent: cyclohexane/ethyl acetate=1/2) to give the title compound (135 mg, yield 38%).
IR νₘₐₓ(film) 3400-3200, 2926, 2855, 1751, 1669, 1651, 1547, 1461 cm⁻¹.
400 MHz ¹H NMR (CDCl₃ + D₂O) δ 0.86-0.89 (12H, m), 1.25 (60H, bs), 1.40-1.78 (10H, m), 2.00-2.02 (4H, m), 2.30-2.34 (2H, m), 2.51-2.55 (2H, m), 3.21 (1H, m), 3.25 (3H, s), 3.43 (2H, s), 3.53-3.74 (12H, m), 3.85 (1H, m), 4.07 (1H, d, J=10.2 Hz), 4.15-4.40 (4H, m), 4.45 (1H, d, J=7.8 Hz), 4.50-4.65 (11H, m), 4.70 (1H, d, J=3.5 Hz), 4.95 (1H, m), 5.25-5.42 (12H, m), 5.90-5.97 (5H, m), 7.72 (1H, d, J=9.8 Hz). FABMS (positive-ion) m/z; 1588 [M+H]⁺, 1610 [M+Na]⁺.
HRFABMS; Calcd. for C₈₃H₁₄₇NO₂₃P₂:Na: 1610.9736. Found: 1610.9709.

### (2) 2-(Phosphonooxy)ethyl 3-O-decyl-2-deoxy-6-O-{3-O-[(R)-3-methoxydecyl]-2-O-[(Z)-11-octadecenoyl]-4-O-phosphono-β-D-glucopyranosyl}-2-(3-oxotetradecanoylamino)-α-D-glucopyranoside (Step Ce1)

A reaction was performed in the same manner as in Example 5(6) using 2-(diallylphosphonooxy)ethyl 3-O-decyl-2-deoxy-6-O-{6-O-allyloxycarbonyl-4-O-diallylphosphono-3-O-[(R)-3-methoxydecyl]-2-O-[(Z)-11-octadecenoyl]-β-D-glucopyranosyl}-2-(3-oxotetradecanoylamino)-α-D-glucopyranoside obtained in (1) (203 mg, 0.128 mmol) to give the title compound in the form of wax (95 mg, yield 55%).
IR νₘₐₓ(KBr) 3600-3200, 2924, 2853, 1723, 1650, 1547, 1467 cm⁻¹.
400 MHz ¹H NMR (CDCl₃+CD₃OD, 4:1) δ 0.86-0.90 (12H, m), 1.26 (60H, bs), 1.40-1.62 (8H, m), 1.71-1.73 (2H, m), 2.00-2.04 (4H, m), 2.32-2.36 (2H, m), 2.56-2.58 (2H, m), 3.30 (3H, s), 3.31-4.24 (22H, m), 4.48 (1H, d, J=8.2 Hz, anomeric H), 4.73 (1H, d, J=3.4 Hz, anomeric H), 4.87 (1H, t, J=8.2 Hz), 5.33-5.37 (2H, m). FABMS (negative-ion) m/z; 1342 [M-H]⁻.
Anal. Calcd. for C₆₇H₁₂₇NO₂₁P_{2˙}1.4 H₂O: C, 59.85; H, 9.52; N, 1.04; P, 4.61. Found: C, 59.96; H, 9.32; N, 1.19; P, 4.47.

### [Example 7]

2-(Phosphonooxy)ethyl 6-O-{3-O-[(R)-3-methoxydecyl]-6-O-methyl-2-O-[(Z)-11-octadecenoyl]-4-O-phosphono-β-D-glucopyranosyl}-2,3-di-O-dodecyl-α-D-glucopyranoside (Compound No. 7)

### (1) Allyl 2,3-di-O-dodecyl-4,6-O-isopropylidene-α-D-glucopyranoside

Allyl 4,6-O-isopropylidene-α-D-glucopyranoside (2.90 g, 11.14 mmol) was dissolved in DMF (29 mL). Dodecyl iodide (8.00 g, 27.00 mmol) and sodium hydride (55% dispersion in oil, 2.36 g, 54.00 mmol) were added to the solution under ice cooling, and the mixture was stirred at room temperature overnight. After completion of the reaction, the reaction solution was diluted with ethyl acetate, washed with ice water and saturated brine, dried over magnesium sulfate, filtrated and concentrated in vacuo. The resulting residue was purified by silica gel column chromatography (eluent: cyclohexane/ethyl acetate=9/1) to give the title compound (6.49 g, yield 98%).
400 MHz ¹H NMR (CDCl₃) δ 0.88 (6H, t, J=6.6 Hz), 1.26 (36H, bs), 1.41 (3H, s), 1.48 (3H, s), 1.50-1.60 (4H, m), 3.30 (1H, m), 3.49-3.74 (8H, m), 3.83 (1H, m), 4.08 (1H, dd, J=6.6, 13.3 Hz), 4.18 (1H, dd, J=5.1, 13.3 Hz), 4.90 (1H, d, J=3.7 Hz), 5.22 (1H, m), 5.32 (1H, m), 5.92 (1H, m).

### (2) 2-(Hydroxy)ethyl 2,3-di-O-dodecyl-4,6-O-isopropylidene-α-D-glucopyranoside (Step Bc1)

Allyl 2,3-di-O-dodecyl-4,6-O-isopropylidene-α-D-glucopyranoside obtained in (1) (2.39 g, 4.00 mmol) and NaIO₄ (4.00 g) were dissolved in an ethanol (30 mL)-water (10 mL) mixed solution. OsO₄ (a 2.5% t-butanol solution, 80 mg) was added to the solution, and the mixture was stirred at room temperature for 2 hours. After completion of the reaction, the reaction solution was diluted with ethyl acetate, washed with sodium bicarbonate solution and brine, dried over magnesium sulfate, filtrated and concentrated in vacuo to give crude aldehyde. The obtained crude aldehyde was dissolved in 99% ethanol (50 mL). NaBH₄ (200 mg) was added to the solution, and the mixture was stirred at room temperature for 10 minutes. After completion of the reaction, acetic acid was added to the reaction solution to remove excess reducing agent. The reaction solution was diluted with acetic acid, washed with sodium bicarbonate solution and brine, dried over magnesium sulfate, filtrated and concentrated in vacuo. The resulting residue was purified by silica gel column chromatography (eluent: cyclohexane/ethyl acetate=2/1) to give the title compound (1.66 g, yield 69%).
400 MHz ¹H NMR (CDCl₃) δ 0.88 (9H, t, J=6.6 Hz), 1.26 (36H, bs), 1.40 (3H, s), 1.41-1.43 (4H, m), 1.48 (3H, s), 2.82 (1H, bs, OH), 3.30 (1H, m), 3.51-3.85 (14H, m), 4.89 (1H, d, J=3.7 Hz).

### (3) 2-(Diallylphosphonooxy)ethyl 2,3-di-O-dodecyl-4,6-O-isopropylidene-α-D-glucopyranoside (Step Bc2)

2-(Hydroxy)ethyl 2,3-di-O-dodecyl-4,6-O-isopropylidene-α-D-glucopyranoside (1.47 g, 2.45 mmol) obtained in (2) was dissolved in methylene chloride (30 mL). To the solution were added 1H-tetrazole (400 mg, 5.71 mmol) and diallyl diisopropylphosphoramidite (1.20 g, 4.89 mmol), and the mixture was stirred at room temperature for 20 minutes. Subsequently, THF (30 mL) was added to the reaction solution, and a 30% hydrogen peroxide solution (0.5 g) was then added thereto. The mixture was stirred for 20 minutes. After completion of the reaction, the reaction solution was diluted with ethyl acetate, washed with a 10% aqueous sodium thiosulfate solution and saturated brine, dried over magnesium sulfate, filtrated and concentrated in vacuo. The resulting residue was purified by silica gel column chromatography (eluent: cyclohexane/ethyl acetate=2/1) to give the title compound (1.62 g, yield 87%).
400 MHz ¹H NMR (CDCl₃) δ 0.88 (6H, t, J=6.6 Hz), 1.26 (36H, bs), 1.39 (3H, s), 1.47 (3H, s), 1.50-1.59 (4H, m), 3.29 (1H, m), 3.50-3.88 (11H, m), 4.23-4.27 (2H, m), 4.56-4.59 (4H, m), 4.90 (1H, d, J=3.7 Hz), 5.25-5.42 (4H, m), 5.90-6.00 (2H, m). FABMS (positive-ion) m/z; 761 [M+H]⁺, 783 [M+Na]⁺.

### (4) 2-(Diallylphosphonooxy)ethyl 2,3-di-O-dodecyl-α-D-glucopyranoside (Step Bc3)

2-(Diallylphosphonooxy)ethyl 2,3-di-O-dodecyl-4,6-O-isopropylidene-α-D-glucopyranoside obtained in (3) (6.60 g, 9.10 mmol) was dissolved in methanol (60 mL). To the solution was added p-toluenesulfonic acid/monohydrate (412 mg), and the mixture was stirred at room temperature for 2 hours and concentrated in vacuo. The resulting residue was purified by silica gel column chromatography (eluent: ethyl acetate, then a 10% methanol-ethyl acetate solution) to give the title compound in the form of wax (6.00 g, yield 96%).
400 MHz ¹H NMR (CDCl₃) δ 0.88 (6H, t, J=6.6 Hz), 1.26 (36H, bs), 1.50-1.71 (4H, m), 3.27 (1H, dd, J=3.7, 8.8 Hz), 3.41-3.91 (11H, m), 4.24-4.28 (2H, m), 4.48-4.59 (4H, m), 4.95 (1H, d, J=3.7 Hz), 5.19-5.40 (4H, m), 5.90-6.00 (2H, m).

### (5) 2-(Diallylphosphonooxy)ethyl 6-O-{4-O-diallylphosphono-3-O-[(R)-3-methoxydecyl]-6-O-methyl-2-O-[(Z)-11-octadecenoyl]-β-D-glucopyranosyl}-2,3-di-O-dodecyl-α-D-glucopyranoside (Step Cc1)

Trichloroacetimidoyl 4-O-diallylphosphono-3-O-[(R)-3-methoxydecyl]-6-O-methyl-2-O-[(Z)-11-octadecenoyl]-α-D-glucopyranoside obtained in Example 1(3) (260 mg, 0.279 mmol) and 2-(diallylphosphonooxy)ethyl 2,3-di-O-dodecyl-α-D-glucopyranoside obtained in (4) (197 mg, 0.279 mmol) were dissolved in methylene chloride (10 mL). Molecular sieves 4A (500 mg) were added to the solution, and the mixture was stirred for 30 minutes in a nitrogen atmosphere. Subsequently, AgOTf (180 mg, 0.701 mmol) and TMSOTf(20 mg, 0.090 mmol) were added to the reaction solution, and the mixture was stirred at room temperature for 16 hours in a nitrogen atmosphere. After completion of the reaction, the reaction solution was diluted with methylene chloride, washed with sodium bicarbonate solution and saturated brine, dried over magnesium sulfate, filtrated and concentrated in vacuo. The resulting residue was purified by silica gel chromatography (eluent: cyclohexane/ethyl acetate =1/2) to give the title compound in the form of gum (215 mg, yield 52%).
IR νₘₐₓ(film) 3412, 2926, 2855, 1750, 1652 (w), 1465 cm⁻¹.
400 MHz ¹H NMR (CDCl₃) δ 0.88 (12H, t, J=6.6 Hz), 1.10-1.43 (70H, m), 1.55-1.70 (6H, m), 1.99-2.02 (4H, m), 2.29-2.36 (2H, m), 2.82 (1H, bs, OH), 3.22 (1H, m),
3.25 (3H, s), 3.39 (3H, s), 3.47-3.84 (18H, m), 3.96 (1H, dd, J=1.0, 8.6 Hz), 4.19-4.25 (2H, m), 4.32 (1H, q, J=9.4 Hz), 4.47 (1H, d, J=7.8 Hz), 4.54-4.59 (8H, m), 4.89 (1H, d, J=3.5 Hz), 4.97 (1H, d, J=8.6 Hz), 5.24-5.40 (10H, m), 5.89-5.99 (4H, m). FABMS (positive-ion) m/z; 1513 [M+Na]⁺.
HRFABMS; Calcd. for C₈₀H₁₄₈O₂₀P₂Na: 1513.9937. Found: 1513.9934.

### (6) 2-(Phosphonooxy)ethyl 6-O-{3-O-[(R)-3-methoxydecyl]-6-O-methyl-2-O-[(Z)-11-octadecenoyl]-4-O- phosphono-β-D-glucopyranosyl}-2,3-di-O-dodecyl-α-D-glucopyranoside (Step Cc2)

2-(Diallylphosphonooxy)ethyl 6-O-{4-O-diallylphosphono-3-O-[(R)-3-methoxydecyl]-6-O-methyl-2-O-[(Z)-11-octadecenoyl]-β-D-glucopyranosyl}-2,3-di-O-dodecyl-α-D-glucopyranoside (140 mg, 0.094 mmol) obtained in (5) was dissolved in dry THF (8 mL). To the solution were added triphenylphosphine (16 mg, 0.061 mmol), triethylamine (65 mg, 0.642 mmol), formic acid (54 mg, 0.843 mmol) and Pd(PPh₃)₄ (16 mg, 0.014 mmol), and the mixture was stirred at 55°C for 16 hours in a nitrogen atmosphere. After completion of the reaction, the reaction solution was filtrated and concentrated in vacuo. The resulting residue was purified on a column packed with 6 g of DEAE-cellulose (eluent: 0.05 mol/L ammonium acetate solution of chloroform-methanol-water (2:3:1)) to give a fraction of the target substance. The obtained fraction was collected, and chloroform and a 0.15 mol/L hydrochloric acid solution were added thereto so that the entire mixture contained chloroform-methanol-water (1:1:1) (pH being adjusted to 2-3). The mixture was stirred in a separatory funnel, and the chloroform layer at the bottom was collected and concentrated in vacuo to give the title compound in the form of wax (81 mg, yield 65%).
IR νₘₐₓ(KBr) 3500-2500 (br), 2924, 2853, 1726, 1467 cm⁻¹.
400 MHz ¹H NMR (CDCl₃+CD₃OD, 5:1) δ 0.86-0.90 (12H, m), 1.26 (66H, bs), 1.40-1.44 (2H, m), 1.54-1.62 (6H, m), 1.71-1.75 (2H, m), 2.00-2.04 (4H, m), 2.32-2.38 (2H, m), 3.24-3.39 (6H, m, containing 3H, s, at 3.30 ppm), 3.41 (3H, s), 3.47-3.82 (15H, m), 4.02 (1H, d, J=10.2 Hz), 4.16-4.21 (3H, m), 4.49 (1H, d, J=7.8 Hz, anomeric H), 4.87 (1H, d, J=3.4 Hz, anomeric H), 4.91 (1H, t, J=8.4 Hz), 5.33-5.37 (2H, m).
FABMS (negative-ion) m/z; 1329 [M-H]⁻, 1351 [M-2H+Na]⁻.
Anal. Calcd. for C₆₈H₁₃₂O₂₀P₂: C, 61.33; H, 9.99; P, 4.65. Found: C, 61.39; H, 9.70; P, 4.58.

### [Example 8]

Phosphono 3-O-decyl-6-O-{3-O-[(R)-3-methoxydecyl]-6-O-methyl-2-O-[(Z)-11-octadecenoyl]-4-O-phosphono-β-D-glucopyranosyl}-α-D-glucopyranoside (Compound No. 8)

### (1) 3-O-Decyl-1,2:5,6-di-O-isopropylidene-α-D-glucofuranose

1,2:5,6-Di-O-isopropylidene-α-D-glucofuranose (17.00 g, 65.309 mmol) and decyl p-toluenesulfonate (15.34 g, 64.898 mmol) were dissolved in DMF (65 mL). Sodium hydride (55% dispersion in oil, 3.40 g, 77.916 mmol) was added to the solution under ice cooling, and the mixture was stirred at 0°C for 15 minutes and further at room temperature overnight. After completion of the reaction, methanol was added to the reaction solution under ice cooling to decompose the remaining sodium hydride. The reaction solution was diluted with ethyl acetate, washed with water and saturated brine, dried over magnesium sulfate, filtrated and concentrated in vacuo. The resulting residue was purified by silica gel chromatography (eluent: cyclohexane/ethyl acetate =6/1) to give the title compound as an oil (22.50 g, yield 98%).

### (2) Allyl 3-O-decyl-4,6-O-isopropylidene-α,β-D-glucopyranoside

3-O-decyl-1,2:5,6-di-O-isopropylidene-α-D-glucofuranose obtained in (1) (20.60 g, 51.429 mmol) was dissolved in allyl alcohol (300 mL) containing 2% of hydrochloric acid, and the mixture was refluxed for 30 minutes. The reaction solution was concentrated in vacuo, and the resulting concentrate was dissolved in DMF (30 mL) and 2,2-dimethoxypropane (30 mL). To the solution was added p-toluenesulfonic acid/monohydrate (500 mg), and the mixture was stirred at room temperature for 16 hours. After completion of the reaction, the reaction solution was diluted with ethyl acetate, washed with sodium bicarbonate solution and saturated brine, dried over magnesium sulfate, filtrated and concentrated in vacuo. The resulting residue was purified by silica gel chromatography (eluent: cyclohexane/ethyl acetate =3/1) to give an anomer mixture of the title compound as an oil (mixing ratio of α-anomer to β-anomer=3:1, 16.48 g, yield 80%). Part of the mixture could be separated into an α-anomer and a β-anomer using chromatography. physical constant of α-anomer:
IR νₘₐₓ(film) 3470 (br), 2994, 2925, 2856 cm⁻¹.
400 MHz ¹H NMR (CDCl₃) δ 0.88 (3H, t, J=6.6 Hz), 1.26 (14H, bs), 1.41 (3H, s), 1.49 (3H, s), 1.53-1.60 (2H, m), 2.27 (1H, d, J=7.3 Hz, OH), 3.46-3.86 (8H, m), 4.04 (1H, m), 4.21 (1H, m), 4.93 (1H, d, J=3.7 Hz), 5.22-5.35 (2H, m), 5.93 (1H, m).
Anal. Calcd. For C₂₂H₄₀O₆: C, 65.97; H, 10.07. Found: C, 65.49; H, 9.79. physical constant of β-anomer:
400 MHz ¹H NMR (CDCl₃) δ 0.88 (3H, t, J=6.6 Hz), 1.26 (14H, bs), 1.41 (3H, s), 1.49 (3H, s), 1.55-1.60 (2H, m), 2.29 (1H, d, J=2.2 Hz, OH), 3.25 (1H, m), 3.31 (1H, t, J=8.8 Hz), 3.45 (1H, m), 3.59-3.67 (2H, m), 3.75-3.82 (2H, m), 3.91 (1H, dd, J=5.1, 11.0 Hz), 4.14 (1H, dd, J=6.2, 12.8 Hz), 4.35 (1H, dd, J=5.1, 12.5 Hz), 4.40 (1H, d, J=8.1 Hz), 5.20-5.35 (2H, m), 5.93 (1H, m).
Anal. Calcd. For C₂₂H₄₀O₆: C, 65.97; H, 10.07. Found: C, 65.49; H, 9.79.

### (3) Allyl 3-O-decyl-4,6-O-isopropylidene-2-O-(3-oxotetradecanoyl)-α,β-D-glucopyranoside (Step Bj 1)

Allyl 3-O-decyl-4,6-O-isopropylidene-α,β-D-glucopyranoside obtained in (2) (a 3:1 anomer mixture, 300 mg, 0.750 mmol) was dissolved in methylene chloride-THF (2:1, 6 mL). To the solution were added 3-oxotetradecanoic acid (242 mg, 1.00 mmol) and WSCI hydrochloride (192 mg, 1.000 mmol), and the mixture was stirred at room temperature for 1 hour. After completion of the reaction, the reaction solution was diluted with methylene chloride, washed with water and sodium bicarbonate solution, dried over magnesium sulfate, filtrated and concentrated in vacuo. The resulting residue was purified by silica gel chromatography (eluent: cyclohexane/ethyl acetate =6/1) to give an anomer mixture of the title compound (mixing ratio of α-anomer to β-anomer=3:1, 375 mg, yield 80%). A β-anomer and an α-anomer could be separated from part of the mixture using chromatography.
physical constant of β-anomer:
IR νₘₐₓ(film) 2923, 2853, 1752, 1722, 1636, 1568 cm⁻¹.
400 MHz ¹H NMR (CDCl₃) δ 0.88 (3H, t, J=6.6 Hz), 1.26 (30H, bs), 1.40 (3H, s), 1.41-1.68 (7H, m, containing 3H, s, at 1.49 ppm), 2.56 (2H, t, J=6.3 Hz), 3.23 (1H, m), 3.38-3.56 (3H, m), 3.92 (1H, m), 4.05 (1H, m), 4.30 (1H, m), 4.47 (1H, d, J=8.1 Hz), 4.95 (1H, m), 5.15-5.27 (2H, m), 5.84 (1H, m).
FABMS (positive-ion) m/z; 623 [M-H]⁺, 647 [M+Na]⁺.
physical constant of α-anomer:
IR νₘₐₓ(film) 2926, 2856, 1751, 1720, 1649(w), 1624(w), 1466 cm⁻¹.
400 MHz ¹H NMR (CDCl₃) δ 0.88 (6H, t, J=6.6 Hz), 1.26 (30H, bs), 1.40-1.60 (10H, m, containing two 3H, s, at 1.41 and 1.50), 2.54 (2H, dt, J=2.2, 7.3 Hz), 3.41-3.60 (3H, m), 3.61-3.79 (5H, m), 3.84 (1H, m), 4.00 (1H, m), 4.17 (1H, m), 4.80 (1H, dd, J=4.4, 9.5 Hz, C2-H), 5.04 (1H, d, J=4.4 Hz, C1-H), 5.18-5.32 (2H, m), 5.89 (1H, m). FABMS (positive-ion) m/z; 623 [M-H]⁺, 625 [M+H]⁺, 647 [M+Na]⁺.
HRFABMS; Calcd. for C₃₆H₆₄O₈Na: 647.4499. Found: 647.4490.

### (4) Allyl 3-O-decyl-2-O-(3-oxotetradecanoyl)-α,β-D-glucopyranoside (Step Bj2)

Allyl 3-O-decyl-4,6-O-isopropylidene-2-O-(3-oxotetradecanoyl)-α,β-D-glucopyranoside obtained in (3) (a 3:1 anomer mixture, 2.37 g, 3.793 mmol) was dissolved in methanol (35 mL). To the solution were added p-toluenesulfonic acid (200 mg), and the mixture was stirred at room temperature for 2 hours. After completion of the reaction, the reaction solution was concentrated in vacuo, and the resulting residue was purified by silica gel column chromatography (eluent: cyclohexane/ethyl acetate=3/2) to give an anomer mixture of the title compound (mixing ratio of α-anomer to β-anomer =3:1, 1.31 g, yield 59%). Part of the mixture could be separated into an α-anomer and a β-anomer using chromatography. physical constant of α-anomer:
400 MHz ¹H NMR (CDCl₃) δ 0.88 (3H, t, J=6.6 Hz), 1.26 (30H, bs), 1.51-1.60 (4H, m), 2.05 (2H, m, OH), 2.53 (2H, dt, J=1.5, 6.6 Hz), 3.48 (2H, s), 3.59-3.87 (7H, m), 4.01 (1H, m), 4.18 (1H, m), 4.74 (1H, dd, J=3.7, 10.3 Hz), 5.08 (1H, d, J=3.7 Hz, anomeric H), 5.19-5.32 (2H, m), 5.89 (1H, m).
physical constant of β-anomer:
IR νₘₐₓ(film) 3284 (br), 2921, 2852, 2596-2400 (w), 1752, 1720, 1649, 1632 cm⁻¹. 400 MHz ¹H NMR (CDCl₃) δ 0.88 (3H, t, J=6.6 Hz), 1.26 (30H, bs), 1.51-1.63 (4H, m), 2.07 (1H, m, OH), 2.52-2.57 (2H, m), 3.20 (1H, d, J=2.9 Hz), 3.45 (1H, s), 3.35-3.41 (2H, m), 3.58-3.68 (3H, m), 3.80 (1H, m), 3.91 (1H, m), 4.07 (1H, m), 4.31 (1H, m), 4.46 (1H, d, J=8.1 Hz), 4.94 (1H, m), 5.16-5.28 (2H, m), 5.86 (1H, m).
FABMS (positive-ion) m/z; 583 [M-H]⁺. 607 [M+Na]⁺.

### (5) (E)-1-Propenyl 3-O-decyl-2-O-(3-oxotetradecanoyl)-α,β-D-glucopyranoside (Step Bj3)

Allyl 3-O-decyl-2-O-(3-oxotetradecanoyl)-α,β-D-glucopyranoside obtained in (4) (a 3:1 anomer mixture, 1.15 g, 1.966 mmol) was dissolved in THF (40 mL). To the solution was added Ir[C₈H₁₂(MePh₂P)₂]PF₆ (25 mg), and the mixture was stirred at room temperature for 6 hours in a nitrogen atmosphere. After completion of the reaction, the reaction solution was concentrated in vacuo to give an anomer mixture of the title compound (mixing ratio of α-anomer to β-anomer=3:1, 1.15 g, yield 100%). Part of the mixture could be separated into an α-anomer and a β-anomer using chromatography. The obtained title compound was used for the subsequent reaction without purification. physical constant of α-anomer:
Rf=0.459 (cyclohexane/EtOAc=3/2).
IR νₘₐₓ(KBr) 3408 (br), 2923, 2853, 1740, 1681 (w), 1659 (w) cm⁻¹.
400 MHz ¹H NMR (CDCl₃) δ 0.88 (3H, t, J=6.6 Hz), 1.26 (30H, bs), 1.54-1.60 (7H, m, containing 3H, dd, J=1.5, 6.6 Hz, at 1.55 ppm), 2.00 (1H, m, OH), 2.51-2.55 (2H, m), 3.48 (2H, s), 3.60-3.76 (5H, m), 3.80-3.83 (2H, m), 4.76 (1H, dd, J=3.7, 9.5 Hz), 5.16 (1H, dd, J=6.6, 12.5 Hz), 5.23 (1H, d, J=3.7 Hz), 6.11 (1H, dd, J=1.5, 12.5 Hz). physical constant of β-anomer:
Rf=0.324 (cyclohexane/EtOAc=3/2).
IR νₘₐₓ(film) 3500-3200 (br), 2922, 2853, 1745, 1720, 1682, 1648 (w), 1630 (w) cm⁻¹.
400 MHz ¹H NMR (CDCl₃) δ 0.88 (3H, t, J=5.9-7.3 Hz), 1.26 (30H, bs), 1.51-1.62 (7H, m, containing 3H, d, J=5.9 Hz at 1.54 ppm), 2.02 (1H, bs, OH), 2.52-2.58 (2H, m), 3.19 (1H, d, J=10.3 Hz), 3.36-3.48 (3H, m, containing 2H, s, at 3.45 ppm), 3.57-3.70 (3H, m), 3.81 (1H, m), 3.93 (1H, m), 4.62 (1H, d, J=8.1 Hz), 4.99 (1H, m), 5.10 (1H, m), 6.16 (1H, dd, J=1.5, 12.5 Hz).
FABMS (positive-ion) m/z; 607 [M+Na]⁺.

### (6) (E)-1-Propenyl 6-O-(t-butyldimethylsilyl)-3-O-decyl-2-O-(3-oxotetradecanoyl)-α,β-D-glucopyranoside (Step Bj4)

(E)-1-Propenyl 3-O-decyl-2-O-(3-oxotetradecanoyl)-α,β-D-glucopyranoside obtained in (5) (a 3:1 anomer mixture, 2.89 g, 4.942 mmol) was dissolved in methylene chloride (40 mL). To the solution were added t-butylmethylsilyl chloride (800 mg, 5.315 mmol) and DMAP (650 mg, 5.315 mmol), and the mixture was stirred at room temperature for 16 hours. After completion of the reaction, the reaction solution was directly purified by silica gel column chromatography (eluent: cyclohexane/ethyl acetate=6/1) to give an anomer mixture of the title compound (mixing ratio of α-anomer to β-anomer =3:1, 3.45 g, yield 100%). The β-anomer could be separated from part of the mixture using chromatography.
physical constant of β-anomer:
IR νₘₐₓ(film) 3507 (br), 2927, 2856, 1754, 1721, 1682, 1663, 1621, 1465 cm⁻¹.
400 MHz ¹H NMR (CDCl₃) δ 0.08 (6H, m), 0.86-0.91 (15H, m, containing 9H, s, at 0.90 ppm), 1.25 (30H, bs), 1.50-1.61 (5H, m), 2.55-2.58 (2H, m), 3.12-3.20 (1H, m), 3.36-3.46 (3H, m), 3.54-3.73 (3H, m), 3.83-3.93 (2H, m), 4.57 (1H, d, J=8.8 Hz), 4.98 (1H, m), 5.08 (1H, m), 6.14 (1H, d, J=10.7 Hz).
FABMS (positive-ion) m/z; 697 [M-H]⁺, 721 [M+Na]⁺ (on addition of NaI).

### (7) (E)-1-Propenyl 4-O-allyloxycarbonyl-6-O-(t-butyldimethylsilyl)-3-O-decyl-2-O-(3-oxotetradecanoyl)-α,β-D-glucopyranoside (Step Bj5)

(E)-1-Propenyl 6-O-(t-butyldimethylsilyl)-3-O-decyl-2-O-(3-oxotetradecanoyl)-α,β-D-glucopyranoside (a 3:1 anomer mixture, 3.45 g, 4.935 mmol) obtained in (6) was dissolved in toluene (60 mL). Pyridine (4.00 g, 50.569 mmol) was added to the solution, and triphosgene (2.20 g, 7.414 mmol) was then added thereto under ice cooling. After stirring the mixture for 15 minutes, allyl alcohol (6.90 g, 118.8 mmol) was added thereto and the mixture was stirred for 1 hour under ice cooling. After completion of the reaction, the reaction solution was diluted with ethyl acetate, washed with sodium bicarbonate solution and brine, filtrated and concentrated in vacuo. The resulting residue was purified by silica gel chromatography (eluent: cyclohexane/ethyl acetate =9/1) to give an anomer mixture of the title compound (mixing ratio of α-anomer to β-anomer=3:1, 2.63 g, yield 68%). The β-anomer could be separated from part of the mixture using chromatography.
β-anomer:
IR νₘₐₓ(film) 2927, 2856, 1758, 1722, 1682 (w), 1664 (w), 1628 (w), 1464 cm⁻¹.
400 MHz ¹H NMR (CDCl₃) δ 0.04 (6H, s), 0.86-0.90 (15H, m, containing 9H, s, at 0.88 ppm), 1.26 (30H, bs), 1.42-1.60 (7H, m, containing 3H, d, J=7.3 Hz, at 1.52 ppm), 2.54 (2H, t, J=7.3 Hz), 3.19 (1H, d, J=8.8 Hz), 3.43 (1H, s), 3.47-3.57 (4H, m), 3.72-3.73 (2H, m), 4.57 (1H, d, J=8.1 Hz), 4.61-4.65 (2H, m), 4.78 (1H, t, J=9.5 Hz), 4.97-5.14 (2H, m), 5.27-5.39 (2H, m), 5.92 (1H, m), 6.16 (1H, dd, J=1.5, 12.5 Hz). FABMS (positive-ion) m/z; 805 [M+Na]⁺.

### (8) 4-O-Allyloxycarbonyl-3-O-decyl -2-O-(3-oxotetradecanoyl)-α,β-D-glucopyranose (Step Bj6)

(E)-1-Propenyl 4-O-allyloxycarbonyl-6-O-(t-butyldimethylsilyl)-3-O-decyl-2-O-(3-oxotetradecanoyl)-α,β-D-glucopyranoside obtained in (7) (a 3:1 anomer mixture, 2.20 g, 4.935 mmol) was dissolved in methylene chloride (30 mL) and acetonitrile (60 mL). A 48% aqueous hydrofluoric acid solution (20 mL) was added to the solution, and the mixture was stirred at room temperature for 1.5 days. After completion of the reaction, the reaction solution was diluted with ethyl acetate, washed with water, sodium bicarbonate solution and brine, dried over magnesium sulfate, filtrated and concentrated in vacuo. The resulting residue was purified by silica gel chromatography (eluent: cyclohexane/ethyl acetate =2/3) to give an anomer mixture of the title compound (1.02 g, yield 58%).
IR νₘₐₓ(film) 3430, 2925, 2855, 1754, 1719, 1650 (w), 1466 cm⁻¹.
400 MHz ¹H NMR (CDCl₃) δ 0.88 (6H, t, J=6.6 Hz), 1.26 (30H, bs), 1.45-1.70 (4H, m), 2.50-2.57 (2H, m), 3.47-3.80 (7H, m), 3.90 (1H, t, J=9.5 Hz), 4.02 (1H, m), 4.66 (2H, d, J=5.9 Hz), 4.76-4.88 (1.5H, m), 5.29-5.40 (2H, m), 5.49 (0.5H, bs), 5.93 (1H, m).
FABMS (positive-ion) m/z; 369, 611, 651 [M+Na]⁺.
HRFABMS; Calcd. for C₃₄H₆₀O₁₀Na: 651.4085. Found: 651.4052.

### (9) 4-O-Allyloxycarbonyl-3-O-decyl-6-O-{4-O-diallylphosphono-3-O-[(R)-3-methoxydecyl]-6-O-methyl-2-O-[(Z)-11-octadecenoyl]-β-D-glucopyranosyl}-2-O-(3-oxotetradecanoyl)-α,β-D-glucopyranose (Step Ca1)

Trichloroacetimidoyl 4-O-diallylphosphono-3-O-[(R)-3-methoxydecyl]-6-O-methyl-2-O-[(Z)-11-octadecenoyl]-α-D-glucopyranoside obtained in Example 1(3) (260 mg, 0.279 mmol) and 4-O-allyloxycarbonyl-3-O-decyl-2-O-(3-oxotetradecanoyl)-α,β-D-glucopyranose obtained in (8) (175 mg, 0.279 mmol) were dissolved in methylene chloride (8 mL). Molecular sieves 4A (500 mg) were added to the solution, and the mixture was stirred for 30 minutes in a nitrogen atmosphere. Thereto were then added AgOTf (180 mg, 0.701 mmol) and TMSOTf (20 mg, 0.090 mmol), and the mixture was stirred at room temperature for 16 hours in a nitrogen atmosphere. After completion of the reaction, the reaction solution was diluted with methylene chloride, washed with sodium bicarbonate solution and saturated brine, dried over magnesium sulfate, filtrated and concentrated in vacuo. The resulting residue was purified by silica gel chromatography (eluent: cyclohexane/ethyl acetate =1/1) to give the title compound in the form of gum (anomer mixture, 240 mg, yield 65%).
IR νₘₐₓ(film) 3326 (w), 2926, 2856, 1754, 1720, 1650 (w), 1629 (w), 1465 cm⁻¹.
400 MHz ¹H NMR (CDCl₃) δ 0.88 (12H, t, J=6.6 Hz), 1.26 (60H, bs), 1.40-1.74 (10H, m), 1.99-2.04 (4H, m), 2.19 (0.5H, m), 2.32-2.36 (2H, m), 2.52-2.56 (1.5H, m), 3.21-3.90 (20H, m, containing two 3H, s, at 3.26 and 3.39 ppm, and OH), 4.26 (1H, m), 4.40 (1H, m), 4.51-4.67 (9H, m), 4.77 (1H, dd, J=3.5, 9.8 Hz), 4.85 (1H, m), 4.94 (1H, t, J=7.6 Hz), 5.24-5.40 (8H, m), 5.89-5.97 (3H, m).
FABMS (positive-ion) m/z; 1421 [M+Na]⁺.

### (10) Diallylphosphono 3-O-decyl-6-O-{4-O-diallylphosphono-3-O-[(R)-3-methoxydecyl]-6-O-methyl-2-O-[(Z)-11-octadecenoyl]-β-D-glucopyranosyl}-2-O-(3-oxotetradecanoyl)-α-D-glucopyranoside (Step Ca2)

4-O-Allyloxycarbonyl-3-O-decyl-6-O-{4-O-diallylphosphono-3-O-[(R)-3-methoxydecyl]-6-O-methyl-2-O-[(Z)-11-octadecenoyl]-β-D-glucopyranosyl}-2-O-(3-oxotetradecanoyl)-α,β-D-glucopyranose obtained in (9) (240 mg, 0.171 mmol) was dissolved in methylene chloride (8 mL). To the solution were added sodium sulfate (0.5 g), 1H-tetrazole (173 mg, 2.480 mmol) and diallyl diisopropylphosphoramidite (210 mg, 0.855 mmol), and the mixture was stirred at room temperature for 30 minutes in a nitrogen atmosphere. The reaction solution was directly purified by silica gel chromatography (eluent: cyclohexane/ethyl acetate =3/2) to give the phosphite (264 mg).
The obtained phosphite was dissolved in THF (8 mL). A 30% hydrogen peroxide solution (0.55 mL) was added to the solution, and the mixture was stirred for 30 minutes under ice cooling. After completion of the reaction, the reaction solution was diluted with ethyl acetate, washed with a 10% aqueous sodium thiosulfate solution and saturated brine, dried over magnesium sulfate, filtrated and concentrated in vacuo. The resulting residue was purified by silica gel column chromatography (eluent: cyclohexane/ethyl acetate=1/1) to give the title compound (263 mg, yield 57%).
IR νₘₐₓ(film) 2927, 2856, 1757, 1721, 1650 (w), 1464 cm⁻¹.
400 MHz ¹H NMR (CDCl₃) δ 0.88 (12H, t, J=6.6 Hz), 1.25 (60H, bs), 1.40-1.71 (10H, m), 1.99-2.02 (4H, m), 2.18-2.56 (4H, m), 3.20 (1H, m), 3.26 (3H, s), 3.38 (3H, s), 3.44 (2H, s), 3.45-3.75 (7H, m), 3.80 (1H, t, J=9.6 Hz), 3.96 (1H, m), 4.08 (1H, m), 4.30 (1H, q, J=9.4 Hz), 4.39 (1H, d, J=7.8 Hz, anomeric H), 4.44-4.59 (10H, m), 4.63-4.65 (2H, m), 4.75 (1H, t, J=9.4 Hz), 4.84 (1H, m), 4.91 (1H, m), 5.21-5.42 (12H, m), 5.80 (1H, dd, J=3.5, 6.6 Hz), 5.89-5.99 (5H, m).
FABMS (positive-ion) m/z; 1581 [M+Na]⁺.
HRFABMS; Calcd. for C₈₂H₁₄₄O₂₃P₂Na: 1581.9471. Found: 1581.9447.

### (11) Phosphono 3-O-decyl-6-O-{3-O-[(R)-3-methoxydecyl]-6-O-methyl-2-O-[(Z)-11-octadecenoyl]-4-O-phosphono-β-D-glucopyranosyl}-2-O-(3-oxotetradecanoyl)-α-D-glucopyranoside (Step Ca3)

Diallylphosphono 3-O-decyl-6-O-{4=O-diallylphosphono-3-O-[(R)-3-methoxydecyl]-6-O-methyl-2-O-[(Z)-11-octadecenoyl]-β-D-glucopyranosyl}-2-O-(3-oxotetradecanoyl)-α-D-glucopyranoside obtained in (10) (100 mg, 0.064 mmol) was dissolved in dry THF (5 mL). To the solution were added triphenylphosphine (11 mg, 0.042 mmol), triethylamine (43 mg, 0.425 mmol), formic acid (43 mg, 0.782 mmol) and Pd(PPh₃)₄ (11 mg, 0.010 mmol), and the mixture was stirred at 50°C for 16 hours in a nitrogen atmosphere. After completion of the reaction, the reaction solution was filtrated, concentrated in vacuo and purified on a column packed with 6 g of DEAE-cellulose (eluent: a 0.05 mol/L ammonium acetate solution of chloroform-methanol-water (2:3:1)) to give a fraction of the target substance. The obtained fraction was collected, and chloroform and a 0.15 mol/L hydrochloric acid solution were added thereto so that the entire mixture contained chloroform-methanol-water (1:1:1) (pH being adjusted to 2-3). The mixture was stirred in a separatory funnel, and the chloroform layer at the bottom was collected and concentrated in vacuo to give the title compound in the form of wax (46 mg, yield 54%).
IR νₘₐₓ(KBr) 3505 (w), 2925, 2854, 1747, 1719, 1653 (w), 1466 cm⁻¹.
400 MHz ¹H NMR (CDCl₃+CD₃OD, 5:1) δ 0.87-0.90 (12H, m), 1.27 (60H, bs), 1.40-1.76 (10H, m), 1.99-2.04 (4H, m), 2.19 (0.4H, t, J=7.5 Hz), 2.71 (1.6H, t, J=7.5 Hz), 2.32-2.42 (2H, m), 3.26-3.98 (22H, m, containing two 3H, s, at 3.30 and 3.41 ppm), 4.18 (1H, m), 4.51 (1H, d, J=7.8 Hz, anomeric H), 4.71 (1H, d, J=10.2 Hz), 4.88 (1H, t, J=8.6 Hz), 5.31-5.39 (2H, m), 5.68 (1H, dd, J=3.1, 7.4 Hz).
FABMS (negative-ion) m/z; 1313 [M-H]⁻, 1335 [M-2H+Na]⁻.
Anal. Calcd. for C₆₆H₁₂₄O₂₁P₂·2.5H₂O: C, 58.27; H, 9.56; P, 4.55. Found: C, 58.43; H, 9.39; P, 4.51.

### [Example 9]

Phosphono 3-O-decyl-6-O-{3-O-[(R)-3-methoxydecyl]-2-O-[(Z)-11-octadecenoyl]-4-O-phosphono-β-D-glucopyranosyl}-2-O-(3-oxotetradecanoyl)-α-D-glucopyranoside (Compound No. 9)

### (1) 4-O-Allyloxycarbonyl-3-O-decyl-6-O-{6-O-allyloxycarbonyl-4-O-diallylphosphono-3-O-[(R)-3-methoxydecyl]-2-O-[(Z)-11-octadecenoyl]-β-D-glucopyranosyl}-2-O-(3-oxotetradecanoyl)-α,β-D-glucopyranose (Step Ca1)

Trichloroacetimidoyl 6-O-(allyloxycarbonyl)-4-O-diallylphosphono-3-O-[(R)-3-methoxydecyl]-2-O-[(Z)-11-octadecenoyl]-α,β-D-glucopyranoside obtained in Example 3(4) (340 mg, 0.344 mmol) and 4-O-allyloxycarbonyl-3-O-decyl-2-O-(3-oxotetradecanoyl)-α,β-D-glucopyranose obtained in Example 8(8) (216 mg, 0.344 mmol) were dissolved in methylene chloride (10 mL). Molecular sieves 4A (620 mg) were added to the solution, and the mixture was stirred for 30 minutes in a nitrogen atmosphere. Subsequently, AgOTf (222 mg, 0.864 mmol) and TMSOTf (20 mg, 0.090 mmol) were added thereto, and the mixture was stirred at room temperature for 16 hours in a nitrogen atmosphere. After completion of the reaction, the reaction solution was diluted with methylene chloride, washed with sodium bicarbonate solution and saturated brine, dried over magnesium sulfate, filtrated and concentrated in vacuo. The resulting residue was purified by silica gel chromatography (eluent: cyclohexane/ethyl acetate =3/2) to give the title compound in the form of gum (436 mg, yield 86%).
IR νₘₐₓ(film) 3500-3200, 2927, 2856, 1754, 1650 (w), 1461 cm⁻¹.
400 MHz ¹H NMR (CDCl₃) δ 0.88 (12H, t, J=6.6 Hz), 1.26 (60H, bs), 1.40-1.48 (4H, m), 1.56-1.72 (6H, m), 1.99-2.04 (4H, m), 2.19 (0.4H, m), 2.32-2.40 (2H, m), 2.52-2.58 (1.6H, m), 3.23 (1H, m), 3.26 (3H, s), 3.46-3.90 (13H, m), 4.21-4.64 (14H, m), 4.77 (1H, dd, J=3.1, 9.8 Hz), 4.93 (1H, m), 5.23-5.41 (10H, m), 5.88-5.98 (4H, m). FABMS (positive-ion) m/z; 1491 [M+Na]⁺.
HRFABMS; Calcd. for C₇₉H₁₃₇O₂₂PNa: 1491.9237. Found: 1491.9266.

### (2) Diallylphosphono 4-O-allyloxycarbonyl-3-O-decyl-6-O-[6-O-allyloxycarbonyl-4-O-diallylphosphono-3-O-[(R)-3-methoxydecyl]-2-O-[(Z)-11-octadecenoyl]-β-D-glucopyranosyl]-2-O-(3-oxotetradecanoyl)-α-D-glucopyranoside (Step Ca2)

4-O-Allyloxycarbonyl-3-O-decyl-6-O-[6-O-allyloxycarbonyl-4-O-diallylphosphono-3-O-[(R)-3-methoxydecyl]-2-O-[(Z)-11-octadecenoyl]-β-D-glucopyranosyl]-2-O-(3-oxotetradecanoyl)-α,β-D-glucopyranose obtained in (1) (424 mg, 0.288 mmol) was dissolved in methylene chloride (12 mL). To the solution were added sodium sulfate (0.6 g), 1H-tetrazole (101 mg, 1.442 mmol) and diallyl diisopropylphosphoramidite (141 mg, 0.577 mmol), and the mixture was stirred at room temperature for 20 minutes in a nitrogen atmosphere. The reaction solution was directly purified by silica gel chromatography (eluent: cyclohexane/ethyl acetate =3/2) to give the phosphite (439 mg). The phosphite was dissolved in THF (10 mL), and a 30% hydrogen peroxide solution (1 mL) was added to the solution. The mixture was stirred for 20 minutes under ice cooling. After completion of the reaction, the reaction solution was diluted with ethyl acetate, washed with a 10% aqueous sodium thiosulfate solution and saturated brine, dried over magnesium sulfate, filtrated and concentrated in vacuo. The resulting residue was purified by silica gel column chromatography (eluent: cyclohexane/ethyl acetate=3/2) to give the title compound (355 mg, yield 76%).
IR νₘₐₓ(film) 2927, 2856, 1754, 1721 (w), 1650 (w), 1461 cm⁻¹.
400 MHz ¹H NMR (CDCl₃) δ 0.86-0.89 (12H, m), 1.25 (60H, bs), 1.37-1.46 (4H, m), 1.56-1.75 (6H, m), 1.99-2.02 (4H, m), 2.16-2.20 (0.4H, m), 2.28-2.47 (2H, m), 2.51-2.56 (1.6H, m), 3.20 (1H, m), 3.25 (3H, s), 3.38 (3H, s), 3.45-3.82 (12H, m), 3.94 (1H, dd, J=2.0, 11.0 Hz), 4.06 (1H, m), 4.22-4.30 (2H, m), 4.46 (1H, m), 4.53-4.65 (12H, m), 4.67-5.04 (3H, m), 5.22-5.41 (14H, m), 5.80 (1H, m), 5.88-5.99 (6H, m). FABMS (positive-ion) m/z; 1651 [M+Na]⁺.
HRFABMS; Calcd. for C₈₅H₁₄₆O₂₅P₂Na: 1651.9526. Found: 1651.9546.

### (3) Phosphono 3-O-decyl-6-O-[3-O-[(R)-3-methoxydecyl]-2-O-[(Z)-11-octadecenoyl]-4-O-phosphono-β-D-glucopyranosyl]-2-O-(3-oxotetradecanoyl)-α-D-glucopyranoside (Step Ca3)

Diallylphosphono 4-O-allyloxycarbonyl-3-O-decyl-6-O-[6-O-allyloxycarbonyl-4-O-diallylphosphono-3-O-[(R)-3-methoxydecyl]-2-O-[(Z)-11-octadecenoyl]-β-D-glucopyranosyl]-2-O-(3-oxotetradecanoyl)-α-D-glucopyranoside obtained in (2) (210 mg, 0.129 mmol) was dissolved in dry THF(10 mL). To the solution were added triphenylphosphine (22 mg, 0.084 mmol), triethylamine (86 mg, 0.850 mmol), formic acid (72 mg, 1.564 mmol) and Pd(PPh₃)₄ (22 mg, 0.020 mmol), and the mixture was stirred at 55°C for 16 hours in a nitrogen atmosphere. After completion of the reaction, the reaction solution was filtrated, concentrated in vacuo, and purified on a column packed with 9 g of DEAE-cellulose (eluent: a 0.05 mol/L ammonium acetate solution of chloroform-methanol-water (2:3:1)) to give a fraction of the target substance. The obtained fraction was collected, and chloroform and a 0.15 mol/L hydrochloric acid solution were added thereto so that the entire mixture contained chloroform-methanol-water (1:1:1) (pH being adjusted to about 2). The mixture was stirred in a separatory funnel, and the chloroform layer at the bottom was collected and concentrated in vacuo to give the title compound in the form of wax (80 mg, yield 48%).
IR νₘₐₓ(KBr) 3500-3000, 2925, 2854, 1743, 1720, 1466 cm⁻¹.
400 MHz ¹H NMR (CDCl₃+CD₃OD, 4:1) δ 0.88 (12H, t, J=6.6 Hz), 1.20-1.28 (60H, m), 1.24-1.75 (10H, m), 1.99-2.04 (4H, m), 2.17-2.19 (0.4H, m), 2.31-2.38 (2H, m), 2.54-2.59 (1.6H, m), 3.30 (3H, s), 3.31-4.01 (19H, m), 4.23 (1H, m), 4.50 (1H, d, J=7.8 Hz, anomeric H), 4.70 (1H, m), 4.87 (1H, t, J=8.2-8.6 Hz), 5.34-5.36 (2H, m), 5.67 (1H, dd, J=3.5, 7.4 Hz).
FABMS (negative-ion) m/z; 1299 [M-H]⁻.
Anal. Calcd. for C₆₅H₁₂₂O₂₁P₂: C, 59.98; H, 9.45; P, 4.76. Found: C, 60.10; H, 9.23; P, 4.47.

### [Example 10]

2-(Phosphonooxy)ethyl 2,3-di-O-dodecyl-6-O-[6-O-methyl-3-O-[(R)-3-methoxydecyl]-2-O-[(Z)-11-octadecenyl]-4-O-phosphono-β-D-glucopyranosyl]-α-D-glucopyranoside (Compound No. 10)

### (1) 2-(Diallylphosphonooxy)ethyl 2,3-di-O-dodecyl-6-O-[6-O-methyl-3-O-[(R)-3-methoxydecyl]-2-O-[(Z)-11-octadecenyl]-4-O-diallylphosphono-β-D-glucopyranosyl]-α-D-glucopyranoside and 2-(diallylphosphonooxy)ethyl 2,3-di-O-dodecyl-6-O-[6-O-methyl-3-O-[(R)-3-methoxydecyl]-2-O-[(Z)-11-octadecenyl]-4-O-diallylphosphono-α-D-glucopyranosyl]-α-D-glucopyranoside (Step Cc1)

Trichloroacetimidoyl 4-O-diallylphosphono-3-O-[(R)-3-methoxydecyl]-6-O-methyl-2-O-[(Z)11-octadecenoyl]-α,β-D-glucopyranoside obtained according to Example 1(3) (500 mg, 0.552 mmol) and 2-(diallylphosphonooxy)ethyl 2,3-di-O-dodecyl-α-D-glucopyranoside obtained in Example 7(4) were dissolved in methylene chloride (7.5 mL). Molecular sieves 4A (630 mg) and AgOTf (12.5 mg, 0.055 mmol) were added to the solution, and the mixture was stirred at room temperature for 1 hour in a nitrogen atmosphere. After completion of the reaction, the reaction solution was diluted with ethyl acetate, washed with sodium bicarbonate solution and saturated brine, dried over magnesium sulfate, filtrated and concentrated in vacuo. The resulting residue was purified by silica gel chromatography (eluent: ethyl acetate/methylene chloride =1/2) to give the title compounds in the form of gum, i.e., 2-(diallylphosphonooxy)ethyl 2,3-di-O-dodecyl-6-O-{6-O-methyl-3-O-[(R)-3-methoxydecyl]-2-O-[(Z)-11-octadecenyl]-4-O-diallylphosphono-β-D-glucopyranosyl}-α-D-glucopyranoside (180 mg, yield 22%) and 2-(diallylphosphonooxy)ethyl 2,3-di-O-dodecyl-6-O-{6-O-methyl-3-O-[(R)-3-methoxydecyl]-2-O-[(Z)-11-octadecenyl]-4-O-diallylphosphono-α-D-glucopyranosyl}-α-D-glucopyranoside (120 mg, yield 14%). Physical constant of 2-(diallylphosphonooxy)ethyl 2,3-di-O-dodecyl-6-O-{6-O-methyl-3-O-[(R)-3-methoxydecyl]-2-O-[(Z)-11-octadecenyl]-4-O-diallylphosphono-β-D-glucopyranosyl}-α-D-glucopyranoside:
400 MHz ¹H NMR (CDCl₃) δ 0.88 (12H, t, J=6.6 Hz), 1.26 (68H, bs), 1.40-1.79 (10H, m),1.99-2.07 (4H, m), 2.60 (1H, bs, OH), 3.17-3.90 (24H, m, containing two 3H, s, at 3.28 and 3.39 ppm), 4.05 (1H, m), 4.20-4.30 (4H, m), 4.35 (1H, d, J=7.8 Hz), 4.53-4.60 (8H, m), 4.92 (1H, d, J=3.7 Hz), 5.21-5.24 (4H, m), 5.30-5.40 (8H, m), 5.89-6.00 (4H, m).
FABMS m/z; [M+H]⁺,1499 [M+Na]⁺.
HRFABMS; Calcd. for C₈₀H₁₅₀O₁₉P₂Na: 1500.0144. Found: 1500.0145. Physical constant of 2-(diallylphosphonooxy)ethyl 2,3-di-O-dodecyl-6-O-{6-O-methyl-3-O-[(R)-3-methoxydecyl]-2-O-[(Z)-11-octadecenyl]-4-O-diallylphosphono-α-D-glucopyranosyl}-α-D-glucopyranoside:
400 MHz ¹H NMR (CDCl₃) δ 0.88 (12H, t, J=6.6 Hz), 1.26 (68H, bs), 1.40-1.79 (10H, m), 1.99-2.07 (4H, m) 3.17-3.90 (24H, m, containing two 3H, s, at 3.28 and 3.39 ppm), 3.99 (1H, m), 4.20-4.30 (4H, m), 4.53-4.58 (8H, m), 4.88 (1H, d, J=3.7 Hz), 4.92 (1H, d, J=3.7 Hz), 5.20-5.30 (4H, m), 5.89-6.00 (4H, m).
FABMS m/z; 1499 [M+Na]⁺.
HRFABMS; Calcd. for C₈₀H₁₅₀O₁₉P₂Na:1500.0144. Found:1500.0121.

### (2) 2-(Phosphonooxy)ethyl 2,3-di-O-dodecyl-6-O-{6-O-methyl-3-O-[(R)-3-methoxydecyl]-2-O-[(Z)-11-octadecenyl]-4-O-phosphono-β-D-glucopyranosyl}-α-D-glucopyranoside (Step Cc2)

2-(Diallylphosphonooxy)ethyl 2,3-di-O-dodecyl-6-O-{6-O-methyl-3-O-[(R)-3-methoxydecyl]-2-O-[(Z)-11-octadecenyl]-4-O-diallylphosphono-β-D-glucopyranosyl}-α-D-glucopyranoside obtained in (1) (180 mg, 0.12 mmol) was dissolved in dry THF (10 mL). To the solution were added triethylamine (150 mg, 1.480 mmol), formic acid (120 mg, 2.700 mmol), Pd(PPh₃)₄ (40 mg, 0.255 mmol) and triphenylphosphine (40 mg, 0.152 mmol), and the mixture was stirred at 36°C for 12 hours in a nitrogen atmosphere. After completion of the reaction, the reaction solution was filtrated, concentrated in vacuo, and purified on a column packed with 8 g of DEAE-cellulose (eluent: a 0.05 mol/L ammonium acetate solution of chloroform-methanol-water (2:3:1)) to give a fraction of the target substance. The obtained fraction was collected, and chloroform and a 0.15 mol/L hydrochloric acid solution were added thereto so that the entire mixture contained chloroform-methanol-water (1:1:1). The mixture was stirred in a separatory funnel, and the chloroform layer at the bottom was collected and concentrated in vacuo to give the title compound in the form of wax (100 mg, yield 67%).
IR νₘₐₓ(KBr) 2954, 2923, 2853, 1654 cm⁻¹.
400 MHz ¹H NMR (CDCl₃+CD₃OD, 5:1) δ 0.88 (12H, t, J=6.6 Hz), 1.26 (68H, bs), 1.39-1.75 (10H, m), 1.75-1.90 (2H, m), 1.98-2.04 (4H, m), 3.23-4.85 (26H, m, containing two 3H, s at 3.28 and 3.39 ppm), 4.00-4.10 (2H, m), 4.10-4.30 (2H, m), 4.40 (1H, d, J=7.8 Hz), 4.94 (1H, d, J=3.7 Hz), 5.33-5.38 (2H, m).
FABMS (negative-ion) m/z; 1315 [M-H]⁻.
Anal Calcd for C₆8H₁₃₄O₁₉P₂.5/2H₂O; C; 60.01,H; 10.31,P; 4.33, Found, C; 59.93, H; 10.28, P; 4.55.

### [Example 11]

2-(Phosphonooxy)ethyl 2,3-di-O-dodecyl-6-O-{6-O-methyl-3-O-[(R)-3-methoxydecyl]-2-O-[(Z)-11-octadecenyl]-4-O-phosphono-α-D-glucopyranosyl}-α-D-glucopyranoside (Compound No. 11, Step Cc2)

A reaction was performed in the same manner as in Example 10(2) using 2-(diallylphosphonooxy)ethyl 2,3-di-O-dodecyl-6-O-{6-O-methyl-3-O-[(R)-3-methoxydecyl]-2-O-[(Z)-11-octadecenyl]-4-O-diallylphosphono-α-D-glucopyranosyl}-α-D-glucopyranoside obtained in Example 10(1) (120 mg, 0.081 mmol) to give the title compound in the form of wax (90 mg, yield 84%).
IR νₘₐₓ(KBr) 3480, 2955, 2852, 1654 cm⁻¹.
400 MHz ¹H NMR (CDCl₃+CD₃OD, 5:1) δ 0.88 (12H, t, J=6.6 Hz), 1.26 (68H, br s), 1.39-1.70 (10H, m), 1.75-1.90 (2H, m), 1.98-2.04 (4H, m), 3.23-4.85 (26H, m, containing two 3H, s at 3.28 and 3.39 ppm), 4.00-4.25 (4H, m), 4.90 (1H, d, J=3.7 Hz), 4.99 (1H, d, J=3.7 Hz), 5.33-5.38 (2H, m).
FABMAS (negative ion) m/z; 1315 [M-H]⁻.
Anal Calcd for C₆₈H₁₃₄O₁₉P₂.H₂O; C; 60.01,H; 10.31,P; 4.33, Found, C; 59.93, H; 10.28, P; 4.55.

### [Example 12]

Phosphono 6-O-{4-O-phosphono-3-O-[(R)-3-methoxydecyl]-6-O-methyl-2-O-[(Z)-11-octadecenoyl]-β-D-glucopyranosyl}-3-O-dodecyl-2-O-[(R)-3-hydroxytetradecyl]-α,β-D-glucopyranoside (Compound No. 12)

### (1) (E)-1-Propenyl 4-O-allyloxycarbonyl-2-O-[(R)-3-(allyloxycarbonyloxy)tetradecyl]-3-O-dodecyl-α-D-glucopyranoside

(E)-1-Propenyl 4-O-allyloxycarbonyl-2-O-[(R)-3-(allyloxycarbonyloxy)tetradecyl]-6-O-(t-butyldimethylsilyl)-3-O-dodecyl-α-D-glucopyranoside obtained in Reference Example 11 (840 mg, 0.951 mmol) was dissolved in a methylene chloride (5 mL)-acetonitrile (10 mL) mixed solvent. A 48% aqueous hydrofluoric acid solution (200 mg) was added to the solution, and the mixture was stirred at room temperature for 30 minutes. After completion of the reaction, the reaction solution was diluted with ethyl acetate, washed with a saturated sodium bicarbonate solution, dried over magnesium sulfate, filtrated and concentrated in vacuo. The resulting residue was purified by silica gel column chromatography (eluent: cyclohexane/ethyl acetate=2/1) to give the title compound (467 mg, yield 64%).
IR νₘₐₓ(KBr) 3527, 2925, 2854, 1747, 1679 cm⁻¹.
400 MHz ¹H NMR (CDCl₃) δ 0.88 (6H, t, J=6.6 Hz), 1.25 (36H, bs), 1.50-1.65 (7H, m), 1.87-1.92 (2H, m), 2.33 (1H, OH), 3.37 (1H, dd, J=2.9, 9.5 Hz), 3.55-3.81 (8H, m), 4.60-4.67 (4H, m), 4.72 (1H, m), 4.80 (1H, m), 5.13 (1H, d, J=3.7 Hz), 5.18 (1H, qd, J=6.6, 12.5 Hz), 5.25-5.40 (4H, m), 5.89-5.98 (2H, m), 6.16 (1H, qd, J=1.5, 12.5 Hz).
FABMS (positive-ion) m/z; 711 [M+H]⁺, 791 [M+Na]⁺.
HRFABMS; Calcd. for C₄₃H₇₆O₁₁Na: 791.5286. Found: 791.5281.

### (2) 4-O-Allyloxycarbonyl-2-O-[(R)-3-(allyloxycarbonyloxy)tetradecyl]-3-O-dodecyl-D-glucopyranose (Step Bb1)

(E)-1-Propenyl 4-O-allyloxycarbonyl-2-O-[(R)-3-(allyloxycarbonyloxy)tetradecyl]-3-O-dodecyl-D-α-glucopyranoside obtained in (1) (500 mg, 01651 mmol) was dissolved in acetonitrile (25 mL). A 48% aqueous hydrofluoric acid solution (1.46 mL) was added to the solution, and the mixture was stirred at room temperature for 16 hours. After completion of the reaction, the reaction solution was diluted with methylene chloride, washed with a saturated sodium bicarbonate solution, dried over magnesium sulfate, filtrated and concentrated in vacuo. The resulting residue was purified by silica gel column chromatography (eluent: hexane/ethyl acetate=2/1) to give the title compound as an oil (340 mg, yield 72%).
400 MHz ¹H NMR (CDCl₃) δ 0.86 (6H, t, J=6.6 Hz), 1.25 (360H, bs), 1.49-1.65(4H, m), 1.80-2.00 (2H, m), 2.39 (1H, m), 3.2 (1H, d, d, J= 2.9, 9.5 Hz), 3.60-4.00 (8H, m), 4.58-4.72 (5H, m), 4.80-4.90 (1H, m), 5.25-5.40 (4H, m), 5.88-6.00 (2H, m). FABMS (positive-ion) m/z; 751 [M+Na]⁺.

### (3) 4-O-Allyloxycarbonyl-2-O-[(R)-3-(allyloxycarbonyloxy)tetradecyl]-3-O-dodecyl-6-O-{4-O-diallylphosphono-3-O-[(R)-3-methoxydecyl]-6-O-methyl-2-O-[(Z)-11-octadecenoyl]-β-D-glucopyranosyl}-D-glucopyranose (Step Ca1)

Trichloroacetimidoyl 4-O-diallylphosphono-3-O-[(R)-3-methoxydecyl]-6-O-methyl-2-O-[(Z)-11-octadecenoyl]-α-D-glucopyranoside obtained in Example 1(3) (666 mg, 0.700 mmol) and 4-O-allyloxycarbonyl-2-O-[(R)-3-(allyloxycarbonyloxy)tetradecyl]-3-O-dodecyl-D-glucopyranose obtained in (2) (520 mg, 0.700 mmol) were dissolved in methylene chloride (20 mL). Molecular sieves 4A (400 mg) were added to the solution, and the mixture was stirred at room temperature for 20 minutes in a nitrogen atmosphere. Thereto was further added AgOTf (20 mg, 0.070 mmol), and the mixture was stirred at room temperature for 20 minutes in a nitrogen atmosphere. After completion of the reaction, the reaction solution was diluted with methylene chloride, washed with sodium bicarbonate solution and saturated brine, dried over magnesium sulfate, filtrated and concentrated in vacuo. The resulting residue was purified by silica gel chromatography (eluent: cyclohexane/ethyl acetate =2/1) to give the title compound in the form of gum (520 mg, yield 52%).
400 MHz ¹H NMR (CDCl₃) δ 0.88 (12H, t, J=6.6 Hz), 1.25 (66H, bs), 1.40-1.90 (13H, m), 1.92-2.02 (4H, m), 2.30-2.42 (2H, m), 3.20-3.90 (20H, m, containing 3H, s, at 3.17 ppm and 3H, s, at 3.37 ppm), 4.10-4.70 (14H, m), 4.80-4.99 (2H, m), 5.20-5.4 (11Hm), 5.89-6.00 (4H, m).
FABMS (positive-ion) m/z; 1521 [M+Na]⁺.
HRFABMS; Calcd. for C₈₂H₁₄₇O₂₁PNa: 1522.007. Found: 1522.0068.

### (4) Diallylphosphono 4-O-allyloxycarbonyl-2-O-[(R)-3-(allyloxycarbonyloxy)tetradecyl]-3-O-dodecyl-6-O-{4-O-diallylphosphono-3-O-[(R)-3-methoxydecyl]-6-O-methyl-2-[(Z)-11-octadecenoyl]-β-D-glucopyranosyl}-α,β-D-glucopyranoside (Step Ca2)

4-O-Allyloxycarbonyl-2-O-[(R)-3-(allyloxycarbonyloxy) tetradecyl]-3-O-dodecyl-6-O-{4-O-diallylphosphono-3-O-[(R)-3-methoxydecyl]-6-O-methyl-2-O-[(Z)-11-octadecenoyl]-β-D-glucopyranosyl}-D-glucopyranose obtained in (3) (430 mg, 0.287 mmol) was dissolved in methylene chloride (60 mL). To the solution were added sodium sulfate (900 mg), 1H-tetrazole (84.3 mg, 1.2 mmol) and diallyl diisopropylphosphoramidite (105 mg, 0.43 mmol). The mixture was stirred at room temperature for 30 minutes in a nitrogen atmosphere. After completion of the reaction, the reaction solution was directly purified by silica gel chromatography (eluent: hexane/ethyl acetate =2/1) to give the phosphite as an oil (500 mg).
The phosphite was dissolved in THF (25 mL). A 30% hydrogen peroxide solution (500 mg) was added to the solution, and the mixture was stirred for 30 minutes under ice cooling. After completion of the reaction, the reaction solution was diluted with ethyl acetate, washed with a 10% sodium thiosulfate solution and brine, dried over magnesium sulfate, filtrated and concentrated in vacuo. The resulting residue was purified by silica gel chromatography (eluent: hexane/ethyl acetate =3/2) to give the title compound as an oil (430 mg, yield 90%).
400 MHz ¹H NMR (CDCl₃) δ 0.80-1.90 (12H, m), 1.26 (66H, bs), 1.40-1.75 (10H, m), 1.80-1.90 (2H, m), 1.99-2.05 (4H, m), 2.25-2.50 (2H, m), 3.18-3.90 (221H, m, containing two 3H, s at 3.27 and 3.38 ppm), 4.00 (1H, m), 4.20-4.405 (21H, m), 4.56-4.63 (11H, m), 4.70-5.05 (2H, m), 5.23-5.43 (2H, m), 5.86-5.98 (5H, m). FABMS(positive-ion) m/z; 1681 [M+Na]⁺.

### (5) Phosphono 6-O- {4-O-phosphono-3-O-[(R)-3-methoxydecyl]-6-O-methyl-2-[(Z)-11-octadecenoyl]-β-D-glucopyranosyl}-3-O-dodecyl-2-O-[(R)-3-hydroxytetradecyl]-α,β-D-glucopyranoside (Step Ca3)

Diallylphosphono 4-O-allyloxycarbonyl-2-O-[(R)-3-(allyloxycarbonyloxy) tetradecyl]-3-O-dodecyl-6-O-{4-O-diallylphosphono-3-O-[(R)-3-methoxydecyl]-6-O-methyl-2-[(Z)-11-octadecenoyl]-β-D-glucopyranosyl}-α,β-D-glucopyranoside obtained in (4) (180 mg, 0.090 mmol) was dissolved in dry THF (10 mL). To the solution were added triphenylphosphine (30 mg, 0.114 mmol), triethylamine (111 mg, 1.099 mmol), formic acid (93 mg, 2.020 mmol) and Pd(PPh₃)₄ (30 mg, 0.026 mmol). The mixture was stirred at 36°C for 12 hours in a nitrogen atmosphere. After completion of the reaction, the reaction solution was filtrated, concentrated in vacuo and purified on a column packed with 8 g of DEAE-cellulose (eluent: a 0.05 mol/L ammonium acetate solution of chloroform-methanol-water (2:3:1)) to give a fraction of the target substance. The obtained fraction was collected, and chloroform and a 0.15 mol/L hydrochloric acid solution were added thereto so that the entire mixture contained chloroform-methanol-water (1:1:1). The mixture was stirred in a separatory funnel, and the chloroform layer at the bottom was collected and concentrated in vacuo to give the title compound in the form of wax (90 mg, yield 75%).
IR νₘₐₓ(KBr) 3506 (w), 3345 (w), 2923, 2853, 1720, 1465, 1466 cm⁻¹.
400 MHz ¹H NMR (CDCl₃-CD₃OD, 5:1) δ 0.86-0.90 (12H, m), 1.27 (66H, bs), 1.40-1.80 (12H, m), 2.00-2.08 (4H, m), 2.24-2.28 (2H, m), 3.13-4.06 (26H, m, containing two 3H, s at 3.30 and 3.40 ppm), 4.2-4.4 (1H, m), 4.45-4.59 (1H, m), 4.92-4.97 (1H, m),5.34-5.38 (2H, m).
FABMS (negative-ion) m/z; 1329 [M-H]⁻, 1322.
Anal. Calcd for C₆₈H₁₃₂O₂₀P₂.3H₂O: C, 58.99; H, 10.00; P, 4.23. Found: C, 58.94; H, 10.04; P4.47.

### [Example 13]

Phosphono 6-O-{4-O-phosphono-3-O-[(R)-3-methoxydecyl]-6-O-methyl-2-O-[(Z)-11-octadecenyl]-β-D-glucopyranosyl}-3-O-dodecyl-2-O-[(R)-3-hydroxytetradecyl]-α,β-D-glucopyranoside (Compound No. 13)

### (1) (E)-1-Propenyl 4-O-allyloxycarbonyl-6-O-{4-O-diallylphosphono-3-O-[(R)-3-methoxydecyl]-6-O-methyl-2-O-[(Z)-11-octadecenyl]-β-D-glucopyranosyl}-3-O-dodecyl-2-O-[(R)-3-(allyloxycarbonyloxy)tetradecyl]-α-D-glucopyranoside and (E)-1-propenyl 4-O-allyloxycarbonyl-6-O-{4-O-diallylphosphono-3-O-[(R)-3-methoxydecyl]-6-O-methyl-2-O-[(Z)-11-octadecenyl]-α-D-glucopyranosyl}-3-O-dodecyl-2-O-[(R)-3-(allyloxycarbonyloxy)tetradecyl]-α-D-glucopyranoside (Step Cf1)

Trichloroacetimidoyl 4-O-diallylphosphono-3-O-[(R)-3-methoxydecyl]-6-O-methyl-2-O-[(Z)11-octadecenoyl]-α,β-D-glucopyranoside obtained according to Example 1(3) (760 mg 0.840 mmol) and (E)-1-propenyl 4-O-allyloxycarbonyl-2-O-[(R)-3-(allyloxycarbonyloxy)tetradecyl]-3-O-dodecyl-α-D-glucopyranoside obtained in Example 12(1) (645 mg, 0.840 mmol) were dissolved in methylene chloride (50 mL). Molecular sieves 4A (400 mg) and AgOTf (21 mg, 0.0840 mmol) were added to the solution, and the mixture was stirred at room temperature for 60 minutes in a nitrogen atmosphere. After completion of the reaction, the reaction solution was diluted with methylene chloride, washed with sodium bicarbonate solution and saturated brine, dried over magnesium sulfate, filtrated and concentrated in vacuo. The resulting residue was purified by silica gel chromatography (eluent: hexane/ethyl acetate =4/1) to give the title compound in the form of gum, i.e., (E)-1-propenyl 4-O-allyloxycarbonyl-6-O-{4-O-diallylphosphono-3-O-[(R)-3-methoxydecyl]-6-O-methyl-2-O-[(Z)-11-octadecenyl]-β-D-glucopyranosyl}-3-O-dodecyl-2-O-[(R)-3-(allyloxycarbonyloxy)tetradecyl]-α-D-glucopyranoside (400 mg, yield 32%) and (E)-1-propenyl 4-O-allyloxycarbonyl-6-O-{4-O-diallylphosphono-3-O-[(R)-3-methoxydecyl]-6-O-methyl-2-O-[(Z)-11-octadecenyl]-α-D-glucopyranosyl}-3-O-dodecyl-2-O-[(R)-3-(allyloxycarbonyloxy)tetradecyl]-α-D-glucopyranoside (460 mg, yield 37%).
Physical constant of (E)-1-propenyl 4-O-allyloxycarbonyl-6-O-{4-O-diallylphosphono-3-O-[(R)-3-methoxydecyl]-6-O-methyl-2-O-[(Z)-11-octadecenyl]-β-D-glucopyranosyl}-3-O-dodecyl-2-O-[(R)-3-(allyloxycarbonyloxy)tetradecyl]-α-D-glucopyranoside:
400 MHz ¹H NMR (CDCl₃) δ 0.88 (12H, t, J=6.6 Hz), 1.25 (66H, bs), 1.40-1.80 (13H, m, containing 3H, dd, J=1.5, 6.6 Hz at 1.55 ppm), 1.82-1.89 (2H, m),1.98-2.02 (4H, m) 3.1-3.2 (2H, m), 3.22-3.79 (20H, m, containing 3H, s, at 3.28 ppm, and 3H, s, at 3.37 ppm),3.8-4.0 (4H, m),4.19-4.3 (2h, m),4.53-4.87 (12H, m),5.03-5.4 (12H, m), 5.88-5.97 (4H, m), 6.13 (1H, qd, J=1.5, J=12.5 Hz).
FABMS (positive-ion) m/z; [M+Na,] 1547⁺.
HRFABMS; Calcd. for C₈₅H₁₅₃O₂₀PNa:1500.0591 Found;1548.0624.
Physical constant of (E)-1-propenyl 4-O-allyloxycarbonyl-6-O-{4-O-diallylphosphono-3-O-[(R)-3-methoxydecyl]-6-O-methyl-2-O-[(Z)-11-octadecenyl]-α-D-glucopyranosyl}-3-O-dodecyl-2-O-[(R)-3-(allyloxycarbonyloxy)tetradecyl]-α-D-glucopyranoside:
400 MHz ¹H NMR (CDCl₃) δ 0.88 (12H, t, J=6.6 Hz), 1.25 (66H, bs), 1.40-1.80 (13H, m, containing 3H, dd, J=1.5, 6.6 Hz at 1.55 ppm), 1.82-2.02 (6H, m), 3.22-4.0 (22H, m, containing 3H, s, at 3.28 ppm, and 3H, s, at 3.37 ppm), 4.23 (1H, m), 4.53-4.88 (12H, m), 5.03-5.4 (12H, m), 5.89-5.98 (4H, m), 6.13 (1H, qd, J=1.5, 12.5 Hz, NH).
FABMS (positive-ion) m/z; [M+Na,] 1547⁺.
HRFABMS; Calcd. for C₈₅H₁₅₃O₂₀PNa: 1548.0590, Found; 1548.0582.

### (2) 4-O-Allyloxycarbonyl-2-O-[(R)-3-(allyloxycarbonyloxy)tetradecyl]-3-O-dodecyl-6-O-{4-O-diallylphosphono-3-O-[(R)-3-methoxydecyl]-6-O-methyl-2-O-[(Z)-11-octadecenyl]-β-D-glucopyranosyl}-D-glucopyranose (Step Cf2)

(E)-1-Propenyl 4-O-allyloxycarbonyl-6-O-{4-O-diallylphosphono-3-O-[(R)-3-methoxydecyl]-6-O-methyl-2-O-[(Z)-11-octadecenyl]-β-D-glucopyranosyl}-3-O-dodecyl-2-O-[(R)-3-(allyloxycarbonyloxy)tetradecyl]-α-D-glucopyranoside obtained in (1) (370 mg, 0.242 mmol) was dissolved in THF (20 mL) and water (0.5 mL). Iodine (92.5 mg, 0.365 mmol) was added to the solution, and the mixture was stirred at room temperature for 4 hours. After completion of the reaction, the reaction solution was diluted with ethyl acetate, washed with an aqueous sodium thiosulfate solution and saturated brine, dried over magnesium sulfate, filtrated and concentrated in vacuo. The resulting residue was purified by silica gel chromatography (eluent: hexane/ethyl acetate =3/1) to give the title compound in the form of gum (250 mg, yield 69%). 400 MHz ¹H NMR (CDCl₃) δ 0.86-0.89 (12H, m), 1.25 (66H, bs), 1.40-2.02 (17H, m, containing OH), 3.10-3.90 (20H, m, containing 3H, s, at 3.17 ppm, and 3H, s, at 3.37 ppm4.0-4.70 (14H, m), 4. 81 (1H, m), 5.18-5.42 (11H, m), 5.89-6.00 (4H, m).
FABMS (positive-ion) m/z; 1507 [M+Nal]⁺.
HRFABMS; Calcd. for C₈₂H₁₄₉O₂₀PNa;1508.0277. Found; 1508.0294.

### (3) Diallylphosphono 4-O-allyloxycarbonyl-2-O-[(R)-3-(allyloxycarbonyloxy)tetradecyl]-3-O-dodecyl-6-O-{4-O-diallylphosphono-3-O-[(R)-3-methoxydecyl]-6-O-methyl-2-O-[(Z)-11-octadecenyl]-β-D-glucopyranosyl}-α,β-D-glucopyranoside (Step Ca2)

4-O-Allyloxycarbonyl-2-O-[(R)-3-(allyloxycarbonyloxy)tetradecyl]-3-O-dodecyl-6-O-{4-O-diallylphosphono-3-O-[(R)-3-methoxydecyl]-6-O-methyl-2-O-[(Z)-11-octadecenyl]-β-D-glucopyranosyl}-D-glucopyranose obtained in (2) (250 mg, 0.168 mmol) was dissolved in methylene chloride (30 mL). To the solution were added sodium sulfate (540 mg), 1H-tetrazole (51 mg, 0.700 mmol) and diallyl diisopropylphosphoramidite (64 mg, 0.250 mmol). The mixture was stirred at room temperature for 30 minutes in a nitrogen atmosphere. After completion of the reaction, the reaction solution was directly purified by silica gel chromatography (eluent: hexane/ethyl acetate =3/1) to give the phosphite as an oil (268 mg).

The obtained phosphite was dissolved in THF (25 mL). A 30% hydrogen peroxide solution (500 mg) was added to the solution, and the mixture was stirred for 30 minutes under ice cooling. After completion of the reaction, the reaction solution was diluted with ethyl acetate, washed with a 10% sodium thiosulfate solution and brine, dried over magnesium sulfate, filtrated and concentrated in vacuo. The resulting residue was purified by silica gel chromatography (eluent: hexane/ethyl acetate =2/1) to give an anomer mixture of the title compound as an oil (240 mg, yield 86%).
400 MHz ¹H NMR (CDCl₃) δ 0.80-1.90 (12H, m), 1.26 (66H, bs), 1.40-1.70 (10H, m), 1.70-1.80 (2H, m), 1.80-1.90 (2H, m), 1.90-205 (4H, m), 3.10 (1H, m), 3.27 (3H, s), 3.33 (1H, m), 3.38 (3H, m), 3.40-390 (14H, m), 4.1-4.2 (1H, m), 4.25 (1H, m), 4.40-4.80 (14H, m), 5.1 (1H, m5.19-5.40 (14H, m), 5.86-5.98 (6H, m).
FABMS(positive-ion) m/z; 1667 [M+Na]⁺.
HRFABMS,C₈₈H₁₅₈O₂₃P₂ Calcd. for: 1668.0567. Found: 1668.0590.

### (4) Phosphono 6-O-{4-O-phosphono-3-O-[(R)-3-methoxydecyl]-6-O-methyl-2-O-[(Z)-11-octadecenyl]-β-D-glucopyranosyl}-3-O-dodecyl-2-O-[(R)-3-hydroxytetradecyl]-α,β-D-glucopyranoside (Step Ca3)

Diallylphosphono 4-O-allyloxycarbonyl-2-O-[(R)-3-(allyloxycarbonyloxy)tetradecyl]-3-O-dodecyl-6-O-{4-O-diallylphosphono-3-O-[(R)-3-methoxydecyl]-6-O-methyl-2-O-[(Z)-11-octadecenyl]-β-D-glucopyranosyl}-α,β-D-glucopyranoside obtained in (3) (anomer mixture, 150 mg, 0.091 mmol) was dissolved in dry THF (20 mL). To the solution were added triphenylphosphine (30 mg, 0.114 mmol), triethylamine (111 mg, 1.09 mmol), formic acid (93 mg, 2.02 mmol) and Pd(PPh₃)₄ (30 mg, 0.026 mmol). The mixture was stirred at 36°C for 12 hours in a nitrogen atmosphere. After completion of the reaction, the reaction solution was filtrated, concentrated in vacuo and purified on a column packed with 8 g of DEAE-cellulose (eluent: a 0.05 mol/L ammonium acetate solution of chloroform-methanol-water (2:3:1)) to give a fraction of the target substance. The obtained fraction was collected, and chloroform and a 0.15 mol/L hydrochloric acid solution were added thereto so that the entire mixture contained chloroform-methanol-water (1:1:1). The mixture was stirred in a separatory funnel, and the chloroform layer at the bottom was collected and concentrated in vacuo to give an anomer mixture of the title compound in the form of wax (88 mg, yield 73%).
IR νₘₐₓ(KBr) 3291 (w), 3076 (w), 2925, 2853, 1628, 1554, 1466 cm⁻¹. 400 MHz ¹H NMR (CDCl₃-CD₃OD, 5:1) δ 0.86-0.90 (12H, m), 1.27 (62H, bs), 1.40-1.75 (12H, m), 1.99-2.03 (4H, m), 2.20-2.26 (2H, m), 3.23-4.12 (26H, m, containing two 3H, s at 3.30 and 3.40 ppm), 4.76 (1H, d, J=8.1 Hz), 5.33-5.39 (2H, m), 5.71 (1H, dd, J=3.3, 7.0 Hz).
FABMS (negative-ion) m/z; 1315.
Anal. Calcd for C₆₈H₁₃₄O₁₉P₂.3H₂O: C; 59.52,H; 9.93, P; 4.42. Found: C; 59.94, H; 10.29, P; 4.52.

### [Reference Example 11]

(E)-1-Propenyl 4-O-allyloxycarbonyl-2-O-[(R)-3-(allyloxycarbonyloxy)tetradecyl]-6-O-(t-butyldimethylsilyl)-3-O-decyl-α-D-glucopyranoside

### (1) 1,2:5,6-Di-O-isopropylidene-3-O-decyl-α-D-glucofuranose

1,2:5,6-Di-O-isopropylidene-α-D-glucofuranose (17.0 g, 65.31 mmol) and 1-(methanesulfonyloxy)decane (15.34 g, 64.90 mmol) were dissolved in DMF (65 mL). Sodium hydride (55% dispersion in oil, 3.40 mg, 77.92 mmol) was added to the solution under ice cooling. The mixture was stirred at 0°C for 15 minutes and then at room temperature overnight. Subsequently, methanol was added to the reaction solution under ice cooling to decompose sodium hydride. The reaction solution was diluted with ethyl acetate, washed with water and saturated brine, dried over magnesium sulfate, filtrated and concentrated in vacuo. The resulting residue was purified by silica gel chromatography (eluent: cyclohexane/ethyl acetate =9/1, then 4/1) to give the title compound as an oil (22.5 g, yield 98%).
IR νₘₐₓ(film) 2987, 2928, 2857 cm⁻¹.
400 MHz ¹H NMR (CDCl₃) δ 0.88 (3H, t, J=6.6 Hz), 1.27 (14H, bs), 1.32 (3H, s), 1.36 (3H, s), 1.43 (3H, s), 1.50 (3H, s), 1.55-1.58 (2H, m), 3.52 (1H, m), 3.59 (1H, m), 3.86 (1H, d, J=3.7 Hz), 3.99 (1H, dd, J=5.9, 8.8 Hz), 4.08 (1H, d, J=5.9 Hz), 4.11 (1H, t, J=6.6 Hz), 4.14 (1H, m), 4.31 (1H, m), 4.53 (1H, d, J=4.4 Hz), 5.88 (1H, d, J=4.4 Hz).

### (2) Allyl 3-O-decyl-α-D-glucopyranoside

The compound obtained in (1) (20.6 g) was dissolved in allyl alcohol (300 mL) containing 2% of hydrochloric acid, and the mixture was refluxed for 15 minutes. The reaction solution was concentrated in vacuo, and the resulting residue was purified by silica gel chromatography (eluent: cyclohexane/ethyl acetate =1/1, then 1/3) to give the title compound as an oil (14.6g, yield 98%, a,b-mixture). The title compound was used for the subsequent reaction without further purification.

### (3) Allyl 3-O-decyl-4,6-O-isopropylidene-α,β-D-glucopyranoside

The compound obtained in (2) (1.20 g, 3.33 mmol) was dissolved in DMF (2.5 mL). To the solution were added 2,2-dimethoxypropane (2.5 mL) and p-TsOH (50 mg), and the mixture was stirred at room temperature for 6 hours. The reaction solution was diluted with ethyl acetate, washed with sodium bicarbonate solution and saturated brine, dried over magnesium sulfate, filtrated and concentrated in vacuo. The resulting residue was purified by silica gel chromatography (eluent: cyclohexane/ethyl acetate =3/1) to give the title compound as an oil, i.e., an α-form (902 mg, yield 68%) and a β-form (293 mg, yield 22%).
α-form:
400 MHz ¹H NMR (CDCl₃) δ 0.88 (3H, t, J=6.6 Hz), 1.26 (14H, bs), 1.41 (3H, s), 1.49 (3H, s), 1.53-1.60 (2H, m), 2.27 (1H, d, J=7.3 Hz, OH), 3.46-3.86 (8H, m), 4.04 (1H, m), 4.21 (1H, m), 4.92 (1H, d, J=3.7 Hz), 5.22-5.35 (2H, m), 5.93 (1H, m).
β-form:
400 MHz ¹H NMR (CDCl₃) δ 0.88 (3H, t, J=6.6 Hz), 1.26 (14H, bs), 1.41 (3H, s), 1.49 (3H, s), 1.55-1.60 (2H, m), 2.29 (1H, d, J=2.2 Hz, OH), 3.25 (1H, m), 3.31 (1H, t, J=8.8 Hz), 3.45 (1H, m), 3.59-3.67 (2H, m), 3.75-3.82 (2H, m), 3.91 (1H, dd, J=5.1, 11.0 Hz), 4.14 (1H, dd, J=6.2, 12.8 Hz), 4.35 (1H, dd, J=5.1, 12.5 Hz), 4.40 (1H, d, J=8.1 Hz), 4.92 (1H, d, J=3.7 Hz), 5.20-5.35 (2H, m), 5.93 (1H, m).

### (4) Allyl 2-O-[(R)-3-(t- butyldimethylsilyloxy)tetradecyl]-3-O-decyl-4,6-O-isopropylidene-α-D-glucopyranoside

The α-form obtained in (3) (4.58 g, 11.45 mmol) and (R)-3-(t-butyldimethylsilyloxy)-1-(methylsulfonyloxy)tetradecane (4.84 g, 11.45 mmol) were dissolved in DMF (3 mL). Sodium hydride (55% dispersion in oil, 1.50 g, 34.38 mmol) was added to the solution, and the mixture was stirred at room temperature overnight and further at 50°C for 2 hours. Then, methanol was added to the reaction solution under ice cooling to decompose sodium hydride. The reaction solution was then diluted with ethyl acetate, washed with water and saturated brine, dried over magnesium sulfate, filtrated and concentrated in vacuo. The resulting residue was purified by silica gel chromatography (eluent: cyclohexane/ethyl acetate =9/1) to give the title compound as an oil (5.60 g, yield 67%).
400 MHz ¹H NMR (CDCl₃) δ 0.04 (6H, s), 0.878 (9H, s), 0.88 (6H, t, J=6.6 Hz), 1.26 (32H, bs), 1.39-1.59 (10H, m, containing 3H, s at d 1.41 ppm and 3H, s at d 1.48 ppm), 1.67-1.77 (2H, m), 3.29 (1H, dd, J=3.7, 8.8 Hz), 3.45-3.85 (10H, m), 4.04 (1H, dd, J=6.6, 12.5 Hz), 4.18 (1H, dd, J=5.1, 12.5 Hz), 4.92 (1H, d, J=3.7 Hz), 5.20-5.34 (2H, m), 5.92 (1H, m).
FABMS (positive-ion) m/z, 727 [M+H]⁺.

### (5) Allyl 2-O-[(R)-3-hydroxytetradecyl]-3-O-decyl-α-D-glucopyranoside

The compound obtained in (4) (500 mg, 0.688 mmol) was dissolved in methanol (10 mL). To the solution was added p-toluenesulfonic acid (24 mg), and the mixture was stirred at room temperature for 40 minutes; concentrated in vacuo, and then diluted with ethyl acetate. The solution was washed with sodium bicarbonate solution and saturated brine, dried over magnesium sulfate, filtrated and concentrated in vacuo. The resulting residue was purified by silica gel chromatography (eluent: cyclohexane/ethyl acetate =1/1) to give the title compound (217 mg, yield 58%).
IR νₘₐₓ(KBr) 3358 (broad), 3270 (broad), 2921, 2850, 1466 cm⁻¹.
400 MHz ¹H NMR (CDCl₃) δ 0.88 (6H, t, J=6.6 Hz), 1.26 (32H, bs), 1.40-1.73 (6H, m), 1.93 (1H, t, J=6.5 Hz, OH), 2.44 (1H, d, J=2.2 Hz, OH), 2;93 (1H, bs, OH), 3.30 (1H, dd, J=3.7, 9.5 Hz), 3.50-3.92 (10H, m), 4.05 (1H, m), 4.20 (1H, m), 5.01 (1H, d, J=3.7 Hz), 5.22-5.34 (2H, m), 5.95 (1H, m).
FABMS (positive-ion) m/z, 573 [M+H]⁺, 595 [M+Na]⁺.
HRFABMS, Calcd. for C₃₃H₆₄O₇Na: 595.4550. Found: 595.4541.

### (6) (E)-1-Propenyl 2-O-[(R)-3-hydroxytetradecyl]-3-O-decyl-α-D-glucopyranoside

The compound obtained in (5) (210 mg, 0.367 mmol) was dissolved in tetrahydrofuran (THF, 4 mL). To the solution was added Ir[C₈H₁₂(MePh₂P)₂]PF₆ (5 mg) activated by hydrogen. The mixture was stirred at room temperature overnight and then concentrated in vacuo to give the title compound (210 mg, quantitative). The obtained title compound was used for the subsequent reaction without purification.
400 MHz ¹H NMR (CDCl₃) δ 0.88 (6H, t, J=6.6 Hz), 1.26 (32H, bs), 1.41-1.73 (9H, m), 1.87 (1H, br, OH), 2.47 (1H, br, OH), 2.74 (1H, bs, OH), 3.32 (1H, m), 3.54-3.92 (10H, m), 5.17 (1H, d, J=3.7 Hz), 5.20 (1H, m), 6.19 (1H, J=1.5, 2.5 Hz).
FABMS (positive-ion) m/z, 573 [M+H]⁺, 595 [M+Na]⁺.

### (7) (E)-1-Propenyl 6-O-t-butyldimethylsilyl-3-O-decyl-2-O-[(R)-3-hydroxytetradecyl]-α-D-glucopyranoside

The compound obtained in (6) (200 mg, 0.349 mmol) was dissolved in methylene chloride (4 mL). To the solution were added t-butyldimethylsilyl chloride (58 mg, 0.384 mmol) and DMAP (47 mg, 0.384 mmol). The mixture was stirred at room temperature overnight and then directly purified by silica gel chromatography (eluent: cyclohexane/ethyl acetate=4/1) to give the title compound as an oil (217 mg, yield 90%).
IR νₘₐₓ(film) 3456 (broad), 2926, 2855, 1465 cm⁻¹.
400 MHz ¹H NMR (CDCl₃) δ 0.08 (6H, s), 0.88 (6H, t, J=6.6 Hz), 0.90 (9H, s), 1.26 (32H, bs), 1.40-1.75 (9H, m, containing 3H, d, J=7.3 Hz at 1.55 ppm), 2.75 (1H, bs, OH), 2.84 (1H, bs, OH), 3.30 (1H, m), 3.53-3.87 (10H, m), 5.14 (1H, d, J=2.9 Hz), 5.20 (1H, m6.20 (1H, d, J=12.5 Hz).
FABMS (positive-ion) m/z, 687 [M+H]⁺, 709 [M+Na]⁺.
HRFABMS Calcd. for C₃₉H₇₈O₇SiNa: 709.5414. Found: 709.5393.

### (8) (E)-1-Propenyl 4-O-allyloxycarbonyl-2-O-[(R)-3-(allyloxycarbonyloxy)tetradecyl]-6-O-(t-butyldimethylsilyl)-3-O-decyl-α-D-glucopyranoside

The compound obtained in (7) (203 mg, 0.293 mmol) was dissolved in toluene (6 mL). Pyridine (0.50 g, 6.32 mmol) was added to the solution, and triphosgene (200 mg, 0.674 mmol) was further added thereto under ice cooling. The mixture was vigorously stirred for 10 minutes. Allyl alcohol (0.60 g, 10.331 mmol) was further added to the reaction solution, and the mixture was stirred for 1 hour under ice cooling. After completion of the reaction, the reaction solution was diluted with ethyl acetate, washed with sodium bicarbonate solution and saturated brine, dried over magnesium sulfate, filtrated and concentrated in vacuo. The resulting residue was purified by silica gel column chromatography (eluent: cyclohexane/ethyl acetate=14/1) to give a mixture of the title compound and an unknown substance (325 mg). The obtained title compound was used for the subsequent reaction without further purification.

### [Test Example 1]

Test for inhibition against TNFα production in human whole blood (*in vitro)*

This test was performed according to the method by Hartman et al. (D.A. Hartman, S.J. Ochalski and R.P. Carlson; The effects of antiinflammatory and antiallergic drugs on cytokine release after stimulation of human whole blood by lipopolysaccharide and zymosan A: Inflamm. Res., 44, 269 (1995)).

Peripheral blood was obtained from a healthy volunteer in the presence of heparin. 360 µL of whole blood was added to a 96-well block to which 20 µL of a dimethyl sulfoxide solution of the analyte compound was previously added. 20 µL of lipopolysaccharide (LPS) (derived from *E. coli* O26:B6 available from SIGMA Inc.)(final concentration: 10 ng/mL) was further added thereto as a stimulant, and the mixture was sufficiently mixed and incubated under conditions of 37°C and 5% CO₂ for 6 hours. After completion of the incubation, the mixture was cooled to 4°C to discontinue the reaction, and the mixture was immediately centrifuged at 2000 rpm for 15 minutes. The supernatant plasma was then separated and collected. The TNFα produced and released in the plasma was measured by an enzyme-linked immunosorbent assay (ELISA) kit (made by Biosource International). The inhibition rate was determined from the amount of cytokine produced in the presence and in the absence of the analyte compound. IC₅₀ values were calculated from the average of the inhibition rates by the least-square method. The results are shown in Table 1. For comparison, an IC₅₀ value was calculated in the same manner as in the case of the test compound, using a compound described in U.S. Patent No. 5935938 as a comparative compound. The structural formula of the comparative compound is shown.

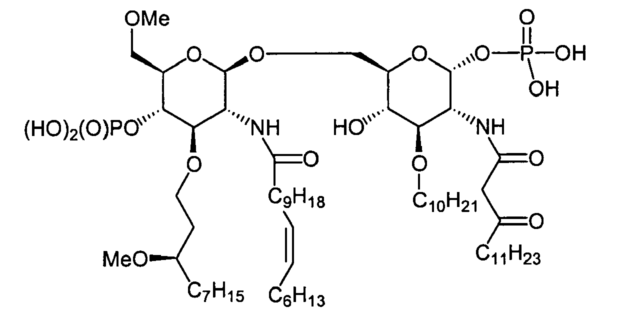

**[Table 1]**

| Test Compound | IC₅₀ (nM) |
|---|---|
| Compound 1 | 0.49 |
| Compound 2 | 0.51 |
| Compound 3 | 0.65 |
| Comparative Compound | 0.97 |

### [Test Example 2]

Test for inhibition against TNFα production in human whole blood *(in vitro)*

This test was performed in the same manner as in Test Example 1. The obtained results are shown in Tables 2 and 3. The same comparative compound as in Test Example 1 was used.

**[Table 2]**

| Test Compound | IC₅₀ (nM) |
|---|---|
| Compound 8 | 0.06 |
| Comparative Compound | 2.94 |

**[Table 3]**

| Test Compound | IC₅₀ (nM) |
|---|---|
| Compound 9 | 0.83 |
| Comparative Compound | 8.24 |

### [Test Example 3]

Test for inhibitory activity against TNFα production in mice *(in vivo)*

This test was performed according to the method by Y. Endo et al. (British Journal of Pharmacology 128, 5-12 (1999)).

A group of five C3H/HeN mice (male, 7-week old) known to be highly sensitive to lipopolysaccharide (LPS) was used. Galactosamine (GalN, 1 g/5 mL) and LPS (0.05 mg/5 mL) were each dissolved in normal saline, and they were mixed in equal amounts to prepare a GalN·LPS solution. The prepared GalN·LPS solution was injected into the tail vein at a rate of 10 mL/kg. The test compound was dissolved in a 0.1% aqueous triethylamine solution and the mixture was injected into the tail vein at a rate of 10 mL/kg substantially simultaneously with the injection of the GalN·LPS solution. An hour after the injection of the GalN·LPS solution, blood was obtained from the inferior vena cava of the mice under ether anesthesia, and the plasma was separated. The amount of TNFα produced in the plasma was measured using a commercially available TNFα ELISA kit. The inhibitory activity against TNFα production of the test compound was calculated as an inhibition rate against TNFα produced in response to the GalN.LPS injection. A regression line was obtained from the inhibition rates and the doses for the mice. The ID₅₀ value (a dose at which the inhibition rate is 50%) was calculated from the regression line. The test results are shown in Tables 4 to 6.

**[Table 4]**

| Test Compound | ID₅₀ (mg/kg) |
|---|---|
| Compound 1 | 0.45 |
| Compound 2 | <0.2 |
| Compound 3 | 0.96 |
| Comparative Compound | 2.12 |

**[Table 5]**

| Test Compound | ID₅₀ (mg/kg) |
|---|---|
| Compound 8 | 0.55 |
| Comparative Compound | 3.28 |

**[Table 6]**

| Test Compound | ID₅₀ (mg/kg) |
|---|---|
| Compound 9 | <0.2 |
| Comparative Compound | 1.82 |

### [Test Example 4]

Survival test for mouse fatal state *(in vivo)*

This test was performed according to the method by H. Takada (The Journal of Infectious Diseases 162, 428-434 (1990)).

Whether mice were alive or not was observed 72 hours after injection of a GalN·LPS solution, and the survival rate was calculated. The strain, age in week and gender of the mice used, the method of preparing the GalN·LPS solution and the compound solution administered, the administration route, the timing and the like were the same as in Test Example 3. A group of 8 mice was used in the test. The test results are shown in Tables 7 to 9.

**[Table 7]**

| | | Survival rate (%) of mice after 72 hours | | |
|---|---|---|---|---|
| Dose (mg/kg) | Test Compound: | Compound 2 | Compound 3 | Comparative Compound |
| 0.3 | | 38 | 50 | 25 |
| 1 | | 75 | 100 | 63 |
| 3 | | 100 | 100 | 100 |

**[Table 8]**

| | | Survival rate (%) of mice after 72 hours | |
|---|---|---|---|
| Dose (mg/kg) | Test Compound: | Compound 8 | Comparative Compound |
| 0.3 | | 50 | 50 |
| 1 | | 75 | 75 |
| 3 | | 100 | 50 |

**[Table 9]**

| | | Survival rate (%) of mice after 72 hours | |
|---|---|---|---|
| Dose (mg/kg) | Test Compound: | Compound 9 | Comparative Compound |
| 0.3 | | 13 | 13 |
| 1 | | 38 | 0 |
| 3 | | 100 | 25 |

### Industrial Applicability

The compound of the present invention has excellent macrophage activity inhibitory action and is useful as an anti-inflammatory agent, an anti-autoimmune disease agent, an immunosuppressive agent, an antiseptic agent or a prognosis-improving agent after coronary artery bypass surgery.

## Claims

1. A compound represented by the following general formula: 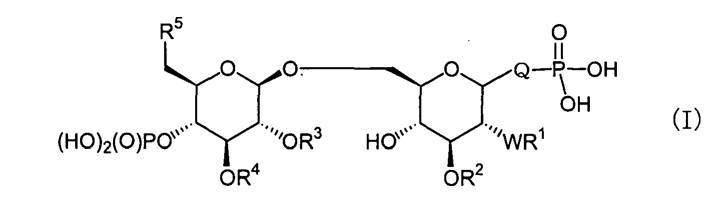 [wherein Q represents an oxygen atom, a C₁-C₃ alkylene group, a -O-Alk- group or a -O-Alk-O- group (in which Alk represents a C₁-C₃ alkylene group),
W represents an oxygen atom or a -NH- group,
R¹ (when W is a -NH- group) represents a C₁-C₂₀ alkanoyl group which may be substituted by at least one group selected from the following Substituent group A, a C₃-C₂₀ alkenoyl group which may be substituted by at least one group selected from the following Substituent group A or a C₃-C₂₀ alkynoyl group which may be substituted by at least one group selected from the following Substituent group A,
R¹ (when W is an oxygen atom), R², R³ and R⁴, which may be the same or different, represent a hydrogen atom, a C₁-C₂₀ alkyl group which may be substituted by at least one group selected from the following Substituent group A, a C₂-C₂₀ alkenyl group which may be substituted by at least one group selected from the following Substituent group A, a C₂-C₂₀ alkynyl group which may be substituted by at least one group selected from the following Substituent group A, a C₁-C₂₀ alkanoyl group which may be substituted by at least one group selected from the following Substituent group A, a C₃-C₂₀ alkenoyl group which may be substituted by at least one group selected from the following Substituent group A or a C₃-C₂₀ alkynoyl group which may be substituted by at least one group selected from the following Substituent group A,
R⁵ represents a hydrogen atom, a halogen atom, a hydroxyl group, a C₁-C₆ alkoxy group which may have an oxo group, a C₂-C₆ alkenyloxy group which may have an oxo group or a C₂-C₆ alkynyloxy group which may have an oxo group,
the Substituent group A consisting of a halogen atom, a hydroxyl group, an oxo group, a C₁-C₂₀ alkoxy group which may have an oxo group, a (C₁-C₂₀ alkoxy) C₁-C₂₀ alkoxy group, a {(C₁-C₂₀ alkoxy) C₁-C₂₀ alkoxy} C₁-C₂₀ alkoxy group, a C₂₋C₂₀ alkenyloxy group which may have an oxo group, a C₂-C₂₀ alkynyloxy group which may have an oxo group, a C₁-C₂₀ alkanoyloxy group which may have an oxo group, a C₃-C₂₀ alkenoyloxy group which may have an oxo group and a C₃-C₂₀ alkynoyloxy group which may have an oxo group], or a pharmacologically acceptable salt thereof.

2. The compound according to claim 1, wherein W is a -NH- group and R¹ is a C₈-C₁₈ alkanoyl or C₈-C₁₈ alkenoyl group, which may have a substituent selected from the Substituent group A, or a pharmacologically acceptable salt thereof.

3. The compound according to claim 1, wherein W is a -NH- group and R¹ is a C₁₀-C₁₈ alkanoyl or C₁₀-C₁₈ alkenoyl group, which may have a substituent selected from the Substituent group A, or a pharmacologically acceptable salt thereof.

4. The compound according to claim 1, wherein W is a -NH- group and R¹ is a C₁₂-C₁₆ alkanoyl or C₁₂-C₁₆ alkenoyl group, which may have a substituent selected from the Substituent group A, or a pharmacologically acceptable salt thereof.

5. The compound according to claim 1, wherein W is an oxygen atom and R¹, R², R³ and R⁴, which may be the same or different, are a C₄-C₁₈ alkyl, C₄-C₁₈ alkenyl, C₄-C₁₈ alkanoyl or C₄-C₁₈ alkenoyl group, which may have a substituent selected from the Substituent group A, or a pharmacologically acceptable salt thereof.

6. The compound according to claim 1, wherein W is an oxygen atom and R¹, R², R³ and R⁴, which may be the same or different, are a C₈-C₁₈ alkyl, C₈-C₁₈ alkenyl, C₈-C₁₈ alkanoyl or C₈-C₁₈ alkenoyl group, which may have a substituent selected from the Substituent group A, or a pharmacologically acceptable salt thereof.

7. The compound according to claim 1, wherein W is an oxygen atom and R¹, R², R³ and R⁴, which may be the same or different, are a C₁₀-C₁₈ alkyl, C₁₀-C₁₈ alkenyl, C₁₀-C₁₈ alkanoyl or C₁₀-C₁₈ alkenoyl group, which may have a substituent selected from the Substituent group A, or a pharmacologically acceptable salt thereof.

8. The compound according to claim 1, wherein W is an oxygen atom and R¹, R², R³ and R⁴, which may be the same or different, are a C₁₂-C₁₆ alkyl, C₁₂-C₁₆ alkenyl, C₁₂-C₁₆ alkanoyl or C₁₂-C₁₆ alkenoyl group, which may have a substituent selected from the Substituent group A, or a pharmacologically acceptable salt thereof.

9. The compound according to claim 1, wherein W is an oxygen atom, R¹ and R³, which may be the same or different, are a C₁₂-C₁₆ alkanoyl or C₁₂-C₁₆ alkenoyl group, which may have a substituent selected from the Substituent group A, and R² and R⁴, which may be the same or different, are a C₁₂-C₁₆ alkyl or a C₁₂-C₁₆ alkenyl group, which may have a substituent selected from the Substituent group A, or a pharmacologically acceptable salt thereof.

10. The compound according to claim 1, wherein W is an oxygen atom, R¹ and R³, which may be the same or different, are a decanoyl, dodecanoyl, tetradecanoyl, dodecenoyl, tetradecenoyl or octadecenoyl group, which may have a substituent selected from the Substituent group A, and R² and R⁴, which may be the same or different, are decyl, dodecyl, tetradecyl, dodecenyl, tetradecenyl or octadecenyl group, which may have a substituent selected from the Substituent group A, or a pharmacologically acceptable salt thereof.

11. The compound according to any one of claims 1 to 10, wherein the substituent selected from the Substituent group A is a fluorine atom, a hydroxyl group, a C₁-C₂₀ alkoxy group, a C₁₂-C₁₄ alkenyloxy group, a C₁₂-C₁₄ alkanoyloxy group or a C₁₂-C₁₄ alkenoyloxy group, or a pharmacologically acceptable salt thereof.

12. The compound according to any one of claims 1 to 10, wherein the substituent selected from the Substituent group A is a dodecyloxy group, a tetradecyloxy group, a dodecenyloxy group, a tetradecenyloxy group, a dodecanoyloxy group, a tetradecanoyloxy group, a dodecenoyloxy group, a tetradecenoyloxy group or an octadecenoyl group, or a pharmacologically acceptable salt thereof.

13. The compound according to any one of claims 1 to 12, wherein R⁵ is a halogen atom, a hydroxyl group or an unsubstituted C₁-C₆ alkoxy group, or a pharmacologically acceptable salt thereof.

14. The compound according to any one of claims 1 to 12, wherein R⁵ is a fluorine atom, a hydroxyl group or a methoxy group, or a pharmacologically acceptable salt thereof.

15. The compound according to any one of claims 1 to 14, wherein Q is an oxygen atom, or a pharmacologically acceptable salt thereof.

16. The compound according to any one of claims 1 to 14, wherein Q is a phosphonoethyl group, or a pharmacologically acceptable salt thereof.

17. The compound according to any one of claims 1 to 16, wherein position 1 of the right-side glucose or glucosamine takes the α configuration, or a pharmacologically acceptable salt thereof.

18. Phosphono 3-O-decyl-2-deoxy-6-O-{3-O-[(R)-3-methoxydecyl]-6-O-methyl-2-O-[(Z)-11-octadecenoyl]-4-O-phosphono-β-D-glucopyranosyl}-2-(3-oxotetradecanoylamino)-α-D-glucopyranoside,
phosphono 3-O-decyl-2-deoxy-6-O-{3-O-[(R)-3-methoxydecyl]-6-O-methyl-2-O-[(Z)-11-octadecenyl]-4-O-phosphono-β-D-glucopyranosyl}-2-(3-oxotetradecanoylamino)-α-D-glucopyranoside,
phosphono 3-O-decyl-2-deoxy-6-O- {3-O-[(R)-3-methoxydecyl]-2-O-[(Z)-11-octadecenoyl]-4-O-phosphono-β-D-glucopyranosyl}-2-(3-oxotetradecanoylamino)-α-D-glucopyranoside,
phosphono 3-O-decyl-2-deoxy-6-O-{3-O-[(R)-3-methoxydecyl]-2-O-[(Z)-11-octadecenyl]-4-O-phosphono-β-D-glucopyranosyl}-2-(3-oxotetradecanoylamino)-α-D-glucopyranoside,
2-(phosphonooxy)ethyl 3-O-decyl-2-deoxy-6-O-{3-O-[(R)-3-methoxydecyl]-6-O-metilyl-2-O-[(Z)-11-octadecenoyl]-4-O-phosphono-β-D-glucopyranosyl}-2-(3-oxotetradecanoylamino)-α-D-glucopyranoside,
2-(phosphonooxy)ethyl 3-O-decyl-2-deoxy-6-O-{3-O-[(R)-3-methoxydecyl]-2-O-[(Z)-11-octadecenoyl]-4-O-phosphono-β-D-glucopyranosyl}-2-(3-oxotetradecanoylamino)-α-D-glucopyranoside,
2-(phosphonooxy)ethyl 6-O-{3-O-[(R)-3-methoxydecyl]-6-O-methyl-2-O-[(Z)-11-octadecenoyl]-4-O-phosphono-β-D-glucopyranosyl}-2,3-di-O-dodecyl-α-D-glucopyranoside,
phosphono 3-O-decyl-6-O-{3-O-[(R)-3-methoxydecyl]-6-O-methyl-2-O-[(Z)-11-octadecenoyl]-4-O-phosphono-β-D-glucopyranosyl}-2-O-(3-oxotetradecanoyl)-α-D-glucopyranoside,
phosphono 3-O-decyl-6-O-{3-O-[(R)-3-methoxydecyl]-2-O-[(Z)-11-octadecenoyl]-4-O-phosphono-β-D-glucopyranosyl}-2-O-(3-oxotetradecanoyl)-α-D-glucopyranoside,
2-(phosphonooxy)ethyl 2,3-di-O-dodecyl-6-O-{6-O-methyl-3-O-[(R)-3-methoxydecyl]-2-O-[(Z)-11-octadecenyl]-4-O-phosphono-β-D-glucopyranosyl}-α-D-glucopyranoside or
phosphono 6-O-{4-O-phosphono-3-O-[(R)-3-methoxydecyl]-6-O-methyl-2-O-[(Z)-11-octadecenyl]-β-D-glucopyranosyl}-3-O-dodecyl-2-O-[(R)-3-hydroxytetradecyl]-α,β-D-glucopyranoside according to claim 1, or a pharmacologically acceptable salt thereof.

19. Phosphono 3-O-decyl-6-O-{3-O-[(R)-3-methoxydecyl]-6-O-methyl-2-O-[(Z)-11-octadecenoyl]-4-O-phosphono-β-D-glucopyranosyl}-2-O-(3-oxotetradecanoyl)-α-D-glucopyranoside or
phosphono 3-O-decyl-6-O-{3-O-[(R)-3-methoxydecyl]-2-O-[(Z)-11-octadecenoyl]-4-O-phosphono-β-D-glucopyranosyl}-2-O-(3-oxotetradecanoyl)-α-D-glucopyranoside according to claim 1, or a pharmacologically acceptable salt thereof.

20. A medicament comprising the compound according to any one of claims 1 to 19 as an active ingredient.

21. An agent for prophylaxis or treatment of inflammation, comprising the compound according to any one of claims 1 to 19 as an active ingredient.

22. An agent for prophylaxis or treatment of an autoimmune disease, comprising the compound according to any one of claims 1 to 19 as an active ingredient.

23. An agent for prophylaxis or treatment of sepsis, comprising the compound according to any one of claims 1 to 19 as an active ingredient.

24. An immunosuppressive agent comprising the compound according to any one of claims 1 to 19 as an active ingredient.

25. A prognosis-improving agent after coronary artery bypass surgery, comprising the compound according to any one of claims 1 to 19 as an active ingredient.

26. Use of the compound according to any one of claims 1 to 19 or a pharmacologically acceptable salt thereof for producing a pharmaceutical composition.

27. A method for prophylaxis or treatment of inflammation, which comprises administering a pharmacologically effective dose of the compound according to any one of claims 1 to 19 or a pharmacologically acceptable salt thereof to a warm-blooded animal.

28. A method for prophylaxis or treatment of an autoimmune disease, which comprises administering a pharmacologically effective dose of the compound according to any one of claims 1 to 19 or a pharmacologically acceptable salt thereof to a warm-blooded animal.

29. A method for prophylaxis or treatment of sepsis, which comprises administering a pharmacologically effective dose of the compound according to any one of claims 1 to 19 or a pharmacologically acceptable salt thereof to a warm-blooded animal.

30. A method for immunosuppression, which comprises administering a pharmacologically effective dose of the compound according to any one of claims 1 to 19 or a pharmacologically acceptable salt thereof to a warm-blooded animal.

31. A method for improving prognosis after coronary artery bypass surgery, which comprises administering a pharmacologically effective dose of the compound according to any one of claims 1 to 19 or a pharmacologically acceptable salt thereof to a warm-blooded animal.
